(19) Europäisches Patentamt
European Patent Office
Office européen des brevets

(11) EP 2 527 319 A1

(12) # EUROPEAN PATENT APPLICATION

(43) Date of publication:
**28.11.2012 Bulletin 2012/48**

(21) Application number: **11384001.1**

(22) Date of filing: **24.05.2011**

(51) Int Cl.:
***C07C 229/08*** (2006.01)
***C07C 275/02*** (2006.01)
***C07C 53/126*** (2006.01)
***C07C 57/145*** (2006.01)
***C07C 59/245*** (2006.01)
***C07C 59/255*** (2006.01)
***C07C 63/06*** (2006.01)
*A61K 31/197* (2006.01)
*A61P 29/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Laboratorios del. Dr. Esteve, S.A.**
**08041 Barcelona (ES)**

(72) Inventors:
- **Plata Salaman, Carlos Ramon**
**Esplugues de Llobregat (Barcelona) (ES)**
- **Tesson, Nicolas**
**L' Hospitalet de Llobregat (ES)**
- **Trilla Castano, Montserrat**
**08013 Barcelona (ES)**
- **Cardenas Romana, Lydia**
**08042 Barcelona (ES)**

(74) Representative: **Peters, Hajo et al**
**ZACCO GmbH**
**Bayerstrasse 83**
**80335 München (DE)**

(54) **Crystalline forms of pregabalin and co-formers in the treatment of pain**

(57) The present invention relates to new crystalline forms of pregabalin and co-formers, processes for preparation of the same and their use as medicament or in pharmaceutical formulations, more particularly for the treatment of pain.

EP 2 527 319 A1

## Description

The present invention relates to new crystalline forms of pregabalin with at least one co-former, processes for preparation of the same, pharmaceutical compositions comprising those and their use as a medicament or in a pharmaceutical formulation, more particularly for the treatment of pain.

Pain is a complex response that has been functionally categorized into sensory, autonomic, motor, and affective components. The sensory aspect includes information about stimulus location and intensity while the adaptive component may be considered to be the activation of endogenous pain modulation and motor planning for escape responses. The affective component appears to include evaluation of pain unpleasantness and stimulus threat as well as negative emotions triggered by memory and context of the painful stimulus.

In general, pain conditions can be divided into chronic and acute. Chronic pain includes neuropathic pain and chronic inflammatory pain, for example arthritis, or pain of unknown origin, as fibromyalgia. Acute pain usually follows non-neural tissue injury, for example tissue damage from surgery or inflammation, or migraine.

Pregabalin is an anticonvulsant drug used for neuropathic pain and as an adjunct therapy for partial seizures with or without secondary generalization in adults. It has also been found effective for generalized anxiety disorder and is approved for this use in the European Union. Recent studies have shown that pregabalin is effective at treating chronic pain in disorders such as fibromyalgia and spinal cord injury. Pregabalin, (*S*)-3-(aminomethyl)-5-methylhexanoic acid has the following formula:

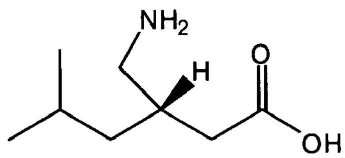

Pregabalin has no activity at GABA, or benzodiazepine receptors, and there are no known pharmacokinetic drug-drug interactions with pregabalin. It has a predictable pharmacokinetic profile with a rapid onset of action and has been found to be safe and efficacious in patients with painful diabetic neuropathy. Pregabalin has demonstrated efficacy in treating neuropathic pain and sleep interference associated with diabetic peripheral neuropathy and postherpetic neuralgia.

Pharmaceutically acceptable salts of pregabalin formed with organic or inorganic acids or bases were suggested in a patent family arising from WO 93/23383 (e.g., EP 641 330). While no salts were actually made, it was disclosed that "the acid addition salts [...] are prepared either by dissolving the free base in aqueous or aqueous alcohol solution or other suitable solvents containing the appropriate acid and isolating the salt by evaporating the solution. Examples of pharmaceutically acceptable salts are hydrochlorides, hydrobromide, hydrosulfates, etc., as well as sodium, potassium, and magnesium, etc., salts." (See WO 93/23383 at pages 6-7.)

It appears that only two pregabalin acid addition salts have been made: pregabalin hydrochloride (WO 2005/041927) and pregabalin (S)-mandelate (WO 96/40617).

Co-crystal of (S)-3-(ammoniomethyl)-5-methylhexanoate and (S)-mandelic acid is disclosed in Acta Crystallographica, Section E: Structure Reports Online (2007), E63(10), o3938, So3938/1-So3938/11. This document discloses the co-crystal of (*S*)-pregabalin and (*S*)-mandelic acid which is used to resolve racemic pregabalin on an industrial scale. However, it does not disclose any specific advantage of the co-crystal itself. Examples of that would include: improvement of solubility, bioavailability of pregabalin or any other improvement in physicochemical properties.

Although pregabalin is used as the free form in the marketed pharmaceutical, it is desirable to provide an alternative pharmaceutically compound. In particular, the free form of pregabalin has solubility in water of 32 mg/ml at pH 7.4. A more water soluble salt or co-crystal could be more convenient for use in aqueous pharmaceutical formulations for faster or immediate release.

For other uses though, it could be advantageous if the crystalline form (the co-crystal or the salt) would be less soluble than the free form. This could then be useful to obtain slow or sustained release formulations. Furthermore, the salt or co-crystal should be sufficiently stable, both in the solid state and in the solution, and it should be obtainable in solid crystalline form.

Thus, it would be desirable to find a crystalline form, like a salt or a co-crystal of pregabalin and at least one co-former with a different solubility than free pregabalin thus being either more soluble or less soluble in water or (preferably also) sufficiently stable or (again preferably also) stable against formation of impurities.

Thus, on one hand in order to increase absorption rate and extent and hence bioavailability of pregabalin, it is an object of the present invention to improve the aqueous solubility and dissolution rate of pregabalin or (or as well as) the retard of precipitation of dissolved pregabalin by providing new drugable forms of pregabalin through a new crystalline

form of pregabalin with a co-former. A parallel or further object of the present invention would be the retard of precipitation of dissolved pregabalin by providing new drugable forms of pregabalin through a new crystalline form of pregabalin with a co-former.

Another object of the present invention would be to provide new means of improving the properties of pregabalin, especially in regard to the treatment of pain, by providing new drugable forms of pregabalin through a new crystalline form of pregabalin with a co-former. Especially desirable improvements/advantages of the new drugable form would include:

- improvement of physicochemical properties in order to facilitate the formulation, the manufacture, or to enhance the absorption and/or the bioavailability
- being easily obtainable, easy to manufacture
- allowing more flexibility in formulating, or facilitating its formulation,
- being highly soluble, thus allowing better dissolution rates, especially if dissolving in an aqueous physiological surrounding; or
- being less soluble, thus allowing to obtain improved slow or sustained release formulations, especially if dissolving in an aqueous physiological surrounding
- improve stability of the co-crystal in comparison with the pregabalin alone
- allowing new routes of administration

Other possible - especially clinical - advantages that would desirably be provided in new drugable forms by the new crystalline form of pregabalin and at least one co-former (especially if compared to any of the active principles alone) are listed below:

- providing more effective pain relief for a broader spectrum of pain,
- reducing adverse drug reactions providing a better safety profile at higher doses,
- enhancing the treatment adherence or compliance of patients,
- increasing the level of efficacy or reaching at least an efficacy similar to the one achievable by pregabalin used alone at higher doses associated with a better safety profile, or
- allowing a reduction of dose - with the desired activity - still reducing the side effects.

This objective was achieved by providing new crystalline forms of pregabalin and different co-formers. These new crystalline forms show improved properties if compared to pregabalin alone. New crystalline forms thus obtained have a specific stoichiometry which depends upon the structure of each coformer. Under the proper circumstance this is also another advantage of this new solid drugable form possibly achieving some modulation of the pharmacological effects.

"Co-former" as used herein is defined as a component with which pregabalin is able to form co-crystals or salts. The co-former is part of the crystal lattice. Typically, co-formers will have the ability to form complementary non-covalent interactions with the active agent, for example the ability to form hydrogen bonds with the active agent in the case of co-crystals.

The physicochemical properties are improved. The formulation of the association is even easier with a solid to manipulate and an enhanced stability. The solubility may be also enhanced or - if desired (see above) - lowered.

Another advantage is that the combination of one active principle and a co-former into one unique species seems to allow for a better Pharmacokinetic/Pharmacodynamic (PKPD) which helps in the treatment of pain.

Especially desirable improvements/advantages of the new drugable form as required above - like improvement of physicochemical properties - seem to have been achieved. The tested physicochemical properties include hygroscopicity and solubility.

The main object of the invention accordingly is a crystalline form of pregabalin with at least one co-former.

In one embodiment (proviso) the crystalline form of the salt pregabalin hydrochloride is excluded.

In a further embodiment (proviso) the crystalline form of the salt pregabalin (S)-mandelate is excluded.

In a further embodiment (proviso) the co-crystal of (S)-pregabalin and (S)-mandelic acid are excluded.

In a further embodiment (proviso) the crystalline form of the acid addition salts of pregabalin being hydrochloride, hydrobromide, hydrosulfate salts are excluded.

In a further embodiment (proviso) the crystalline form of sodium, potassium, and magnesium salt of pregabalin are excluded.

In another embodiment (proviso)

- the crystalline form of the salt pregabalin hydrochloride,
- the crystalline form of the salt pregabalin (S)-mandelate,

- the co-crystal of (S)-pregabalin and (S)-mandelic acid,
- the crystalline form of the acid addition salts of pregabalin being hydrochloride, hydrobromide, hydrosulfate salts,
- the crystalline form of sodium, potassium, and magnesium salt of pregabalin are excluded

In one embodiment of the crystalline form according to the invention the crystalline form is the crystalline form of a salt or is a co-crystal.

In another embodiment of the crystalline form according to the invention the co-former is selected from the group consisting of tartaric acid, benzoic acid, capric acid, caprylic acid, lauric acid, maleic acid, L-proline, nicotinamide, malic acid, urea or their stereoisomers. In a preferred embodiment the co-former is selected from the group consisting of tartaric acid, malic acid, benzoic acid, capric acid, caprylic acid, lauric acid, maleic acid, L-proline.

In another embodiment of the crystalline form according to the invention the crystalline form is the crystalline form of a salt of pregabalin and a co-former being a salt-co-former. Preferably, the pregabalin is (S)-pregabalin.

In another embodiment of the crystalline form according to the invention the crystalline form is the crystalline form of a salt of pregabalin and a co-former being a salt-co-former, the salt-co-former is selected from the group consisting of tartaric acid, benzoic acid, capric acid, caprylic acid, lauric acid, maleic acid, L-proline, nicotinamide, malic acid, urea or their stereoisomers. Preferably the co-former being a salt-co-former is selected from the group consisting of tartaric acid, malic acid, maleic acid, or their stereoisomers. Even more preferably, the co-former being a salt-co-former is selected from the group consisting of L-(+)-tartaric acid, L-malic acid, or maleic acid.

In another embodiment of the crystalline form according to the invention the crystalline form is the crystalline form of a salt of pregabalin and a co-former being a salt-co-former, the salt is selected from the group consisting of (*S*)-pregabalin - L-(+)-tartaric acid (1:1), (*S*)-pregabalin - L-(+)-tartaric acid - $H_2O$ (1:1:1), (*S*)-pregabalin - L-malic acid (1:1), (*S*)-pregabalin - maleic acid (1:1), (S)-pregabalin - maleic acid - $H_2O$ (1:1:0.5) or (*S*)-pregabalin - maleic acid anhydrous (1:1). Preferably the salt is selected from the group consisting of (*S*)-pregabalin - L-(+)-tartaric acid - $H_2O$ (1: 1:1), (*S*)-pregabalin - L-malic acid (1:1), or (*S*)-pregabalin - maleic acid (1:1).

In one embodiment of the crystalline form according to the invention the crystalline form is the crystalline form of a salt of (*S*)-pregabalin - L-(+)-tartaric acid (1:1).

In one embodiment the crystalline form of a salt of (*S*)-pregabalin - L-(+)-tartaric acid (1:1) shows an X-Ray powder diffraction pattern with peaks [2θ] at 7.1, 14.2, 17.5, 21.3, and 23.8 [°]; preferably at 7.1, 14.2, 17.1, 17.5, 20.6, 20.8, 21.3, 22.0, 23.8, 24.0, 24.8, 25.1, 25.4, 26.0 and 27.4 [°]; more preferably at 7.08, 14.16, 17.13, 17.51, 20.60, 20.81, 21.29, 21.96, 23.84, 24-03, 24.81, 25.08, 25.36, 26.01 and 27.43 [°]. The 2θ values were obtained using copper radiation ($Cu_{K\alpha}$). In an embodiment the crystalline form of a salt of (*S*)-pregabalin - L-(+)-tartaric acid (1:1) shows an endothermic peak corresponding to the melting point having an onset at 128°C or 127.7 °C or 127.70 °C.

In one embodiment of the crystalline form according to the invention the crystalline form is the crystalline form of a salt of (S)-pregabalin - L-(+)-tartaric acid - $H_2O$ (1:1:1).

In one embodiment the crystalline form of a salt of (*S*)-pregabalin - L-(+)-tartaric acid - $H_2O$ (1:1:1) shows an X-Ray powder diffraction pattern with peaks [2θ] at 9.2, 12.0, 14.0, 14.2, 15.3, 16.0, 17.9, 18.4, 19.9, 20.5, 24.0, 24.7, 24.8, and 27.1 [°]; preferably at 9.2, 12.0, 14.0, 14.2, 15.3, 16.0, 17.9, 18.4, 19.4, 19.9, 20.5, 21.9, 24.0, 24.7, 24.8, 25.4, 26.0, 27.1, 28.2, 28.7, 29.5, 31.0, 32.2, 33.1, 34.9, 36.1, 36.5, 37.4 and 39.1 [°]; more preferably at 9.16, 11.95, 14.04, 14.23, 15.26, 15.99, 17.86, 18.41, 19.44, 19.90, 20.52, 21.88, 23.97, 24.67, 24.76, 25.35, 26.00, 27.06, 28.19, 28.71, 29.47, 30.98, 32.16, 33.11, 34.93, 36.11, 36.45, 37.39 and 39.06 [°]. The 2θ values were obtained using copper radiation ($Cu_{K\alpha}$). In an embodiment the crystalline form of a salt of (*S*)-pregabalin - L-(+)-tartaric acid - $H_2O$ (1:1:1) shows an endothermic peak corresponding to the melting point having an onset at 103°C or 102.8 °C or 102.79 °C. In an embodiment the crystalline form of a salt of (S)-pregabalin - L-(+)-tartaric acid - $H_2O$ (1:1:1) crystallizes in a monoclinic crystal system with the following unit cell dimensions:

- a = 7.45 Å
- b = 11.61 Å
- c = 9.75 Å
- β angle of 98.14°.

In one embodiment of the crystalline form according to the invention the crystalline form is the crystalline form of a salt of (*S*)-pregabalin - L-malic acid (1:1).

In one embodiment the crystalline form of a salt of (*S*)-pregabalin - L-malic acid (1:1) shows an X-Ray powder diffraction pattern with peaks [2θ] at 6.7, 13.3, 17.7 and 23.3 [°]; preferably at 6.7, 13.3, 15.4, 16.8, 17.7, 19.3, 20.1, 20.4, 21.3, 23.3, 25.5, 26.1, 26.5, 26.8, 27.1, 28.1 and 28.8 [°]; more preferably at 6.65, 13.32, 15.36, 16.84, 17.74, 19.33, 20.05, 20.40, 21.28, 23.25, 25.50, 26.09, 26.46, 26.84, 27.09, 28.13 and 28.81 [°]. The 2θ values were obtained using copper radiation ($Cu_{K\alpha}$). In an embodiment the crystalline form of a salt of (*S*)-pregabalin - L-malic acid (1:1) shows an endothermic peak corresponding to the melting point having an onset at 81 °C or 80.6 °C or 80.58 °C. In an embodiment

the crystalline form of a salt of (*S*)-pregabalin - L-malic acid (1:1) crystallizes in an orthorhombic crystal system with the following unit cell dimensions:

- a=7.56 Å
- b = 7,60 Å
- c = 26,48 Å

In one embodiment of the crystalline form according to the invention the crystalline form is the crystalline form of a salt of (*S*)-pregabalin - maleic acid (1:1).

In one embodiment the crystalline form of a salt of (*S*)-pregabalin - maleic acid (1:1) shows an X-Ray powder diffraction pattern with peaks [2θ] at 4.6, 9.3, 17.9 and 25.63 [°]; preferably at 4.6, 9.3, 12.1, 13.8, 14.9, 16.3, 17.4, 17.9, 18.4, 18.6, 18.8, 19.6, 20.3, 20.8, 22.0, 23.8, 24.9, 25.0, 25.6, 26.5, 27.2 and 29.2 [°]; more preferably at 4.64, 9.26, 12.14, 13.80, 14.89, 16.33, 17.40, 17.87, 18.40, 18.59, 18.76, 19.61, 20.29, 20.82, 21.98, 23.77, 24.86, 24.98, 25.63, 26.47, 27.20 and 29.16 [°]. The 2θ values were obtained using copper radiation ($Cu_{K\alpha}$). In an embodiment the crystalline form of a salt of (*S*)-pregabalin - maleic acid (1:1) shows an endothermic peak corresponding to the melting point having an onset at 82 °C or 82.5 °C or 82.53°C. In an embodiment the crystalline form of a salt of (*S*)-pregabalin - maleic acid (1:1) crystallizes in an orthorhombic crystal system with the following unit cell dimensions:

- a = 7.42 Å
- b = 10,27 Å
- c = 38,22 Å

In one embodiment of the crystalline form according to the invention the crystalline form is the crystalline form of a salt of (*S*)-pregabalin - maleic acid - $H_2O$ (1:1:0.5)

In one embodiment the crystalline form of a salt of (*S*)-pregabalin - maleic acid - $H_2O$ (1:1:0.5) shows an X-Ray powder diffraction pattern with peaks [2θ] at 5.9, 11.9, 18.7, 23.3, 23.8, 29.8 and 29.9 [°] or at 5.93, 11.85, 18.65, 23.32, 23.82, 29.87 and 29.92 [°]; preferably at 5.9, 6.9, 11.9, 12.6, 13.8, 17.0, 17.5, 18.1, 18.4, 18.7, 19.3, 19.5, 19.8, 21.0, 21.7, 22.5, 23.3, 23.8, 24.8, 25.2, 25.3, 25.9, 26.2, 29.8, 29.9, 31.3 and 36.1 [°]; more preferably at 5.93, 6.94, 11.85, 12.62, 13.76, 17.02, 17.50, 18.14, 18.35, 18.65, 19.31, 19.45, 19.84, 21.00, 21.71, 22.50, 23.32, 23.82, 24.78, 25.21, 25.33, 25.87, 26.22, 29.87, 29.92, 31.29 and 36.05 [°]. The 2θ values were obtained using copper radiation ($Cu_{K\alpha}$). In an embodiment the crystalline form of a salt of (*S*)-pregabalin - maleic acid - $H_2O$ (1:1:0.5) shows an endothermic peak corresponding to the melting point having an onset at 58°C or 58.4 °C or 58.54 °C. In an embodiment the crystalline form of a salt of (*S*)-pregabalin - maleic acid - $H_2O$ (1:1:0.5) crystallizes in a monoclinic crystal system with the following unit cell dimensions:

- a = 30.48 Å
- b = 5.62 Å
- c = 17.98 Å
- β angle of 101,71°.

In one embodiment of the crystalline form according to the invention the crystalline form is the crystalline form of a salt of (*S*)-pregabalin - maleic acid anhydrous (1:1).

In one embodiment the crystalline form of a salt of (S)-pregabalin - maleic acid anhydrous (1:1) shows an X-Ray powder diffraction pattern with peaks [2θ] at 7.7, 15.4, 16.8, 17.7, 19.5, 23.1, and 24.0 [°] or at 7.7, 11.3, 15.4, 16.0, 16.8, 17.0, 17.7, 19.5, 20.7, 22.6, 23.1, 24.0, 25.1 and 25.9 [°]; preferably at 7.7, 10.6, 11.3, 15.4, 15.6, 16.0, 16.8, 17.0, 17.7, 19.5, 20.7, 21.3, 22.6, 23.1, 23.5, 24.0, 24.4, 25.1, 25.9, 27.2, 30.2, 31.0, 31.5, 34.0, 36.2 and 39.1 [°]; more preferably at 7.66, 10.57, 11.25, 15.36, 15.57, 16.02, 16.79, 16.95, 17.67, 19.47, 20.69, 21.25, 22.62, 23.13, 23.45, 23.97, 24.44, 25.05, 25.86, 27.19, 30.17, 30.99, 31.52, 33.99, 36.17 and 39.05 [°]. The 2θ values were obtained using copper radiation ($Cu_{K\alpha}$). In an embodiment the crystalline form of a salt of (*S*)-pregabalin - maleic acid anhydrous (1:1) shows an endothermic peak corresponding to the melting point having an onset at 73 °C or 73.5 °C or 73.47 °C.

In a very preferred embodiment of the crystalline form according to the invention the compound is a co-crystal comprising pregabalin as a free base or as its pharmaceutically acceptable salt and at least one co-former being a co-crystal former.

In an embodiment of the crystalline form being a co-crystal the pregabalin is (S)-pregabalin.

In an embodiment of the crystalline form being a co-crystal the co-crystal former is selected from the group consisting of tartaric acid, benzoic acid, capric acid, caprylic acid, lauric acid, maleic acid, proline, or their stereoisomers; more preferably the co-former is selected from the group consisting of tartaric acid, benzoic acid, maleic acid, proline, caprylic acid, capric acid, lauric acid or their stereoisomers; most preferably the co-former is selected from the group

consisting of benzoic acid, L-proline, caprylic acid, capric acid, or lauric acid.

In an embodiment of the crystalline form being a co-crystal the co-crystal is selected from (*S*)-pregabalin - L-tartaric acid (1:1), (*S*)-pregabalin - benzoic acid (1:1), (*S*)-pregabalin - L-proline - HCl (1:1:1), (*S*)-pregabalin - maleic acid - $H_2O$ (1:1:0.5), (*S*)-pregabalin - caprylic acid (1:1), (*S*)-pregabalin - capric acid (1:1), (*S*)-pregabalin - maleic acid anhydrous (1:1), or (*S*)-pregabalin - lauric acid (1:1); more preferably the co-crystal is selected from (*S*)-pregabalin - benzoic acid (1:1), (*S*)-pregabalin - L-proline - HCl (1:1:1). (*S*)-pregabalin - caprylic acid (1:1), (*S*)-pregabalin - capric acid (1:1); or (*S*)-pregabalin - lauric acid (1:1).

In one embodiment of the crystalline form according to the invention the crystalline form is the crystalline form of a co-crystal of (*S*)-pregabalin - L-(+)-tartaric acid (1:1).

In one embodiment the crystalline form of a co-crystal of (*S*)-pregabalin - L-(+)-tartaric acid (1:1) shows an X-Ray powder diffraction pattern with peaks [2θ] at 7.1, 14.2, 17.5, 21.3, and 23.8 [°]; preferably at 7.1, 14.2, 17.1, 17.5, 20.6, 20.8, 21.3, 22.0, 23.8, 24.0, 24.8, 25.1, 25.4, 26.0 and 27.4 [°]; more preferably at 7.08, 14.16, 17.13, 17.51, 20.60, 20.81, 21.29, 21.96, 23.84, 24.03, 24.81, 25.08, 25.36, 26.01 and 27.43 [°]. The 2θ values were obtained using copper radiation ($Cu_{K\alpha}$). In an embodiment the crystalline form of a co-crystal of (*S*)-pregabalin - L-(+)-tartaric acid (1:1) shows an endothermic peak corresponding to the melting point having an onset at 128°C or 127.7 °C or 127.70 °C.

In one embodiment of the crystalline form according to the invention the crystalline form is the crystalline form of a co-crystal of (*S*)-pregabalin - benzoic acid (1:1).

In one embodiment the crystalline form of a co-crystal of (*S*)-pregabalin - benzoic acid (1:1) shows an X-Ray powder diffraction pattern with peaks [2θ] at 5.5, 18.5, 21.4 and 23.7 [°]; preferably at 5.5, 14.2, 15.8, 16.7, 17.4, 18.0, 18.5, 19.5, 21.4, 22.3, 23.2, 23.7, 24.4, 24.5 and 27.0 [°]; more preferably at 5.53, 14.15, 15.75, 16.67, 17.40, 18.02, 18.48, 19.51, 21.43, 22.30, 23.15, 23.67, 24.37, 24.49 and 26.96 [°]. The 2θ values were obtained using copper radiation ($Cu_{K\alpha}$). In an embodiment the crystalline form of a co-crystal of (*S*)-pregabalin - benzoic acid (1:1) shows an endothermic peak corresponding to the melting point having an onset at 128°C or 128,3 °C or 128.30 °C. In an embodiment the crystalline form of a co-crystal of (*S*)-pregabalin - benzoic acid (1:1) crystallizes in an orthorhombic crystal system with the following unit cell dimensions:

- a = 6.61 Å
- b = 7,73 Å
- c=31.82 Å

In one embodiment of the crystalline form according to the invention the crystalline form is the crystalline form of a co-crystal of (*S*)-pregabalin - L-proline - HCl (1:1:1).

In one embodiment the crystalline form of a co-crystal of (*S*)-pregabalin - L-proline - HCl (1:1:1) shows an X-Ray powder diffraction pattern with peaks [2θ] at 8.1, 16.3, 17.4, 21.2, 22.2, 25.9, 27.2 and 30.3 [°]; preferably at 8.1, 12.9, 14.1, 14.6, 16.3, 17.4, 19.2, 20.2, 21.2, 22.2, 24.6, 25.5, 25.9, 26.3, 27.2, 28.3, 29.1, 30.3, 32.1, 34.2 and 35.7 [°]; more preferably at 8.05, 12.88, 14.08, 14.57, 16.25, 17.40, 19.15, 20.16, 21.24, 22.22, 24.59, 25.54, 25.94, 26.32, 27.15, 28.29, 29.06, 30.25, 32.13, 34.20 and 35.72 [°]. The 2θ values were obtained using copper radiation ($Cu_{K\alpha}$). In an embodiment the crystalline form of a co-crystal of (*S*)-pregabalin - L-proline - HCl (1:1:1) shows an endothermic peak corresponding to the melting point having an onset at 107 °C or 107.3 °C or 107.32 °C.

In one embodiment of the crystalline form according to the invention the crystalline form is the crystalline form of a co-crystal of (*S*)-pregabalin - maleic acid - $H_2O$ (1:1:0.5)

In one embodiment the crystalline form of a co-crystal of (*S*)-pregabalin - maleic acid - $H_2O$ (1:1:0.5) shows an X-Ray powder diffraction pattern with peaks [2θ] at 5.9, 11.9, 18.7, 23.3, 23.8, 29.8 and 29.9 [°] or at 5.93, 11.85, 18.65, 23.32, 23.82, 29.87 and 29.92 [°]; preferably at 5.9, 6.9, 11.9, 12.6, 13.8, 17.0, 17.5, 18.1, 18.4, 18.7, 19.3, 19.5, 19.8, 21.0, 21.7, 22.5, 23.3, 23.8, 24.8, 25.2, 25.3, 25.9, 26.2, 29.8, 29.9, 31.3 and 36.1 [°]; more preferably at 5.93, 6.94, 11.85, 12.62, 13.76, 17.02, 17.50, 18.14, 18.35, 18.65, 19.31, 19.45, 19.84, 21.00, 21.71, 22.50, 23.32, 23.82, 24.78, 25.21, 25.33, 25.87, 26.22, 29.87, 29.92, 31.29 and 36.05 [°]. The 2θ values were obtained using copper radiation ($Cu_{K\alpha}$). In an embodiment the crystalline form of a co-crystal of (*S*)-pregabalin - maleic acid - $H_2O$ (1:1:0.5) shows an endothermic peak corresponding to the melting point having an onset at 58 °C or 58.5 °C or 58.54 °C. In an embodiment the crystalline form of a co-crystal of (*S*)-pregabalin - maleic acid - $H_2O$ (1:1:0.5) crystallizes in a monoclinic crystal system with the following unit cell dimensions:

- a=30.48 Å
- b = 5.61 Å
- c = 17.98 Å
- β angle of 101,71°.

In one embodiment of the crystalline form according to the invention the crystalline form is the crystalline form

of a co-crystal of (*S*)-pregabalin - caprylic acid (1:1).

In one embodiment the crystalline form of a co-crystal of (*S*)-pregabalin - caprylic acid (1:1) shows an X-Ray powder diffraction pattern with peaks [2θ] at 4.7, 20.0, 21.3, 21.4, and 23.0 [°]; preferably at 4.7, 9.5, 12.3, 13.4, 16.5, 17.9, 18.2, 19.0, 20.0, 21.3, 21.4, 23.0, 23.4, 24.0 and 25.4 [°]; more preferably at 4.74, 9.50, 12.31, 13.42, 16.45, 17.92, 18.24, 18.99, 20.02, 21.29, 21.44, 22.97, 23.35, 23.98 and 25.42 [°]. The 2θ values were obtained using copper radiation ($Cu_{K\alpha}$). In an embodiment the crystalline form of a co-crystal of (*S*)-pregabalin - caprylic acid (1:1) shows an endothermic peak corresponding to the melting point having an onset at 116 °C or 116.3 °C or 116.30 °C. In an embodiment the crystalline form of a co-crystal of (*S*)-pregabalin - caprylic acid (1:1) crystallizes in a orthorombic crystal system with the following unit cell dimensions:

- a = 6.62 Å
- b = 7.77 Å
- c = 37.00 Å.

In one embodiment of the crystalline form according to the invention the crystalline form is the crystalline form of a co-crystal of (*S*)-pregabalin - capric acid (1:1).

In one embodiment the crystalline form of a co-crystal of (*S*)-pregabalin - capric acid (1:1) shows an X-Ray powder diffraction pattern with peaks [2θ] at 4.3, 8.6, 19.6, 20.7, 21.9, and 22.9 [°]; preferably at 4.3, 8.6, 12.2, 13.1, 13.5, 14.9, 17.2, 17.7, 18.1, 18.6, 19.6, 20.2, 20.7, 21.9, 22.9, 23.3, 23.7, 26.9, 27.5 and 37.3 [°]; more preferably at 4.31, 8.59, 12.15, 13.07, 13.54, 14.85, 17.22, 17.72, 18.12, 18.64, 19.60, 20.20, 20.66, 21.89, 22.94, 23.25, 23.73, 26.93, 27.46 and 37.29 [°]. The 2θ values were obtained using copper radiation ($Cu_{K\alpha}$). In an embodiment the crystalline form of a co-crystal of (*S*)-pregabalin - capric acid (1:1) shows an endothermic peak corresponding to the melting point having an onset at 117 °C or 117.1 °C or 117.08 °C. In an embodiment the crystalline form of a co-crystal of (*S*)-pregabalin - capric acid (1:1) crystallizes in a orthorombic crystal system with the following unit cell dimensions:

- a = 6.61 Å
- b = 7.79 Å
- c = 40.99 Å.

In one embodiment of the crystalline form according to the invention the crystalline form is the crystalline form of a co-crystal of (*S*)-pregabalin - maleic acid anhydrous (1:1).

In one embodiment the crystalline form of a co-crystal of (*S*)-pregabalin - maleic acid anhydrous (1:1) shows an X-Ray powder diffraction pattern with peaks [2θ] at 7.7, 15.4, 16.8, 17.7, 19.5, 23.1, and 24.0 [°] or at 7.7, 11.3, 15.4, 16.0, 16.8, 17.0, 17.7, 19.5, 20.7, 22.6, 23.1, 24.0, 25.1 and 25.9 [°]; preferably at 7.7, 10.6, 11.3, 15.4, 15.6, 16.0, 16.8, 17.0, 17.7, 19.5, 20.7, 21.3, 22.6, 23.1, 23.5, 24.0, 24.4, 25.1, 25.9, 27.2, 30.2, 31.0, 31.5, 34.0, 36.2 and 39.1 [°]; more preferably 7.66, 10.57, 11.25, 15.36, 15.57, 16.02, 16.79, 16.95, 17.67, 19.47, 20.69, 21.25, 22.62, 23.13, 23.45, 23.97, 24.44, 25.05, 25.86, 27.19, 30.17, 30.99, 31.52, 33.99, 36.17 and 39.05 [°]. The 2θ values were obtained using copper radiation ($Cu_{K\alpha}$). In an embodiment the crystalline form of a co-crystal of (*S*)-pregabalin - maleic acid anhydrous (1:1) shows an endothermic peak corresponding to the melting point having an onset at 73 °C or 73.5 °C or 73.47 °C.

In one embodiment of the crystalline form according to the invention the crystalline form is the crystalline form of a co-crystal of (*S*)-pregabalin - lauric acid (1:1).

In one embodiment the crystalline form of a co-crystal of (*S*)-pregabalin - lauric acid (1:1) shows an X-Ray powder diffraction pattern with peaks [2θ] at 4.0, 8.0, 20.1, 20.8, 21.2, and 23.0 [°] or at 4.0, 8.0, 12.9, 17.9, 19.2, 20.1, 20.8, 21.2, 22.5, 23.0 and 23.3 [°]; preferably at 4.0, 8.0, 12.1, 12.9, 13.7, 14.4, 16.5, 17.4, 17.9, 18.4, 19.2, 20.1, 20.8, 21.2, 22.5, 23.0, 23.3, 23.7, 24.2, 25.2, 26.7, 27.1, 27.7, 29.3 and 32.2 [°]; more preferably at 4.02, 8.03, 12.07, 12.87, 13.69, 14.43, 16.54, 17.44, 17.85, 18.42, 19.19, 20.12, 20.84, 21.22, 22.45, 22.97, 23.25, 23.69, 24.16, 25.24, 26.65, 27.06, 27.68, 29.30 and 32.23 [°]. The 2θ values were obtained using copper radiation ($Cu_{K\alpha}$). In an embodiment the crystalline form of a co-crystal of (*S*)-pregabalin - lauric acid (1:1) shows an endothermic peak corresponding to the melting point having an onset at 122 °C or 122.0 °C or 121.99 °C. In an embodiment the crystalline form of a co-crystal of (*S*)-pregabalin - lauric acid (1:1) crystallizes in a orthorombic crystal system with the following unit cell dimensions:

- a = 6.76 Å
- b = 7.76 Å
- c = 43.80 Å.

In an embodiment of the crystalline form according to invention the crystal form is selected from forms of (*S*)-pregabalin - L-(+)-tartaric acid (1:1), (*S*)-pregabalin - maleic acid - $H_2O$ (1:1:0.5) or (*S*)-pregabalin - maleic acid anhydrous (1:1).

In one embodiment of the crystalline form according to the invention the crystalline form is of (*S*)-pregabalin - L-(+)-tartaric acid (1:1).

In one embodiment the crystalline form of (*S*)-pregabalin - L-(+)-tartaric acid (1:1) shows an X-Ray powder diffraction pattern with peaks [2θ] at 7.1, 14.2, 17.5, 21.3, and 23.8 [°]; preferably at 7.1, 14.2, 17.1, 17.5, 20.6, 20.8, 21.3, 22.0, 23.8, 24.0, 24.8, 25.1, 25.4, 26.0 and 27.4 [°]; more preferably at 7.08, 14.16, 17.13, 17.51, 20.60, 20.81, 21.29, 21.96, 23.84, 24.03, 24.81, 25.08, 25.36, 26.01 and 27.43 [°]. The 2θ values were obtained using copper radiation ($Cu_{K\alpha}$). In an embodiment the crystalline form of (*S*)-pregabalin - L-(+)-tartaric acid (1:1) shows an endothermic peak corresponding to the melting point having an onset at 128 °C or 127.7 °C or 127.70°C.

In one embodiment of the crystalline form according to the invention the crystalline form is of (*S*)-pregabalin - maleic acid - $H_2O$ (1:1:0.5)

In one embodiment the crystalline form of (*S*)-pregabalin - maleic acid - $H_2O$ (1:1:0.5) shows an X-Ray powder diffraction pattern with peaks [2θ] at 5.9, 11.9, 18.7, 23.3, 23.8, 29.8 and 29.9 [°] or at 5.93, 11.85, 18.65, 23.32, 23.82, 29.87 and 29.92 [°]; preferably at 5.9, 6.9, 11.9, 12.6, 13.8, 17.0, 17.5, 18.1, 18.4, 18.7, 19.3, 19.5, 19.8, 21.0, 21.7, 22.5, 23.3, 23.8, 24.8, 25.2, 25.3, 25.9, 26.2, 29.8, 29.9, 31.3 and 36.1 [°]; more preferably 5.93, 6.94, 11.85, 12.62, 13.76, 17.02, 17.50, 18.14, 18.35, 18.65, 19.31, 19.45, 19.84, 21.00, 21.71, 22.50, 23.32, 23.82, 24.78, 25.21, 25.33, 25.87, 26.22, 29.87, 29.92, 31.29 and 36.05 [°]. The 2θ values were obtained using copper radiation ($Cu_{K\alpha}$). In an embodiment the crystalline form of (*S*)-pregabalin - maleic acid - $H_2O$ (1:1:0.5) shows an endothermic peak corresponding to the melting point having an onset at 58 °C or 58.5 °C, or 58.54 °C. In an embodiment the crystalline form of (*S*)-pregabalin - maleic acid - $H_2O$ (1:1:0.5) crystallizes in a monoclinic crystal system with the following unit cell dimensions:

- a = 30.48 Å
- b = 5.61 Å
- c = 17.98 Å
- β angle of 101,71°.

In one embodiment of the crystalline form according to the invention the crystalline form is of (*S*)-pregabalin - maleic acid anhydrous (1:1).

In one embodiment the crystalline form of (*S*)-pregabalin - maleic acid anhydrous (1:1) shows an X-Ray powder diffraction pattern with peaks [2θ] at 7.7, 15.4, 16.8, 17.7, 19.5, 23.1, and 24.0 [°] or at 7.7, 11.3, 15.4, 16.0, 16.8, 17.0, 17.7, 19.5, 20.7, 22.6, 23.1, 24.0, 25.1 and 25.9 [°]; preferably at 7.7, 10.6, 11.3, 15.4, 15.6, 16.0, 16.8, 17.0, 17.7, 19.5, 20.7, 21.3, 22.6, 23.1, 23.5, 24.0, 24.4, 25.1, 25.9, 27.2, 30.2, 31.0, 31.5, 34.0, 36.2 and 39.1 [°]; more preferably 7.66, 10.57, 11.25, 15.36, 15.57, 16.02, 16.79, 16.95, 17.67, 19.47, 20.69, 21.25, 22.62, 23.13, 23.45, 23.97, 24.44, 25.05, 25.86, 27.19, 30.17, 30.99, 31.52, 33.99, 36.17 and 39.05 [°]. The 2θ values were obtained using copper radiation ($Cu_{K\alpha}$). In an embodiment the crystalline form of (*S*)-pregabalin - maleic acid anhydrous (1:1) shows an endothermic peak corresponding to the melting point having an onset at 73 °C or 73.5 °C or 73.47 °C.

In a further embodiment of the crystalline form according to the invention in general the ratio between pregabalin and co-former is chosen in such a way that if compared to either pregabalin alone and/or to a mixture of pregabalin and co-former

- the solubility of the co-crystal is increased; and/or
- the dose response of the co-crystal is increased; and/or
- the efficacy of the co-crystal is increased; and/or
- the dissolution of the co-crystal is increased; and/or
- the bioavailability of the co-crystal is increased; and/or
- the stability of the co-crystal is increased; and/or
- the hygroscopicity of the co-crystal is decreased; and/or
- the form diversity of the co-crystal is decreased; and/or
- the morphology of the co-crystal is modulated.

Pregabalin is a well-known pharmaceutical product already used for a long time worldwide. Due to the therapeutic interest in pregabalin in the treatment of pain symptoms, or anxiety a further object of the present invention is a medicament or pharmaceutical composition comprising at least one crystalline form of pregabalin with at least one co-former pregabalin and co-former (either as a salt or as a co-crystal).

Thus, the invention also relates to a medicament (or pharmaceutical composition) comprising at least one crystalline form - either as a salt or as a co-crystal - according to this invention and optionally one or more pharmaceutically acceptable excipients.

The medicament or pharmaceutical composition according to the present invention may be in any form suitable for the application to humans and/or animals, preferably humans including infants, children and adults and can be

produced by standard procedures known to those skilled in the art. The medicament can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the route of administration.

The medicament or pharmaceutical composition of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments or pharmaceutical compositions may for example be injected intramuscularly, intraperitoneally, or intravenously.

Medicaments or pharmaceutical compositions according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or controlled release. The multiparticulate forms, such as pellets or granules, may e.g. be filled into a capsule, compressed into tablets or suspended in a suitable liquid.

Suitable controlled release formulations, materials and methods for their preparation are known from the prior art, e.g. from the table of contents of "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, I, Basic Concepts, Bruck, S.D. (Ed.), CRD Press Inc., Boca Raton (1983) y de Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective descriptions are hereby incorporated by reference and form part of the disclosure.

Medicaments or pharmaceutical compositions according to the present invention may also comprise an enteric coating, so that their dissolution is dependent on pH-value. Due to said coating the medicament may pass the stomach undissolved and the respective pharmaceutical composition and their component(s) is/are liberated in the intestinal tract. Preferably the enteric coating is soluble at a pH value of 5 to 7.5. Suitable materials and methods for the preparation are known from the prior art.

Typically, the medicaments or pharmaceutical compositions according to the present invention may contain 1-60% by weight of the combination of pregabalin and co-former as defined herein and 40-99% by weight of one or more auxiliary substances (additives/excipients).

The compositions of the present invention may also be administered topically or via a suppository.

The daily dosage for humans and animals (especially adults) may vary depending on factors that have their basis in the respective species or other factors, such as age, sex, weight or degree of illness and so forth. The daily dosage for humans preferably is in the range of 50 to 2000 milligrams of pregabalin or preferably 100 to 1000 mg, or most preferably 200 to 750 mg to be administered during one or several intakes per day e.g, in doses of 100, 200, 300, 450 or 600 mg.

The daily dosage for humans in the medicament or in the pharmaceutical composition according to the invention preferably is in the range in which the pharmaceutical composition is arranged to comprise between 100 and 600 (preferably 150 und 300) milligrams of pregabalin to be administered during one or several (preferably two) intakes per day e.g, in doses of 100, 200, 300, 450 or 600 mg of pregabalin or any dose in between in the composition.

A further aspect of the invention relates to the crystalline form either as a salt or as a co-crystal according to the invention for use in the treatment of pain, preferably neuropathic pain, fibromyalgia or spinal cord injury, or of generalized anxiety disorder. Preferably this use is provided for in form of a medicament or of a pharmaceutical composition according to the invention as described above.

The pain mentioned above may also be chronic pain, but also severe to moderate pain.

A further aspect of the invention relates to a medicament or pharmaceutical composition according to the invention for the treatment of pain, preferably neuropathic pain, fibromyalgia or spinal cord injury, or of generalized anxiety disorder.

A related further aspect of the invention is aimed at the use of the crystalline form of pregabalin with at least one co-former according to the invention as described above in the manufacture of a medicament for the treatment of pain, preferably neuropathic pain, fibromyalgia or spinal cord injury, or of generalized anxiety disorder. The pain mentioned

above may also be chronic pain, but also severe to moderate pain.

Another object of the current invention is a method of treatment of pain, preferably neuropathic pain, fibromyalgia or spinal cord injury, or of generalized anxiety disorder, by providing to a patient in need thereof a sufficient amount of the crystalline form of pregabalin with at least one co-former, or the medicament (or pharmaceutical composition) according to the invention as described above. The pain mentioned above may also be chronic pain, but also severe to moderate pain.

The pain mentioned above may be acute pain, chronic pain, neuropathic pain, hyperalgesia, allodynia or cancer pain, including diabetic neuropathy, fibromyalgia or osteoarthritis; as well as severe to moderate pain; including also rheumatoid arthritis, ankylosing spondylitis, sciatica and frozen shoulder. The pain mentioned above may also be acute pain, chronic pain (acute and chronic pain), neuropathic pain, nociceptive pain (visceral and/or somatic pain), mild and severe to moderate pain, hyperalgesia, pain related to central sensitization, allodynia or cancer pain, including diabetic neuropathy or diabetic peripheral neuropathy and osteoarthritis, fibromyalgia; rheumatoid arthritis, ankylosing spondylitis, frozen shoulder or sciatica.

"Sciatica" or "sciatic neuritis" is defined herein as a set of symptoms including pain that derive from irritation of the sciatic nerve or its roots,

"Frozen shoulder" or "adhesive capsulitis" is defined herein as a symptom wherein the connective tissue surrounding the shoulder joint or the shoulder capsule itself is causing chronic pain, becoming inflamed and stiff.

"Ankylosing spondylitis" or "Morbus Bechterew" is a chronic, inflammatory arthritis and autoimmune disease. It mainly affects joints in the spine and the sacroilium in the pelvis, causing eventual fusion of the spine.

"Pain related to central sensitization" / "central pain syndrome" is defined within this application as a neurological condition caused by damage to or dysfunction of the central nervous system (CNS), which includes the brain, brainstem, and spinal cord. This syndrome can *inter alia* be caused by stroke, multiple sclerosis, tumors, epilepsy, brain or spinal cord trauma, or Parkinson's disease.

"Nociceptive pain " is defined as a type of pain caused by activation of nociceptors. It can be divided into somatic and visceral pain. "Visceral pain" is pain generally originating from the organs, whereas "(deep) somatic pain" originates from ligaments, tendons, bones, blood vessels, fasciae and muscles.

A further aspect of the invention relates to a process for the production of a crystalline form according to the invention comprising the steps of:

(a) dissolving or suspending pregabalin and co-former in a solvent or mixture of solvents,
(b) optionally mixing or stirring the solution or suspension,
(c) optionally before, during or after step (b) cooling or keeping the mixed solution/suspension to or at ambient temperature or below;
(d) optionally filtering-off and/or washing the resulting solid with a solvent, and
(e) drying the solid optionally at ambient temperature and/or vacuum.

The present invention is illustrated below with the help of the following examples and illustrated by the following figures. These are given solely by way of example and do not limit the invention.

**<u>Brief description of the figures:</u>**

**<u>Figure 1:</u>**

***Brief description of the figures:***

<u>**Figure 1:**</u> Differential scanning calorimetry (DSC) analysis of (*S*)-pregabalin - L-(+)-tartaric acid (1:1) **(EXAMPLE 1)**
<u>**Figure 2:**</u> Thermogravimetric analysis (TGA) of (*S*)-pregabalin - L-(+)-tartaric acid (1:1) **(EXAMPLE 1)**
<u>**Figure 3:**</u> X-ray powder diffraction (XRPD) pattern of (*S*)-pregabalin - L-(+)-tartaric acid (1:1) **(EXAMPLE 1)**
<u>**Figure 4:**</u> Differential scanning calorimetry (DSC) analysis of (*S*)-pregabalin - L-(+)-tartaric acid - $H_2O$ (1:1:1) (salt) **(EXAMPLE 2)**
<u>**Figure 5:**</u> Thermogravimetric analysis (TGA) of (*S*)-pregabalin - L-(+)-tartaric acid - $H_2O$ (1:1:1) (salt) **(EXAMPLE 2)**
<u>**Figure 6:**</u> X-ray powder diffraction (XRPD) pattern of (*S*)-pregabalin - L-(+)-tartaric acid - $H_2O$ (1:1:1) (salt) **(EXAMPLE 2)**
<u>**Figure 7:**</u> Crystal structure of (*S*)-pregabalin - L-(+)-tartaric acid - $H_2O$ (1:1:1) (salt) **(EXAMPLE 2)**
<u>**Figure 8:**</u> Differential scanning calorimetry (DSC) of (*S*)-pregabalin - L-malic acid (1:1) (salt) **(EXAMPLE 3)**
<u>**Figure 9:**</u> Thermogravimetric analysis (TGA) of (*S*)-pregabalin - L-malic acid (1:1) (salt) **(EXAMPLE 3)**

**Figure 10:** X-ray powder diffraction (XRPD) pattern of (*S*)-pregabalin - L-malic acid (1:1) (salt) **(EXAMPLE 3)**
**Figure 11:** Crystal structure of (*S*)-pregabalin - L-malic acid (1:1) (salt) **(EXAMPLE 3)**
**Figure 12:** Differential scanning calorimetry (DSC) analysis of (*S*)-pregabalin - maleic acid (1:1) (salt) **(EXAMPLE 4)**
**Figure 13:** Thermogravimetric analysis (TGA) of (*S*)-pregabalin - maleic acid (1:1) (salt) **(EXAMPLE 4)**
**Figure 14:** X-ray powder diffraction (XRPD) pattern of (*S*)-pregabalin - maleic acid (1:1) (salt) **(EXAMPLE 4)**
**Figure 15:** Crystal structure of (*S*)-pregabalin - maleic acid (1:1) (salt) **(EXAMPLE 4)**
**Figure 16:** Differential scanning calorimetry (DSC) analysis of (*S*)-pregabalin - benzoic acid (1:1) (co-crystal) **(EXAMPLE 5)**
**Figure 17:** Thermogravimetric analysis (TGA) of (*S*)-pregabalin - benzoic acid (1:1) (co-crystal) **(EXAMPLE 5)**
**Figure 18:** X-ray powder diffraction (XRPD) pattern of (*S*)-pregabalin - benzoic acid (1:1) (co-crystal) **(EXAMPLE 5)**
**Figure 19:** Crystal structure of (*S*)-pregabalin - benzoic acid (1:1) (co-crystal) **(EXAMPLE 5)**
**Figure 20:** Differential scanning calorimetry (DSC) analysis of (*S*)-pregabalin - L-proline - HCl (1:1:1) (co-crystal) **(EXAMPLE 6)**
**Figure 21:** Thermogravimetric analysis (TGA) of (*S*)-pregabalin - L-proline - HCl (1:1:1) (co-crystal) **(EXAMPLE 6)**
**Figure 22:** X-ray powder diffraction (XRPD) pattern of (*S*)-pregabalin - L-proline - HCl (1:1:1) (co-crystal) **(EXAMPLE 6)**
**Figure 23:** Differential scanning calorimetry (DSC) analysis of (*S*)-pregabalin - maleic acid - $H_2O$ (1:1:0.5) **(EXAMPLE 7)**
**Figure 24:** Thermogravimetric analysis (TGA) of (*S*)-pregabalin - maleic acid - $H_2O$ (1:1:0.5) **(EXAMPLE 7)**
**Figure 25:** X-ray powder diffraction (XRPD) pattern of (*S*)-pregabalin - maleic acid - $H_2O$ (1:1:0.5) **(EXAMPLE 7)**
**Figure 26:** Crystal structure of (*S*)-pregabalin - maleic acid - $H_2O$ (1:1:0.5) **(EXAMPLE 7)**
**Figure 27:** Differential scanning calorimetry (DSC) analysis of (*S*)-pregabalin - caprylic acid (1:1) **(EXAMPLE 8)**
**Figure 28:** Thermogravimetric analysis (TGA) of (*S*)-pregabalin - caprylic acid (1:1) (co-crystal) **(EXAMPLE 8)**
**Figure 29:** X-ray powder diffraction (XRPD) pattern of (*S*)-pregabalin - caprylic acid (1:1) (co-crystal) **(EXAMPLE 8)**
**Figure 30:** Crystal structure of (*S*)-pregabalin - caprylic acid (1:1) (co-crystal) **(EXAMPLE 8)**
**Figure 31:** Differential scanning calorimetry (DSC) analysis of (*S*)-pregabalin - capric acid (1:1) (co-crystal) **(EXAMPLE 9)**
**Figure 32:** Thermogravimetric analysis (TGA) of (*S*)-pregabalin - capric acid (1:1) (co-crystal) **(EXAMPLE 9)**
**Figure 33:** X-ray powder diffraction (XRPD) pattern of (*S*)-pregabalin - capric acid (1:1) (co-crystal) **(EXAMPLE 9)**
**Figure 34:** Crystal structure of (*S*)-pregabalin - capric acid (1:1) (co-crystal) **(EXAMPLE 9)**
**Figure 35:** Differential scanning calorimetry (DSC) analysis of (*S*)-pregabalin - maleic acid anhydrous (1:1) **(EXAMPLE 10)**
**Figure 36:** Thermogravimetric analysis (TGA) of (*S*)-pregabalin - maleic acid **anhydrous** (1:1) **(EXAMPLE 10)**
**Figure 37:** X-ray powder diffraction (XRPD) pattern of (*S*)-pregabalin - maleic acid **anhydrous** (1:1) **(EXAMPLE 10)**
**Figure 38:** Differential scanning calorimetry (DSC) analysis of (*S*)-pregabalin - lauric acid (1:1) (co-crystal) **(EXAMPLE 11)**
**Figure 39:** Thermogravimetric analysis (TGA) of (*S*)-pregabalin - lauric acid (1:1) (co-crystal) **(EXAMPLE 11)**
**Figure 40:** X-ray powder diffraction (XRPD) pattern of (*S*)-pregabalin - lauric acid (1:1) (co-crystal) **(EXAMPLE 11)**
**Figure 41:** Crystal structure of (*S*)-pregabalin - lauric acid (1:1) (co-crystal) **(EXAMPLE 11)**

As a general remark, hydrogen atoms in crystal structure diagrams have been omitted for clarity. The program used: *Mercury* 2.2 - C. F. Macrae, I. J. Bruno, J. A. Chisholm, P. R. Edgington, P. McCabe, E. Pidcock, L. Rodriguez-Monge, R. Taylor, J. van de Streek and P. A. Wood, J. Appl. Cryst. 2008, 41, 466-470.

## EXAMPLES

In general, the new crystalline forms of pregabalin according to the invention were tested to find out their hygroscopicity and solubility. Hygroscopicity of solid state pregabalin salts and co-crystals was tested under storage at ambient and humidity (40 C, 85-95% RH) conditions. Specifically, co-crystal of pregabalin-L-proline·HCl was prepared as a solid crystalline material, which is well soluble in water, but was found to have a hygroscopic tendencies, particularly at humid conditions, to such an extent that it liquefied. However, co-crystal of pregabalin-lauric acid resulted in a non-hygroscopic crystalline form.

Solubility of the formed salts was also tested. All isolated solid state pregabalin salts were soluble in water in the desired concentration range. The solubility of most of the pregabalin salts was lower than that of the free base.

**Example 1: (S)-Pregabalin - L-(+)-tartaric acid (1:1) (co-crystal or salt)**

Evaporation from ethanol and using a slurry in diethyl ether

To a round-bottom flask equipped with magnetic stirrer containing (*S*)-pregabalin (41 mg, 0.26 mmol) and L-(+)-tartaric acid (39 mg, 0.26 mmol, 1.0 eq), was added ethanol (1 mL). The resultant solution was concentrated under vacuum on a rotary evaporator until dryness. Then, to this dried solid was added diethyl ether (0.5 mL) and the resultant suspension was stirred at room temperature for 3 h and at 0-5 °C for 1 h. The solid was filtered with a sintered funnel (porosity 4), washed with cold diethyl ether (2 x 0.5 mL) and dried under vacuum at room temperature to give (*S*)-pregabalin - L-(+)-tartaric acid (1:1) (co-crystal or salt) as a white solid (64 mg, 82%).

**$^1$H NMR**

Proton nuclear magnetic resonance analysis was recorded in deuterated methanol (d4-MeOH) in a Varian Mercury 400 spectrometer, equipped with a broadband probe ATB 1 H/19F/X of 5 mm. Spectrum was acquired dissolving 5-10 mg of sample in 0.6 mL of deuterated solvent.
$^1$H NMR spectrum in d4-MeOD at 400 MHz shows peaks at 4.44 (s, 2H); 2.96 (dd, *J* = 5.1 Hz, *J* = 12.9 Hz, 1 H); 2.91 (dd, *J* = 7.0 Hz, *J* = 12.9 Hz, 1 H); 2.45 (dd, *J* = 4.7 Hz, *J* = 16.0 Hz, 1 H); 2.34 (dd, *J* = 7.4 Hz, *J* = 16.0 Hz, 1 H); 2.21-2.09 (m, 1 H); 1.75-1.63 (m, 1 H); 1.32-1.17 (m, 2H); 0.95 (d, *J* = 6.6 Hz, 3H); 0.93 (d, *J* = 6.6 Hz, 3H) ppm.

**IR**

FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 $cm^{-1}$.
The sample (KBr pellets) shows a Fourier Transform Infra Red spectrum with absorption bands at 3498.0 (s), 3321.1 (s, br), 3154.9 (s), 2956.4 (s), 1699.7 (s), 1498.0 (m), 1410.7 (m), 1265.3 (m), 1130.1 (m), 1068.4 (s), 679.0 (s), 484.2 (m) $cm^{-1}$.

**DSC**

DSC analysis was recorded with a Mettler DSC822$^e$. A sample of 1.5240 mg was weighed into a 40 μL aluminium crucible with a pinhole lid and was heated, under nitrogen (50 mL/min), at 10 °C/min from 30 to 200 °C.

The novel type of crystal of the present invention is characterized in that the endothermic sharp peak corresponding to the melting point has an onset at 127.70 °C (fusion enthalpy -124.13 J/g), measured by DSC analysis (10 °C/min) (see Figure 1).

**TGA**

Thermogravimetric analysis was recorded in a thermogravimetric analyzer Mettler TGA/SDTA851$^e$. A sample of 7.6616 mg was weighed into a 70 μL alumina crucible with a pinhole lid and was heated at 10 °C/min from 30 to 200 °C, under nitrogen (50 mL/min).
The TGA analysis of the crystalline according to the invention shows no significant loss at temperatures lower than the melting point (see Figure 2).

**XRPD**

XRPD analysis was performed using a Philips X'Pert diffractometer with Cu $K_\alpha$, radiation in Bragg-Brentano geometry. The system is equipped with a monodimensional, real time multiple strip detector. The diffractogram was recorded from 3° to 40° (2θ) at a scan rate of 8.8° per minute (see figure 3).
List of selected peaks (only peaks with relative intensity greater than or equal to 1 % are indicated):

| 2θ (°) | d (Å) | I (%) |
|---|---|---|
| 7.08 | 12.48 | 100 |
| 14.16 | 6.25 | 13 |

(continued)

| 2θ (°) | d (Å) | I (%) |
|---|---|---|
| 17.13 | 5.17 | 3 |
| 17.51 | 5.06 | 11 |
| 20.60 | 4.31 | 3 |
| 20.81 | 4.26 | 2 |
| 21.29 | 4.17 | 13 |
| 21.96 | 4.04 | 3 |
| 23.84 | 3.73 | 5 |
| 24.03 | 3.70 | 2 |
| 24.81 | 3.58 | 1 |
| 25.08 | 3.55 | 3 |
| 25.36 | 3.51 | 1 |
| 26.01 | 3.42 | 2 |
| 27.43 | 3.25 | 1 |

**Example 2: (*S*)-pregabalin - L-(+)-tartaric acid - $H_2O$ (1:1:1) (salt)**

Precipitation in isopropanol/water (0.5 g scale-up)

To a round-bottom flask equipped with magnetic stirrer containing (*S*)-pregabalin (300 mg, 1.88 mmol), was added isopropanol (3 mL) and was stirred for a few minutes. Then, a hot aqueous solution of L-(+)-tartaric acid (565 mg, 3.76 mmol, 2 eq., in 0.5 mL) was added dropwise. The resultant solution was stirred at room temperature for 3 h and a white precipitate was formed. The resultant suspension was stirred at 0-5 °C overnight. The solid was filtered with a sintered funnel (porosity 4), washed with cold isopropanol (1 x 0.4 mL) and dried under vacuum at room temperature to give salt of (*S*)-pregabalin - L-(+)-tartaric acid-water (1:1:1) as a white solid (546.0 mg, 89%).

Precipitation in isopropanol/water

To an assay tube equipped with magnetic stirrer containing (*S*)-pregabalin (40 mg, 0.25 mmol), was added isopropanol (0.3 mL) and was stirred for a few minutes. Then, an aqueous solution of L-(+)-tartaric acid (38 mg, 0.25 mmol, 1 eq., in 0.1 mL) was added dropwise. The resultant solution was stirred at room temperature for 3 h and a white precipitate was formed. The resultant suspension was stirred at 0-5 °C for 4 h. The solid was filtered with a sintered funnel (porosity 4), washed with cold mixture isopropanol:water (3:1, 1 x 0.3 mL) and dried under vacuum at room temperature to give salt of (*S*)-pregabalin - L-(+)-tartaric acid-water (1:1:1) as a white solid (34 mg, 41 %).

**$^1$H NMR**

Proton nuclear magnetic resonance analysis was recorded in deuterated methanol (d4-MeOD) in a Varian Mercury 400 spectrometer, equipped with a broadband probe ATB 1 H/19F/X of 5 mm. Spectrum was acquired dissolving 5-10 mg of sample in 0.6 mL of deuterated solvent. $^1$H NMR spectrum in d4-MeOD at 400 MHz shows peaks at 4.44 (s, 2H); 2.96 (dd, *J* = 5.1 Hz, *J* = 12.9 Hz, 1 H); 2.91 (dd, *J* = 7.0 Hz, *J* = 12.9 Hz, 1 H); 2.45 (dd, *J* = 4.7 Hz, *J* = 16.0 Hz, 1 H); 2.34 (dd, *J* = 7.4 Hz, *J* = 16.0 Hz, 1 H); 2.21-2.09 (m, 1 H); 1.75-1.63 (m, 1 H); 1.32-1.17 (m, 2H); 0.95 (d, *J* = 6.6 Hz, 3H); 0.93 (d, *J* = 6.6 Hz, 3H) ppm.

**IR**

FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 $cm^{-1}$.

The sample (KBr pellets) shows a Fourier Transform Infra Red spectrum with absorption bands at 3451.8 (s),

3320.8 (s), 3271.5 (s), 3099.3 (s, br), 2960.3 (s), 1735.9 (s), 1584.3 (m), 1408.0 (s), 1305.4 (m), 1262.6 (m), 1134.5 (m), 1076.5 (s), 904.1 (m), 679.1 (s) $cm^{-1}$.

## DSC

DSC analysis was recorded with a Mettler DSC822$^{e}$. A sample of 1.1960 mg was weighed into a 40 μL aluminium crucible with a pinhole lid and was heated, under nitrogen (50 mL/min), at 10 °C/min from 30 to 200 °C.

The novel type of crystal of this example is characterized for two endothermic peaks corresponding to the water loss at an onset 61.38 °C (fusion enthalpy -87.72 J/g) and the melting point of the anhydrous form at an onset 102.79 °C (fusion enthalpy -15.43 J/g). Next, one exothermic sharp peak corresponding to the further crystallization of form of example 1 and one endothermic sharp peak corresponding to the melting point of the form of example 1 at an onset 123.32 °C (fusion enthalpy -84.84 J/g) were observed (see Figure 4).

## TGA

Thermogravimetric analysis was recorded in a thermogravimetric analyzer Mettler TGA/SDTA851$^{e}$. A sample of 7.8924 mg was weighed into a 70 μL alumina crucible with a pinhole lid and was heated at 10 °C/min from 30 to 200 °C, under nitrogen (50 mL/min).

The TGA analysis of the crystalline form according to the invention shows a weight loss at temperatures lower than the melting point. It corresponds to water loss (3.4%) (see Figure 5).

## XRPD

XRPD analysis was performed using a Philips X'Pert diffractometer with Cu $K_{\alpha}$, radiation in Bragg-Brentano geometry. The system is equipped with a monodimensional, real time multiple strip detector. The diffractogram was recorded from 3° to 40° (2θ) at a scan rate of 8.8° per minute (see Figure 6).

List of selected peaks (only peaks with relative intensity greater than or equal to 1 % are indicated):

| 2θ (°) | d (Å) | I (%) | | 2θ (°) | d (Å) | I (%) |
|---|---|---|---|---|---|---|
| 9.16 | 9.64 | 100 | | 25.35 | 3.50 | 8 |
| 11.95 | 7.40 | 53 | | 26.00 | 3.42 | 1 |
| 14.04 | 6.30 | 19 | | 27.06 | 3.29 | 10 |
| 14.23 | 6.22 | 25 | | 28.19 | 3.16 | 9 |
| 15.26 | 5.80 | 14 | | 28.71 | 3.10 | 2 |
| 15.99 | 5.54 | 11 | | 29.47 | 3.02 | 2 |
| 17.86 | 4.96 | 13 | | 30.98 | 2.88 | 6 |
| 18.41 | 4.81 | 12 | | 32.16 | 2.78 | 9 |
| 19.44 | 4.56 | 8 | | 33.11 | 2.70 | 1 |
| 19.90 | 4.46 | 50 | | 34.93 | 2.56 | 1 |
| 20.52 | 4.32 | 51 | | 36.11 | 2.48 | 4 |
| 21.88 | 4.06 | 7 | | 36.45 | 2.46 | 5 |
| 23.97 | 3.71 | 28 | | 37.39 | 2.40 | 4 |
| 24.67 | 3.60 | 32 | | 39.06 | 2.30 | 1 |
| 24.76 | 3.60 | 26 | | | | |

## Single crystal X-ray diffraction

The crystal structure of this salt has been determined from single crystal X-ray diffraction data. The colourless irregular plate used (0.27 × 0.25 × 0.10 mm) was obtained from the vapour diffusion of a solution in ethanol of (*S*)-pregabalin and L-tartaric acid in a *tert*-butyl methyl ether atmosphere.

Analysis was performed at room temperature using a Bruker Smart Apex diffractometer with graphite monochromated Mo $K_\alpha$ radiation equipped with a CCD detector. Data were collected using phi and omega scans (program used: SMART 5.6). No significant decay of standard intensities was observed. Data reduction (Lorentz and polarization corrections) and absorption correction were applied (program used: SAINT 5.0).

The structure was solved with direct methods and least-squares refinement of $F_o^2$ against all measured intensities was carried out (program used: SHELXTL-NT 6.1). All non-hydrogen atoms were refined with anisotropic displacement parameters.

Relevant structural data:

| | |
|---|---|
| Crystal system | Monoclinic |
| Space group | $P2_1$ |
| a (Å) | 7.4466(6) |
| b (Å) | 11.6104(10) |
| c (Å) | 9.7498(8) |
| β (°) | 98.137(2) |
| Volume ($Å^3$) | 834.5(2) |
| Z | 2 |
| D calc. ($Mg/m^3$) | 1.303 |
| N. of refl. | 3359 |
| Refl. with $I > 2\sigma(I)$ | 2940 |
| R ($I > 2\sigma(I)$) | 0.0412 |

The unit cell contents of this crystal structure of salt of (*S*)-pregabalin - L-(+)-tartaric acid-water (1:1:1) are depicted in Figure 7 (hydrogen atoms have been omitted for clarity; program used: *Mercury* 2.3, C.F. Macrae, I.J. Bruno, J.A. Chisholm, P.R. Edgington, P. McCabe, E. Pidcock, L. Rodriguez-Monge, R. Taylor, J. van de Streek and P.A. Wood, J. Appl. Cryst., 41, 2008, 466-470).

Positions of peaks in XRPD diffractogram simulated from single crystal data are almost identical to those in the experimental one presented above.

**Example 3: (*S*)-Pregabalin - L-malic acid (1:1) (salt)**

<u>Crystallization in IPA/heptane</u>

To an assay tube equipped with magnetic stirrer containing (*S*)-pregabalin (37 mg, 0.23 mmol) and L-malic acid (62 mg, 0.46 mmol, 2.0 eq), was added IPA (0.4 mL). The resultant solution was diluted with heptane (0.9 mL) and stirred at 0-5 °C for 2 h. A white precipitate was formed. The solid was filtered with a sintered funnel (porosity 4) and dried under vacuum at room temperature to give salt of (S)-pregabalin - L-malic acid (1:1) as a white powder (22 mg, 32% yield).

<u>Crystallization in EtOH/heptane</u>

To an assay tube equipped with magnetic stirrer containing (S)-pregabalin (37 mg, 0.23 mmol) and L-malic acid (63 mg, 0.47 mmol, 2.0 eq), was added EtOH (0.4 mL). The resultant solution was diluted with heptane (0.9 mL) and stirred at room temperature overnight. A white precipitate was formed. The solid was filtered with a sintered funnel (porosity 4) and dried under vacuum at room temperature to give form salt of (*S*)-pregabalin - L-malic acid (1:1) as a white powder (55 mg, 81% yield).

<u>Precipitation in acetone</u>

To an assay tube equipped with magnetic stirrer containing (*S*)-pregabalin (37 mg, 0.23 mmol) and L-malic acid (70 mg, 0.52 mmol, 2.3 eq), was added acetone (1.1 mL). The resultant solution was stirred at 0-5 °C for 2 h. A white precipitate was formed. The solid was filtered with a sintered funnel (porosity 4), washed with cold acetone (0.4

mL) and dried under vacuum at room temperature to give salt of (S)-pregabalin - L-malic acid (1:1) as a white powder (32 mg, 47% yield).

Crystallization in EtOH/heptane (1g scale-up)

To a round-bottom flask equipped with magnetic stirrer containing (*S*)-pregabalin (559 mg, 3.51 mmol) and L-malic acid (941 mg, 7.02 mmol, 2.0 eq), was added EtOH (6 mL). The resultant solution was diluted with heptane (13.5 mL) and stirred at room temperature overnight. A white precipitate was formed. The solid was filtered with a sintered funnel (porosity 4), washed with heptane (3 mL) and dried under vacuum at room temperature to give salt of (S)-pregabalin - L-malic acid (1:1) as a white powder (888 mg, 86% yield).

**$^1$H NMR**

Proton nuclear magnetic resonance analysis was recorded in deuterated methanol (d4-MeOH) in a Varian Mercury 400 spectrometer, equipped with a broadband probe ATB 1H/19F/X of 5 mm. Spectrum was acquired dissolving 5-10 mg of sample in 0.6 mL of deuterated solvent.

$^1$H NMR spectrum in d4-MeOD at 400 MHz shows peaks at 4.35 (dd, *J* = 5.1 Hz, *J* = 7.4 Hz, 1 H); 2.96 (dd, *J* = 4.7 Hz, *J* = 12.9 Hz, 1 H); 2.90 (dd, *J* = 7.4 Hz, *J* = 12.9 Hz, 1 H); 2.79 (dd, *J* = 5.1 Hz, *J* = 16.0 Hz, 1H); 2.56 (dd, *J* = 7.4 Hz, *J* = 16.0 Hz, 1H); 2.44 (dd, *J* = 4.7 Hz, *J* = 16.0 Hz, 1H); 2.33 (dd, *J*=7.8Hz, *J*=16.0Hz, 1 H); 2.21-2.09 (m, 1 H); 1.76-1.62 (m, 1 H); 1.32-1.16 (m, 2H); 0.94 (d, *J* = 6.6 Hz, 3H); 0.92 (d, *J* = 6.6 Hz, 3H) ppm.

**$^{13}$C-CPMAS NMR**

CPMAS solid state nuclear magnetic resonance analysis was recorded in a Bruker 400MHz WB spectrometer at 20 °C using a 1 H/BB probe. Sample was carefully packed in a 4-mm diameter cylindrical zirconia rotor with Kel-F end-caps. The operating conditions involved 4.5 μs 90° 1 H pulses and decoupling field strength of 83.3 kHz by SPINAL64 sequence. $^{13}$C spectra was externally referenced to adamantane sample and then the chemical shifts were recalculated to the $Me_4Si$. The typical acquisition parameters for $^{13}$C-CPMAS were: spectral width, 50 kHz; recycle delay, 2 s; acquisition time, 40 ms; contact time, 2.5 ms; and spin rate, 10 kHz.

$^{13}$C-CPMAS NMR: $\delta$ = 18.9; 23.5; 30.3; 32.7; 38.6; 42.5; 43.8; 72.8; 178.6; 180.8; 182.7 ppm.

**IR**

FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 $cm^{-1}$.

The sample (KBr pellets) shows a Fourier Transform Infra Red spectrum with absorption bands at 3388.3 (s), 3199.7 (m), 2952.6 (m), 2908.5 (m), 1703.1 (m), 1522.8 (m), 1414.7 (m), 1306.6 (m), 1257.8 (m), 1204.0 (m), 1081.1 (m), 950.4 (m), 884.2 (m), 664.5 (m), 567.8 (m), 465.8 (m) $cm^{-1}$.

**DSC**

DSC analysis was recorded with a Mettler DSC822$^e$. A sample of 4.2790 mg was weighed into a 40 μL aluminium crucible with a pinhole lid and was heated, under nitrogen (50 mL/min), at 10 °C/min from 30 to 200 °C.

The novel type of crystal of this example is characterized in that the endothermic sharp peak corresponding to the melting point has an onset at 80.58 °C (fusion enthalpy -103.82 J/g), measured by DSC analysis (10 °C/min) (see Figure 8).

**TGA**

Thermogravimetric analysis was recorded in a thermogravimetric analyzer Mettler TGA/SDTA851$^e$. A sample of 9.1815 mg was weighed into a 70 μL alumina crucible with a pinhole lid and was heated at 10 °C/min from 30 to 200 °C, under nitrogen (50 mL/min).

The TGA analysis of this crystalline form according to the invention shows no significant loss at temperatures lower than the melting point (see Figure 9).

**XRPD**

XRPD analysis was performed using a Philips X'Pert diffractometer with Cu $K_\alpha$ radiation in Bragg-Brentano geometry. The system is equipped with a monodimensional, real time multiple strip detector. The diffractogram was recorded from 3° to 40° (2θ) at a scan rate of 8.8° per minute (see Figure 10).

List of selected peaks (only peaks with relative intensity greater than or equal to 1 % are indicated):

| 2θ (°) | d (Å) | I (%) |
|---|---|---|
| 6.65 | 13.29 | 100 |
| 13.32 | 6.65 | 17 |
| 15.36 | 5.77 | 3 |
| 16.84 | 5.26 | 6 |
| 17.74 | 5.00 | 13 |
| 19.33 | 4.59 | 8 |
| 20.05 | 4.43 | 6 |
| 20.40 | 4.35 | 5 |
| 21.28 | 4.18 | 1 |
| 23.25 | 3.83 | 22 |
| 25.50 | 3.49 | 3 |
| 26.09 | 3.42 | 3 |
| 26.46 | 3.37 | 5 |
| 26.84 | 3.32 | 3 |
| 27.09 | 3.29 | 1 |
| 28.13 | 3.17 | 3 |
| 28.81 | 3.10 | 1 |

**Single crystal X-ray diffraction**

The crystal structure of salt of (*S*)-pregabalin - L-malic acid (1:1) has been determined from single crystal X-ray diffraction data. The colourless prismatic crystal used (0.32 × 0.17 × 0.13 mm) was obtained from the vapour diffusion of a solution in IPA of (*S*)-pregabalin and L-malic acid in a cyclohexane atmosphere.

Analysis was performed at room temperature using a Bruker Smart Apex diffractometer with graphite monochromated Mo $K_\alpha$ radiation equipped with a CCD detector. Data were collected using phi and omega scans (program used: SMART 5.6). No significant decay of standard intensities was observed. Data reduction (Lorentz and polarization corrections) and absorption correction were applied (program used: SAINT 5.0).

The structure was solved with direct methods and least-squares refinement of $F_o^2$ against all measured intensities was carried out (program used: SHELXTL-NT 6.1). All non-hydrogen atoms were refined with anisotropic displacement parameters.

Relevant structural data:

| | |
|---|---|
| Crystal system | Orthorhombic |
| Space group | $P2_12_12_1$ |
| a (Å) | 7.5609(6) |
| b (Å) | 7.5959(6) |
| c (Å) | 26.478(2) |
| Volume (Å$^3$) | 1520.7(4) |

(continued)

| Crystal system | Orthorhombic |
|---|---|
| Z | 4 |
| D calc. ($Mg/m^3$) | 1.281 |
| N. of refl. | 3747 |
| Refl. with I > 2σ(I) | 2828 |
| R (I > 2σ(I)) | 0.0471 |

The unit cell contents of this crystal structure are depicted in Figure 11 (hydrogen atoms have been omitted for clarity; program used: *Mercury* 2.3, C.F. Macrae, I.J. Bruno, J.A. Chisholm, P.R. Edgington, P. McCabe, E. Pidcock, L. Rodriguez-Monge, R. Taylor, J. van de Streek and P.A. Wood, J. Appl. Cryst., 41, 2008, 466-470).

Positions of peaks in XRPD diffractogram simulated from single crystal data are almost identical to those in the experimental one presented above.

**Example 4: (*S*)-Pregabalin - maleic acid (1:1) (salt)**

Slurrying in ACN

To an assay tube equipped with magnetic stirrer containing (*S*)-pregabalin (58 mg, 0.36 mmol) and maleic acid (42 mg, 0.36 mmol, 1.0 eq), was added ACN (0.5 mL). The resulting suspension was stirred at room temperature overnight. The solid was filtered with a sintered funnel (porosity 3) and dried under vacuum at room temperature to give salt of (*S*)-pregabalin - maleic acid (1:1) as a white powder (60 mg, 59% yield).

Slurrying in AcOEt

To an assay tube equipped with magnetic stirrer containing (*S*)-pregabalin (58 mg, 0.36 mmol) and maleic acid (42 mg, 0.36 mmol, 1.0 eq), was added AcOEt (0.5 mL). The resulting suspension was stirred at room temperature overnight. The solid was filtered with a sintered funnel (porosity 3), washed with cold AcOEt (0.3 mL) and dried under vacuum at room temperature to give salt of (*S*)-pregabalin - maleic acid (1:1) as a white powder (89 mg, 90% yield).

Wet grinding with AcOiBu (solvent assisted grinding)

To a 2 mL eppendorf containing (*S*)-pregabalin (57 mg, 0.36 mmol), maleic acid (42 mg, 0.36 mmol, 1.0 eq) and three 4 mm stainless steel grinding balls, one drop of AcOiBu was added. The reactor was stirred 45 minutes at a rate of 30 Hz (3 x 15 minutes). The product was dried under vacuum at room temperature overnight. Salt of (*S*)-pregabalin - maleic acid (1:1) was obtained as a white powder in a quantitative yield.

The same experiment was carried out with ACN with the same results.

Slurrying in AcOiBu (1 g scale)

To a round-bottom flask equipped with magnetic stirrer containing (*S*)-pregabalin (566 mg, 3.55 mmol) and maleic acid (434 mg, 3.74 mmol, 1.05 eq), was added AcOiBu (10 mL). The resulting suspension was stirred at room temperature overnight. The solid was filtered with a sintered funnel (porosity 4), washed with AcOiBu (2 x 3 mL) and dried under vacuum at room temperature to give salt of (*S*)-pregabalin - maleic acid (1:1) as a white powder (954 mg, 98% yield).

**$^1$H NMR**

Proton nuclear magnetic resonance analysis was recorded in deuterated methanol (d4-MeOH) in a Varian Mercury 400 spectrometer, equipped with a broadband probe ATB 1 H/19F/X of 5 mm. Spectrum was acquired dissolving 5-10 mg of sample in 0.6 mL of deuterated solvent.

$^1$H NMR spectrum in d4-MeOD at 400 MHz shows peaks at 6.25 (s, 2H); 3.01-2.90 (m, 2H); 2.45 (dd, *J* = 5.5 Hz, *J* = 16.4 Hz, 1 H); 2.38 (dd, *J* = 7.0 Hz, *J* = 16.4 Hz, 1 H); 2.25-2.13 (m, 1 H); 1.76-1.62 (m, 1 H); 1.33-1.19 (m, 2H); 0.95 (d, *J* = 6.6 Hz, 3H); 0.93 (d, *J* = 6.6 Hz, 3H) ppm.

## $^{13}$C-CPMAS NMR

CPMAS solid state nuclear magnetic resonance analysis was recorded in a Bruker 400MHz WB spectrometer at 20 °C using a 1 H/BB probe. Sample was carefully packed in a 4-mm diameter cylindrical zirconia rotor with Kel-F end-caps. The operating conditions involved 4.5 μs 90° 1 H pulses and decoupling field strength of 83.3 kHz by SPINAL64 sequence. $^{13}$C spectrum was externally referenced to adamantane sample and then the chemical shifts were recalculated to the $Me_4Si$. The typical acquisition parameters for $^{13}$C-CPMAS were: spectral width, 50 kHz; recycle delay, 2 s; acquisition time, 40 ms; contact time, 2.5 ms; and spin rate, 10 kHz.

$^{13}$C-CPMAS NMR: δ = 19.7; 21.1; 24.0; 24.4; 25.3; 29.6; 31.1; 37.4; 39.5; 41.5; 42.4; 45.6; 133.9; 135.6; 137.8; 169.8; 170.3; 171.1; 172.5; 173.3; 175.7 ppm.

## IR

FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 $cm^{-1}$.

The sample (KBr pellets) shows a Fourier Transform Infra Red spectrum with absorption bands at 3189.7 (m, br), 2957.3 (m), 1745.6 (m), 1725.1 (m), 1699.8 (m), 1629.1 (m, br), 1498.0 (m), 1242.4 (m), 1195.6 (m), 1140.2 (m), 1055.9 (m), 1002.7 (m), 956.7 (m), 933.0 (m), 891.1 (m), 858.1 (s), 770.5 (m), 652.5 (m) $cm^{-1}$.

## DSC

DSC analysis was recorded with a Mettler DSC822$^e$. A sample of 4.4760 mg was weighed into a 40 μL aluminium crucible with a pinhole lid and was heated, under nitrogen (50 mL/min), at 10 °C/min from 30 to 200 °C.

The novel type of crystal of the present invention is characterized in that the endothermic sharp peak corresponding to the melting point has an onset at 82.53 °C (fusion enthalpy -110.71 J/g), measured by DSC analysis (10 °C/min) (see Figure 12).

## TGA

Thermogravimetric analysis was recorded in a thermogravimetric analyzer Mettler TGA/SDTA851$^e$. A sample of 6.3775 mg was weighed into a 70 μL alumina crucible with a pinhole lid and was heated at 10 °C/min from 30 to 200 °C, under nitrogen (50 mL/min).

The TGA analysis of the crystalline form according to the invention shows no weight loss at temperatures lower than the melting point (see Figure 13).

## XRPD

XRPD analysis was performed using a Philips X'Pert diffractometer with Cu $K_\alpha$ radiation in Bragg-Brentano geometry. The system is equipped with a monodimensional, real time multiple strip detector. The diffractogram was recorded from 3° to 40° (2θ) at a scan rate of 17.6° per minute (see Figure 14).

List of selected peaks (only peaks with relative intensity greater than or equal to 1 % are indicated):

| 2θ (°) | d (Å) | I (%) | | 2θ (°) | d (Å) | I (%) |
|---|---|---|---|---|---|---|
| 4.64 | 19.05 | 100 | | 19.61 | 4.53 | 4 |
| 9.26 | 9.55 | 11 | | 20.29 | 4.38 | 2 |
| 12.14 | 7.29 | 1 | | 20.82 | 4.27 | 1 |
| 13.80 | 6.41 | 1 | | 21.98 | 4.04 | 2 |
| 14.89 | 5.95 | 1 | | 23.77 | 3.74 | 2 |
| 16.33 | 5.43 | 1 | | 24.86 | 3.58 | 1 |
| 17.40 | 5.10 | 3 | | 24.98 | 3.57 | 1 |
| 17.87 | 4.96 | 6 | | 25.63 | 3.47 | 7 |
| 18.40 | 4.82 | 2 | | 26.47 | 3.36 | 4 |

(continued)

| 2θ (°) | d (Å) | I (%) | | 2θ (°) | d (Å) | I (%) |
|---|---|---|---|---|---|---|
| 18.59 | 4.77 | 4 | | 27.20 | 3.28 | 2 |
| 18.76 | 4.73 | 3 | | 29.16 | 3.06 | 1 |

**Single crystal X-ray diffraction**

The crystal structure of salt of (*S*)-pregabalin - maleic acid (1:1) has been determined from single crystal X-ray diffraction data. The colourless plate used (0.40 × 0.31 × 0.08 mm) was obtained from the evaporation of a solution of (*S*)-pregabalin and maleic acid in methyl isobutyl ketone and ethanol.

Analysis was performed at room temperature using a Bruker Smart Apex diffractometer with graphite monochromated Mo $K_\alpha$ radiation equipped with a CCD detector. Data were collected using phi and omega scans (program used: SMART 5.6). No significant decay of standard intensities was observed. Data reduction (Lorentz and polarization corrections) and absorption correction were applied (program used: SAINT 5.0).

The structure was solved with direct methods and least-squares refinement of $F_o^2$ against all measured intensities was carried out (program used: SHELXTL-NT 6.1). All non-hydrogen atoms were refined with anisotropic displacement parameters, except one disordered methyl carbon.

Relevant structural data:

| | |
|---|---|
| Crystal system | Orthorhombic |
| Space group | $P2_12_12_1$ |
| a (Å) | 7.4190(5) |
| b (Å) | 10.2746(7) |
| c (Å) | 38.221(2) |
| Volume (Å$^3$) | 2913.5(6) |
| Z | 8 |
| D calc. (Mg/m$^3$) | 1.255 |
| N. of refl. | 7079 |
| Refl. with I > 2σ(I) | 4344 |
| R(I>2σ(I)) | 0.0554 |

The unit cell contents of this crystal structure are depicted in Figure 15 (hydrogen atoms have been omitted for clarity; program used: *Mercury* 2.3, C.F. Macrae, I.J. Bruno, J.A. Chisholm, P.R. Edgington, P. McCabe, E. Pidcock, L. Rodriguez-Monge, R. Taylor, J. van de Streek and P.A. Wood, J. Appl. Cryst., 41, 2008, 466-470).

Positions of peaks in XRPD diffractogram simulated from single crystal data are almost identical to those in the experimental one presented above.

**Example 5: (*S*)-Pregabalin - benzoic acid (1:1) (co-crystal)**

Grinding (solvent-free grinding)

To a 2 mL eppendorf containing (*S*)-pregabalin (58 mg, 0.36 mmol) and benzoic acid (44 mg, 0.36 mmol, 1.0 eq), three 4 mm stainless steel grinding balls were added. The reactor was stirred 45 minutes at a rate of 30 Hz (3 x 15 minutes). Co-crystal of (*S*)-pregabalin - benzoic acid (1:1) was obtained as a white powder in a quantitative yield.

Wet grinding with water (solvent assisted grinding)

To a 2 mL eppendorf containing (*S*)-pregabalin (60 mg, 0.38 mmol), benzoic acid (46 mg, 0.37 mmol, 1.0 eq) and three 4 mm stainless steel grinding balls, one drop of water was added. The reactor was stirred 45 minutes at a rate of 30 Hz (3 x 15 minutes). The product was dried under vacuum at room temperature overnight. Co-crystal of

(*S*)-pregabalin - benzoic acid (1:1) was obtained as a white powder in a quantitative yield.

Crystallization in IPA/water

To an assay tube equipped with magnetic stirrer containing (*S*)-pregabalin (40 mg, 0.25 mmol) and benzoic acid (60 mg, 0.49 mmol, 2.0 eq), was added a mixture of IPA/water (60/40 vol/vol) (0.3 mL). The resultant suspension was heated until complete dissolution, cooled down to room temperature and seeded with co-crystal obtained by grinding process. A white precipitate was formed and the slurry was stirred at room temperature overnight. The mixture was stirred at 0-5 °C for 20 min. The solid was filtered with a sintered funnel (porosity 3) and dried under vacuum at room temperature to give co-crystal of (*S*)-pregabalin - benzoic acid (1:1) as a white powder (31 mg, 44% yield).

Crystallization in IPA/water (700 mg scale)

To a round-bottom flask equipped with magnetic stirrer containing (*S*)-pregabalin (395 mg, 2.48 mmol) and benzoic acid (605 mg, 4.95 mmol, 2.0 eq), was added a mixture of IPA/water (60/40 vol/vol) (3.5 mL). The resultant suspension was heated until complete dissolution, cooled down to room temperature and seeded with co-crystal obtained by grinding process. A white precipitate was formed and the slurry was stirred at room temperature for 15 min. Then, the mixture was stirred at 0-5 °C for 30 min. The solid was filtered with a sintered funnel (porosity 3) and dried under vacuum at room temperature for 1 h. The product was washed with TBME (2 x 2mL) to give co-crystal (S)-pregabalin - benzoic acid (1:1) as a white powder (424 mg, 61% yield).

**$^{1}$H NMR**

Proton nuclear magnetic resonance analysis was recorded in deuterated methanol (d4-MeOH) in a Varian Mercury 400 spectrometer, equipped with a broadband probe ATB 1 H/19F/X of 5 mm. Spectrum was acquired dissolving 5-10 mg of sample in 0.6 mL of deuterated solvent.

$^{1}$H NMR spectrum in d4-MeOD at 400 MHz shows peaks at 8.03-7.97 (m, 2H); 7.57-7.51 (m, 1 H); 7.47-7.40 (m, 2H); 2.96 (dd, *J* = 3.9 Hz, *J* = 12.9 Hz, 1 H); 2.85 (dd, *J* = 7.8 Hz, *J* = 12.9 Hz, 1 H); 2.44 (dd, *J* = 3.9 Hz, *J* = 16.0 Hz, 1 H); 2.28 (dd, *J* = 8.2 Hz, *J* = 16.0 Hz, 1 H); 2.16-2.03 (m, 1 H); 1.76-1.63 (m, 1 H); 1.31-1.14 (m, 2H); 0.94 (d, *J* = 6.6 Hz, 3H); 0.92 (d, *J* = 6.6 Hz, 3H) ppm.

**$^{13}$C-CPMAS NMR**

CPMAS solid state nuclear magnetic resonance analysis was recorded in a Bruker 400MHz WB spectrometer at 20 °C using a 1 H/BB probe. Sample was carefully packed in a 4-mm diameter cylindrical zirconia rotor with Kel-F end-caps. The operating conditions involved 4.5 μs 90° 1 H pulses and decoupling field strength of 83.3 kHz by SPINAL64 sequence. $^{13}$C spectra was externally referenced to adamantane sample and then the chemical shifts were recalculated to the $Me_4Si$. The typical acquisition parameters for $^{13}$C-CPMAS were: spectral width, 50 kHz; recycle delay, 2 s; acquisition time, 40 ms; contact time, 2.5 ms; and spin rate, 10 kHz.

$^{13}$C-CPMAS NMR: δ = 19.4; 24.5; 25.5; 31.5; 38.5; 41.3; 42.4; 128.1; 129.8; 131.8; 132.9; 167.7; 181.5 ppm.

**IR**

FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 $cm^{-1}$.

The sample (KBr pellets) shows a Fourier Transform Infra Red spectrum with absorption bands at 2847.4 (s, br), 2147.4 (m, br), 1722.8 (s), 1643.5 (m), 1550.1 (m, br), 1385.3 (m), 1339.4 (m), 1242.0 (m, br), 1113.8 (s), 1097.8 (s), 1068.8 (s), 1024.2 (s), 874.5 (s), 852.1 (s), 782.8 (s), 707.3 (s), 653.6 (s) $cm^{-1}$.

**DSC**

DSC analysis was recorded with a Mettler DSC822$^{e}$. A sample of 0.4090 mg was weighed into a 40 μL aluminium crucible with a pinhole lid and was heated, under nitrogen (50 mL/min), at 10 °C/min from 30 to 300 °C.

The novel type of crystal of the present invention is characterized in that the endothermic sharp peak corresponding to the melting point has an onset at 128.30 °C (fusion and decomposition enthalpy -277.50 J/g), measured by DSC analysis (10 °C/min) (see Figure 16).

## TGA

Thermogravimetric analysis was recorded in a thermogravimetric analyzer Mettler TGA/SDTA851e. A sample of 3.6686 mg was weighed into a 70 μL alumina crucible with a pinhole lid and was heated at 10 °C/min from 30 to 200 °C, under nitrogen (50 mL/min).

The TGA analysis of this crystalline form according to the invention shows no weight loss at temperatures lower than the melting point. After the melting the compound starts to decompose (see Figure 17).

## XRPD

XRPD analysis was performed using a Philips X'Pert diffractometer with Cu $K_{\alpha}$ radiation in Bragg-Brentano geometry. The system is equipped with a monodimensional, real time multiple strip detector. The diffractogram was recorded from 3° to 40° (2θ) at a scan rate of 17.6° per minute (see Figure 18).

List of selected peaks (only peaks with relative intensity greater than or equal to 1 % are indicated):

| 2θ (°) | d (Å) | I (%) |
|---|---|---|
| 5.53 | 15.98 | 100 |
| 14.15 | 6.26 | 4 |
| 15.75 | 5.63 | 4 |
| 16.67 | 5.32 | 4 |
| 17.40 | 5.10 | 1 |
| 18.02 | 4.92 | 3 |
| 18.48 | 4.80 | 5 |
| 19.51 | 4.55 | 2 |
| 21.43 | 4.15 | 5 |
| 22.30 | 3.99 | 1 |
| 23.15 | 3.84 | 2 |
| 23.67 | 3.76 | 5 |
| 24.37 | 3.65 | 1 |
| 24.49 | 3.64 | 1 |
| 26.96 | 3.31 | 1 |

## Single crystal X-ray diffraction

The crystal structure of co-crystal (*S*)-pregabalin - benzoic acid (1:1) has been determined from single crystal X-ray diffraction data. The colourless plate used (0.41 × 0.32 × 0.23 mm) was obtained from the evaporation of a solution of (*S*)-pregabalin and benzoic acid in water and ethanol.

Analysis was performed at room temperature using a Bruker Smart Apex diffractometer with graphite monochromated Mo $K_{\alpha}$ radiation equipped with a CCD detector. Data were collected using phi and omega scans (program used: SMART 5.6). No significant decay of standard intensities was observed. Data reduction (Lorentz and polarization corrections) and absorption correction were applied (program used: SAINT 5.0).

The structure was solved with direct methods and least-squares refinement of $F_o^2$ against all measured intensities was carried out (program used: SHELXTL-NT 6.1). All non-hydrogen atoms were refined with anisotropic displacement parameters.

Relevant structural data:

| | |
|---|---|
| Crystal system | Orthorhombic |
| Space group | $P2_12_12_1$ |
| a (Å) | 6.6130(4) |

(continued)

| Crystal system | Orthorhombic |
|---|---|
| b (Å) | 7.7257(5) |
| c (Å) | 31.822(2) |
| Volume (Å$^3$) | 1625.8(3) |
| Z | 4 |
| D calc. (Mg/m$^3$) | 1.149 |
| N. of refl. | 3936 |
| Refl. with I > 2σ(I) | 2933 |
| R(I>2a(I)) | 0.0489 |

The unit cell contents of this crystal structure are depicted in Figure 19 (hydrogen atoms have been omitted for clarity; program used: *Mercury* 2.3, C.F. Macrae, I.J. Bruno, J.A. Chisholm, P.R. Edgington, P. McCabe, E. Pidcock, L. Rodriguez-Monge, R. Taylor, J. van de Streek and P.A. Wood, J. Appl. Cryst., 41, 2008, 466-470).

Positions of peaks in XRPD diffractogram simulated from single crystal data are almost identical to those in the experimental one presented above.

**Example 6: (*S*)-Pregabalin - L-proline - HCl (1:1:1) (co-crystal)**

Slurrying in ACN 800 mg scale)

To a round-bottom flask equipped with magnetic stirrer containing (*S*)-pregabalin (412 mg, 2.59 mmol) and L-proline·HCl (392 mg, 2.59 mmol, 1.0 eq), was added ACN (8 mL). The resultant suspension was stirred at room temperature overnight. The solid was filtered with a sintered funnel (porosity 3), washed with ACN (3 mL) and dried under vacuum at room temperature to give co-crystal of (*S*)-pregabalin - L-proline - HCl (1:1:1) as a white powder (743 mg, 93% yield).

**$^1$H NMR**

Proton nuclear magnetic resonance analysis was recorded in deuterated methanol (d4-MeOH) in a Varian Mercury 400 spectrometer, equipped with a broadband probe ATB 1 H/19F/X of 5 mm. Spectrum was acquired dissolving 5-10 mg of sample in 0.6 mL of deuterated solvent.

$^1$H NMR spectrum in d4-MeOD at 400 MHz shows peaks at 4.00 (dd, $J$ = 6.3 Hz, $J$ = 8.6 Hz, 1 H); 3.38 (dt, $J$ = 7.0 Hz, $J$ = 11.3 Hz, 1 H); 3.23 (dt, $J$=7.4 Hz, $J$ = 11.3 Hz, 1 H); 3.01-2.91 (m, 2H); 2.45 (dd, $J$ = 5.5 Hz, $J$ = 16.4 Hz, 1 H); 2.37 (dd, $J$ = 6.6 Hz, $J$ = 16.4 Hz, 1 H); 2.36-2.25 (m, 1 H); 2.25-2.06 (m, 2H); 2.04-1.91 (m, 2H); 1.75-1.63 (m, 1 H); 1.32-1.20 (m, 2H); 0.95 (d, $J$ = 6.6 Hz, 3H); 0.93 (d, $J$ = 6.6 Hz, 3H) ppm.

**IR**

FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 cm$^{-1}$.

The sample (KBr pellets) shows a Fourier Transform Infra Red spectrum with absorption bands at 2958.6 (m, br), 1980.0 (m, br), 1713.6 (s), 1635.4 (s), 1573.2 (m), 1523.2 (m), 1386.9 (m), 1324.7 (m), 1243.3 (m), 1195.1 (s), 1144.4 (m), 872.0 (s), 847.7 (m), 812.8 (s), 670.6 (s), 635.2 (s) cm$^{-1}$.

**DSC**

DSC analysis was recorded with a Mettler DSC822$^e$. A sample of 4.0490 mg was weighed into a 40 μL aluminium crucible with a pinhole lid and was heated, under nitrogen (50 mL/min), at 10 °C/min from 30 to 300 °C.

The novel type of crystal of the present invention is characterized in that the endothermic sharp peak corresponding to the melting point has an onset at 107.32 °C (fusion enthalpy -103.61 J/g), measured by DSC analysis (10 °C/min) (see Figure 20).

**TGA**

Thermogravimetric analysis was recorded in a thermogravimetric analyzer Mettler TGA/SDTA851$^{e}$. A sample of 5.6337 mg was weighed into a 70 μL alumina crucible with a pinhole lid and was heated at 10 °C/min from 30 to 200 °C, under nitrogen (50 mL/min).

The TGA analysis of this crystalline form according to the invention shows no significant loss at temperatures lower than the melting point (see Figure 21).

**XRPD**

XRPD analysis was performed using a Philips X'Pert diffractometer with Cu $K_{\alpha}$ radiation in Bragg-Brentano geometry. The system is equipped with a monodimensional, real time multiple strip detector. The diffractogram was recorded from 3° to 40° (2θ) at a scan rate of 17.6° per minute (see Figure 22).

List of selected peaks (only peaks with relative intensity greater than or equal to 1 % are indicated):

| **2θ (°)** | **d (Å)** | **I (%)** |
|---|---|---|
| 8.05 | 10.98 | 100 |
| 12.88 | 6.87 | 2 |
| 14.08 | 6.29 | 4 |
| 14.57 | 6.08 | 2 |
| 16.25 | 5.45 | 13 |
| 17.40 | 5.10 | 19 |
| 19.15 | 4.63 | 3 |
| 20.16 | 4.40 | 2 |
| 21.24 | 4.18 | 15 |
| 22.22 | 4.00 | 14 |
| 24.59 | 3.62 | 4 |
| 25.54 | 3.49 | 4 |
| 25.94 | 3.44 | 12 |
| 26.32 | 3.39 | 1 |
| 27.15 | 3.28 | 6 |
| 28.29 | 3.16 | 1 |
| 29.06 | 3.07 | 2 |
| 30.25 | 2.95 | 6 |
| 32.13 | 2.79 | 2 |
| 34.20 | 2.62 | 1 |
| 35.72 | 2.51 | 2 |

**Example 7: (*S*)-Pregabalin - maleic acid - $H_2O$ (1:1:0.5) (co-crystal or salt)**

Slow evaporation from water

To a vial containing (*S*)-pregabalin (58 mg, 0.36 mmol) and maleic acid (42 mg, 0.36 mmol, 1.0 eq), was added water (2 mL). The resultant solution was slowly evaporated at room temperature. After complete evaporation, crystalline form (*S*)-pregabalin - maleic acid - $H_2O$ (1:1:0.5) was obtained as a white solid in a quantitative yield.

Slow evaporation from EtOH

To a vial containing (*S*)-pregabalin (58 mg, 0.36 mmol) and maleic acid (42 mg, 0.36 mmol, 1.0 eq), was added EtOH (7 mL). The mixture was heated until complete dissolution and the solvent was slowly evaporated at room temperature. After complete evaporation, crystalline form (*S*)-pregabalin - maleic acid - $H_2O$ (1:1:0.5) was obtained as a white solid in a quantitative yield.

Slow evaporation from IPA (800 mg scale)

To a vial containing (S)-pregabalin (462 mg, 2.90 mmol) and maleic acid (337 mg, 2.90 mmol, 1.0 eq), was added IPA (8 mL). The mixture was heated until complete dissolution and the solvent was slowly evaporated at room temperature. After complete evaporation, crystalline form (S)-pregabalin - maleic acid - $H_2O$ (1:1:0.5) was obtained as a white solid in a quantitative yield.

**$^{1}$H NMR**

Proton nuclear magnetic resonance analysis was recorded in deuterated methanol (d4-MeOH) in a Varian Mercury 400 spectrometer, equipped with a broadband probe ATB 1H/19F/X of 5 mm. Spectrum was acquired dissolving 5-10 mg of sample in 0.6 mL of deuterated solvent.

$^{1}$H NMR spectrum in d4-MeOD at 400 MHz shows peaks identical to those of form of example 4.

**$^{13}$C-CPMAS NMR**

CPMAS solid state nuclear magnetic resonance analysis was recorded in a Bruker 400MHz WB spectrometer at 20 °C using a 1 H/BB probe. Sample was carefully packed in a 4-mm diameter cylindrical zirconia rotor with Kel-F end-caps. The operating conditions involved 4.5 μs 90° 1 H pulses and decoupling field strength of 83.3 kHz by SPINAL64 sequence. $^{13}$C spectrum was externally referenced to adamantane sample and then the chemical shifts were recalculated to the $Me_4Si$. The typical acquisition parameters for $^{13}$C-CPMAS were: spectral width, 50 kHz; recycle delay, 2 s; acquisition time, 40 ms; contact time, 2.5 ms; and spin rate, 10 kHz.

$^{13}$C-CPMAS NMR : $\delta$ = 22.3; 24.0; 26.0; 31.6; 33.2; 41.5; 43.9; 136.1; 137.1; 170.0; 171.3; 178.9 ppm.

**IR**

FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 $cm^{-1}$.

The sample (KBr pellets) shows a Fourier Transform Infra Red spectrum with absorption bands at 3505.8 (m, br), 3164.8 (m, br), 2958.9 (m), 2874.9 (m), 1701.7 (s), 1577.1 (m), 1476.7 (m, br), 1364.1 (s), 1307.8 (m), 1260.4 (m), 1249.2 (m), 1002.9 (m), 985.0 (m), 877.4 (s), 867.8 (s), 758.3 (m), 688.3 (m), 654.2 (m) $cm^{-1}$.

**DSC**

DSC analysis was recorded with a Mettler DSC822$^{e}$. A sample of 7.6580 mg was weighed into a 40 μL aluminium crucible with a pinhole lid and was heated, under nitrogen (50 mL/min), at 10 °C/min from 30 to 200 °C.

The novel type of crystal of the present invention is characterized in that the endothermic sharp peak corresponding to the melting point has an onset at 58.54 °C (fusion enthalpy -96.88 J/g), measured by DSC analysis (10 °C/min) (see Figure 23).

**TGA**

Thermogravimetric analysis was recorded in a thermogravimetric analyzer Mettler TGA/SDTA851$^{e}$. A sample of 9.4879 mg was weighed into a 70 μL alumina crucible with a pinhole lid and was heated at 10 °C/min from 30 to 200 °C, under nitrogen (50 mL/min).

The TGA analysis of this crystalline form according to the invention shows a progressive weight loss from 30 °C to temperatures above the melting point (see Figure 24).

## Karl Fischer

Karl Fischer analyses were recorded with a Metrohm 787 KF Trinito. Analyses of two samples of 46.9 mg and 38.3 mg were carried out using the following reactants: Hydranal-Composite 5 (Riedel de Haën Ref. 34081), Hydranal Methanol Rapad (Riedel de Haën Ref. 37817) and Hydranal Water Standard 10.0 (Riedel de Haën Ref. 34849 used to calculate the factor).

The KF analysis of the crystalline form according to the invention shows 3.3% water (average of the two analyses) as expected for a hemihydrate.

## XRPD

XRPD analysis was performed using a Philips X'Pert diffractometer with Cu $K_{\alpha}$ radiation in Bragg-Brentano geometry. The system is equipped with a monodimensional, real time multiple strip detector. The diffractogram was recorded from 3° to 40° (2θ) at a scan rate of 17.6° per minute (see Figure 25).

List of selected peaks (only peaks with relative intensity greater than or equal to 1 % are indicated):

| 2θ (°) | d (Å) | I (%) | | 2θ (°) | d (Å) | I (%) |
|---|---|---|---|---|---|---|
| 5.93 | 14.91 | 100 | | 21.71 | 4.09 | 4 |
| 6.94 | 12.73 | 3 | | 22.50 | 3.95 | 1 |
| 11.85 | 7.47 | 13 | | 23.32 | 3.81 | 8 |
| 12.62 | 7.02 | 2 | | 23.82 | 3.74 | 21 |
| 13.76 | 6.43 | 3 | | 24.78 | 3.59 | 1 |
| 17.02 | 5.21 | 2 | | 25.21 | 3.53 | 3 |
| 17.50 | 5.07 | 2 | | 25.33 | 3.52 | 4 |
| 18.14 | 4.89 | 2 | | 25.87 | 3.44 | 1 |
| 18.35 | 4.83 | 2 | | 26.22 | 3.40 | 2 |
| 18.65 | 4.76 | 8 | | 29.87 | 2.99 | 9 |
| 19.31 | 4.60 | 1 | | 29.92 | 2.99 | 8 |
| 19.45 | 4.56 | 2 | | 31.29 | 2.86 | 1 |
| 19.84 | 4.47 | 1 | | 36.05 | 2.49 | 1 |
| 21.00 | 4.23 | 1 | | | | |

## Single crystal X-ray diffraction

The crystal structure of crystalline form (*S*)-pregabalin - maleic acid - $H_2O$ (1:1:0.5) has been determined from single crystal X-ray diffraction data. The colourless crystal used (0.25 × 0.09 × 0.03 mm) was obtained from the evaporation of a solution of (*S*)-pregabalin and maleic acid in ethanol and methyl isobutyl ketone.

Analysis was performed at room temperature using an Oxford Diffraction Gemini diffractometer with graphite monochromated Cu $K_{\alpha}$ radiation equipped with a CCD detector. Data were collected using phi and omega scans. No significant decay of standard intensities was observed. Data reduction (Lorentz and polarization corrections) and absorption correction were applied (software for data collection and processing: CrysAlis$^{Pro}$ 1.171.34.36).

The structure was solved with direct methods and least-squares refinement of $F_o^2$ against all measured intensities was carried out (programs: SIR2006 and SHELXL97). All non-hydrogen atoms were refined with anisotropic displacement parameters, except two disordered carbon atoms of one (*S*)-pregabalin molecule.

Relevant structural data:

| | |
|---|---|
| Crystal system | Monoclinic |
| Space group | C2 |
| a (Å) | 30.479(5) |

(continued)

| Crystal system | Monoclinic |
|---|---|
| b (Å) | 5.6115(6) |
| c (Å) | 17.9812(17) |
| β (°) | 101.709(12) |
| Volume ($Å^3$) | 3011.4(5) |
| Z | 8 |
| D calc. ($Mg/m^3$) | 1.250 |
| N. of refl. | 5536 |
| Refl. with I > 2σ(I) | 3167 |
| R(I>2σ(I)) | 0.0902 |

The unit cell contents of this crystal structure are depicted in Figure 26 (hydrogen atoms have been omitted for clarity; program used: *Mercury* 2.3, C.F. Macrae, I.J. Bruno, J.A. Chisholm, P.R. Edgington, P. McCabe, E. Pidcock, L. Rodriguez-Monge, R. Taylor, J. van de Streek and P.A. Wood, J. Appl. Cryst., 41, 2008, 466-470).

Positions of peaks in XRPD diffractogram simulated from single crystal data are almost identical to those in the experimental one presented above.

**Example 8: (*S*)-Pregabalin - caprylic acid (1:1) (co-crystal)**

Wet grinding with TBME (solvent assisted grinding)

To a 2 mL eppendorf containing (*S*)-pregabalin (53 mg, 0.33 mmol), caprylic acid (52.2 μL, d=0.91 g/mL, 0.33 mmol, 1.0 eq) and three 4 mm stainless steel grinding balls, one drop of TBME was added. The reactor was stirred 45 minutes at a rate of 30 Hz (3 x 15 minutes). The product was dried under vacuum at room temperature overnight. Co-crystal of (S)-pregabalin - caprylic acid (1:1) was obtained as a white powder in quantitative yield.

**$^1$H NMR**

Proton nuclear magnetic resonance analysis was recorded in deuterated methanol (d4-MeOH) in a Varian Mercury 400 spectrometer, equipped with a broadband probe ATB 1H/19F/X of 5 mm. Spectrum was acquired dissolving 5-10 mg of sample in 0.6 mL of deuterated solvent.

$^1$H NMR spectrum in d4-MeOD at 400 MHz shows peaks at 2.96 (dd, *J* = 3.9 Hz, *J* = 12.9 Hz, 1 H); 2.85 (dd, *J* = 7.8 Hz, *J* = 12.9 Hz, 1 H); 2.44 (dd, *J* = 3.9 Hz, *J* = 16.0 Hz, 1 H); 2.27 (dd, *J* = 8.6 Hz, *J* = 16.0 Hz, 1 H); 2.25 (t, *J* = 7.4 Hz, 2H); 2.14-2.03 (m, 1 H); 1.77-1.63 (m, 1 H); 1.65-1.53 (m, 2H); 1.39-1.13 (m, 8+2H); 0.94 (d, *J* = 6.6 Hz, 3H); 0.92 (d, *J* = 6.6 Hz, 3H); 0.90 (t, *J* = 7.0 Hz, 2H) ppm.

IR

FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 $cm^{-1}$.

The sample (KBr pellets) shows a Fourier Transform Infra Red spectrum with absorption bands at 2953.9 (s, br), 2157.9 (m, br), 1732.8 (s), 1642.8 (m), 1531.9 (m, br), 1432.8 (m), 1396.7 (m), 1174.9 (m), 1097.2 (m), 1015.8 (m), 851.6 (s), 818.7 (s), 723.2 (m), 695.5 (s), 560.9 (m) $cm^{-1}$.

**DSC**

DSC analysis was recorded with a Mettler DSC822$^e$. A sample of 2.4020 mg was weighed into a 40 μL aluminium crucible with a pinhole lid and was heated, under nitrogen (50 mL/min), at 10 °C/min from 30 to 200 °C.

The novel type of crystal of the present invention shows multiple phase transitions before melting. The endothermic sharp peak corresponding to the melting point has an onset at 116.30 °C (fusion and decomposition enthalpy

-161.26 J/g), measured by DSC analysis (10 °C/min) (see Figure 27).

**TG**

Thermogravimetric analysis was recorded in a thermogravimetric analyzer Mettler TGA/SDTA851e. A sample of 6.8836 mg was weighed into a 70 μL alumina crucible with a pinhole lid and was heated at 10 °C/min from 30 to 200 °C, under nitrogen (50 mL/min).

The TGA analysis of this crystalline form according to the invention shows no significant loss at temperatures lower than the melting point (see Figure 28).

**XRPD**

XRPD analysis was performed using a Philips X'Pert diffractometer with Cu $K_{\alpha}$ radiation in Bragg-Brentano geometry. The system is equipped with a monodimensional, real time multiple strip detector. The diffractogram was recorded from 3° to 40° (2θ) at a scan rate of 17.6° per minute (see Figure 29).

List of selected peaks (only peaks with relative intensity greater than or equal to 1 % are indicated):

| 2θ (°) | d (Å) | I (%) |
|---|---|---|
| 4.74 | 18.65 | 100 |
| 9.50 | 9.31 | 2 |
| 12.31 | 7.19 | 2 |
| 13.42 | 6.60 | 3 |
| 16.45 | 5.39 | 2 |
| 17.92 | 4.95 | 1 |
| 18.24 | 4.86 | 1 |
| 18.99 | 4.67 | 4 |
| 20.02 | 4.44 | 6 |
| 21.29 | 4.17 | 5 |
| 21.44 | 4.14 | 8 |
| 22.97 | 3.87 | 7 |
| 23.35 | 3.81 | 2 |
| 23.98 | 3.71 | 1 |
| 25.42 | 3.50 | 1 |

**Single crystal X-ray diffraction**

The crystal structure of co-crystal of (*S*)-pregabalin - caprylic acid (1:1) has been determined from single crystal X-ray diffraction data. The colourless prismatic crystal used (0.25 × 0.13 × 0.03 mm) was obtained from the evaporation of a solution of (*S*)-pregabalin and caprylic acid in ethanol and water.

Analysis was performed at room temperature using an Oxford Diffraction Gemini diffractometer with graphite monochromated Cu $K_{\alpha}$ radiation equipped with a CCD detector, under a $N_2$ atmosphere. Data were collected using phi and omega scans. No significant decay of standard intensities was observed. Data reduction (Lorentz and polarization corrections) and absorption correction were applied (software for data collection and processing: CrysAlis$^{Pro}$ 1.171.33.66).

The structure was solved with direct methods and least-squares refinement of $F_o^2$ against all measured intensities was carried out (programs: SIR2006 and SHELXL97). All non-hydrogen atoms were refined with anisotropic displacement parameters.

Relevant structural data:

| | |
|---|---|
| Crystal system | Orthorhombic |
| Space group | $P2_12_12_1$ |
| a (Å) | 6.6171(10) |
| b (Å) | 7.7659(8) |
| c (Å) | 37.000(6) |
| Volume (Å$^3$) | 1901.4(5) |
| Z | 4 |
| D calc. (Mg/m$^3$) | 1.060 |
| N. of refl. | 3506 |
| Refl. with I > 2σ(I) | 895 |
| R(I>2σ(I)) | 0.0527 |

The unit cell contents of this crystal structure are depicted in Figure 30 (hydrogen atoms have been omitted for clarity; program used: *Mercury* 2.3, C.F. Macrae, I.J. Bruno, J.A. Chisholm, P.R. Edgington, P. McCabe, E. Pidcock, L. Rodriguez-Monge, R. Taylor, J. van de Streek and P.A. Wood, J. Appl. Cryst., 41, 2008, 466-470).

Positions of peaks in XRPD diffractogram simulated from single crystal data are almost identical to those in the experimental one presented above.

**Example 9: (*S*)-pregabalin - capric acid (1:1) (co-crystal)**

Wet grinding with TBME (solvent assisted grinding)

To a 2 mL eppendorf containing (*S*)-pregabalin (48 mg, 0.30 mmol), capric acid (52 mg, 0.30 mmol, 1.0 eq) and three 4 mm stainless steel grinding balls, one drop of TBME was added. The reactor was stirred 45 minutes at a rate of 30 Hz (3 x 15 minutes). The product was dried under vacuum at room temperature overnight. Co-crystal (S)-pregabalin - capric acid (1:1) was obtained as a white powder in quantitative yield.

**$^1$H NMR**

Proton nuclear magnetic resonance analysis was recorded in deuterated methanol (d4-MeOH) in a Varian Mercury 400 spectrometer, equipped with a broadband probe ATB 1 H/19F/X of 5 mm. Spectrum was acquired dissolving 5-10 mg of sample in 0.6 mL of deuterated solvent.

$^1$H NMR spectrum in d4-MeOD at 400 MHz shows peaks at 2.96 (dd, *J* = 3.5 Hz, *J* = 12.9 Hz, 1 H); 2.84 (dd, *J* = 7.8 Hz, *J* = 12.9 Hz, 1 H); 2.45 (dd, *J* = 3.1 Hz, *J* = 16.0 Hz, 1 H); 2.33-2.21 (m, 1 H); 2.26 (t, *J* = 7.4 Hz, 2H); 2.15-2.01 (m, 1 H); 1.77-1.63 (m, 1 H); 1.65-1.52 (m, 2H); 1.42-1.12 (m, 12+2H); 0.94 (d, *J* = 6.6 Hz, 3H); 0.92 (d, *J* = 6.6 Hz, 3H); 0.90 (t, *J* = 6.6 Hz, 2H) ppm.

**IR**

FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 cm$^{-1}$.

The sample (KBr pellets) shows a Fourier Transform Infra Red spectrum with absorption bands at 2925.9 (s), 2852.3 (s), 2682.1 (m, br), 2162.0 (m, br), 1731.1 (s), 1641.5 (m), 1533.2 (s, br), 1467.6 (m), 1432.8 (m), 1397.4 (s), 1175.9 (s), 1097.9 (m), 851.9 (m), 819.0 (m), 695.5 (m), 561.0 (m) cm$^{-1}$.

**DSC**

DSC analysis was recorded with a Mettler DSC822$^e$. A sample of 6.0580 mg was weighed into a 40 μL aluminium crucible with a pinhole lid and was heated, under nitrogen (50 mL/min), at 10 °C/min from 30 to 300 °C.

The novel type of crystal of the present invention shows multiple phase transitions before melting. The endothermic sharp peak corresponding to the melting point has an onset at 117.08 °C (fusion and decomposition enthalpy

-154.27 J/g), measured by DSC analysis (10 °C/min) (see Figure 31).

**TGA**

Thermogravimetric analysis was recorded in a thermogravimetric analyzer Mettler TGA/SDTA851e. A sample of 9.2700 mg was weighed into a 70 μL alumina crucible with a pinhole lid and was heated at 10 °C/min from 30 to 250 °C, under nitrogen (50 mL/min).

The TGA analysis of this crystalline form according to the invention shows no weight loss at temperatures lower than the melting point (see Figure 32).

**XRPD**

XRPD analysis was performed using a Philips X'Pert diffractometer with Cu $K_\alpha$ radiation in Bragg-Brentano geometry. The system is equipped with a monodimensional, real time multiple strip detector. The diffractogram was recorded from 3° to 40° (2θ) at a scan rate of 17.6° per minute (see Figure 33).

List of selected peaks (only peaks with relative intensity greater than or equal to 1 % are indicated):

| 2θ (°) | d (Å) | I (%) |
|---|---|---|
| 4.31 | 20.52 | 100 |
| 8.59 | 10.30 | 12 |
| 12.15 | 7.29 | 5 |
| 13.07 | 6.78 | 7 |
| 13.54 | 6.54 | 1 |
| 14.85 | 5.96 | 2 |
| 17.22 | 5.15 | 4 |
| 17.72 | 5.01 | 2 |
| 18.12 | 4.90 | 3 |
| 18.64 | 4.76 | 2 |
| 19.60 | 4.53 | 13 |
| 20.20 | 4.40 | 6 |
| 20.66 | 4.30 | 14 |
| 21.89 | 4.06 | 10 |
| 22.94 | 3.88 | 23 |
| 23.25 | 3.83 | 8 |
| 23.73 | 3.75 | 4 |
| 26.93 | 3.31 | 1 |
| 27.46 | 3.25 | 2 |
| 37.29 | 2.41 | 1 |

**Single crystal X-ray diffraction**

The crystal structure of co-crystal (*S*)-pregabalin - capric acid (1:1) has been determined from single crystal X-ray diffraction data. The colourless prismatic crystal used (0.52 × 0.21 × 0.07 mm) was obtained from the evaporation of a solution of (*S*)-pregabalin and capric acid in ethanol, water and THF.

Analysis was performed at room temperature using an Oxford Diffraction Gemini diffractometer with graphite monochromated Cu $K_\alpha$ radiation equipped with a CCD detector, under a $N_2$ atmosphere. Data were collected using phi and omega scans. No significant decay of standard intensities was observed. Data reduction (Lorentz and polarization corrections) and absorption correction were applied (software for data collection and processing: CrysAlis$^{Pro}$

1.171.34.36).

The structure was solved with direct methods and least-squares refinement of $F_o^2$ against all measured intensities was carried out (programs: SIR2006 and SHELXL97). All non-hydrogen atoms were refined with anisotropic displacement parameters.

Relevant structural data:

| | |
|---|---|
| Crystal system | Orthorhombic |
| Space group | $P2_12_12_1$ |
| a (Å) | 6.6074(6) |
| b (Å) | 7.7910(6) |
| c (Å) | 40.986(8) |
| Volume (Å$^3$) | 2109.9(5) |
| Z | 4 |
| D calc. (Mg/m$^3$) | 1.044 |
| N. of refl. | 3766 |
| Refl. with I> 2σ(I) | 2386 |
| R(I>2σ(I)) | 0.0753 |

The unit cell contents of this crystal structure are depicted in Figure 34 (hydrogen atoms have been omitted for clarity; program used: *Mercury* 2.3, C.F. Macrae, I.J. Bruno, J.A. Chisholm, P.R. Edgington, P. McCabe, E. Pidcock, L. Rodriguez-Monge, R. Taylor, J. van de Streek and P.A. Wood, J. Appl. Cryst., 41, 2008, 466-470).

Positions of peaks in XRPD diffractogram simulated from single crystal data are almost identical to those in the experimental one presented above.

**Example 10: (*S*)-Pregabalin - maleic acid (1:1) (co-crystal or salt)**

Slow evaporation from IPA (800 mg scale) (formation of form obtained in Example 7 and then dehydration)

To a vial containing (S)-pregabalin (462 mg, 2.90 mmol) and maleic acid (337 mg, 2.90 mmol, 1.0 eq), was added IPA (8 mL). The mixture was heated until complete dissolution and the solvent was slowly evaporated at room temperature. After complete evaporation, crystalline form (S)-pregabalin - maleic acid - $H_2O$ (1:1:0.5) (Example 7) was obtained as a white solid in a quantitative yield. This solid was dried under vacuum at room temperature with $P_2O_5$ for two weeks to give (S)-pregabalin - maleic acid (1:1) (co-crystal or salt) as a white powder in a quantitative yield.

**$^1$H NMR**

Proton nuclear magnetic resonance analysis was recorded in deuterated methanol (d4-MeOH) in a Varian Mercury 400 spectrometer, equipped with a broadband probe ATB 1 H/19F/X of 5 mm. Spectrum was acquired dissolving 5-10 mg of sample in 0.6 mL of deuterated solvent.

$^1$H NMR spectrum in d4-MeOD at 400 MHz shows peaks identical to those of form obtained in example 4.

**$^{13}$C-CPMAS NMR**

CPMAS solid state nuclear magnetic resonance analysis was recorded in a Bruker 400MHz WB spectrometer at 20 °C using a 1 H/BB probe. Sample was carefully packed in a 4-mm diameter cylindrical zirconia rotor with Kel-F end-caps. The operating conditions involved 4.5 μs 90° 1H pulses and decoupling field strength of 83.3 kHz by SPINAL64 sequence. $^{13}$C spectrum was externally referenced to adamantane sample and then the chemical shifts were recalculated to the $Me_4Si$. The typical acquisition parameters for $^{13}$C-CPMAS were: spectral width, 50 kHz; recycle delay, 2 s; acquisition time, 40 ms; contact time, 2.5 ms; and spin rate, 10 kHz.

$^{13}$C-CPMAS NMR: δ = 23.1; 24.8; 26.5; 32.2; 33.8; 42.2; 44.6; 136.8; 137.5; 170.8; 171.8; 179.3 ppm.

## IR

FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 $cm^{-1}$.

The sample (KBr pellets) shows a Fourier Transform Infra Red spectrum with absorption bands at The sample (KBr pellets) shows a Fourier Transform Infra Red spectrum with absorption bands at 3166.2 (m, br), 2964.2 (m), 1705.6 (m), 1579.9 (m), 1488.9 (m, br), 1387.9 (s), 1353.3 (m), 1320.0 (m), 1260.8 (m), 1232.4 (m), 865.4 (m), 808.8 (m), 751.1 (s), 697.4 (s), 658.2 (s) $cm^{-1}$.

## DSC

DSC analysis was recorded with a Mettler DSC822$^e$. A sample of 1.7720 mg was weighed into a 40 μL aluminium crucible with a pinhole lid and was heated, under nitrogen (50 mL/min), at 10 °C/min from 30 to 300 °C.

The novel type of crystal of the present invention is characterized in that the endothermic sharp peak corresponding to the melting point has an onset at 73.47 °C (fusion enthalpy -76.13 J/g), measured by DSC analysis (10 °C/min) (see Figure 35).

## TGA

Thermogravimetric analysis was recorded in a thermogravimetric analyzer Mettler TGA/SDTA851$^e$. A sample of 6.0436 mg was weighed into a 70 μL alumina crucible with a pinhole lid and was heated at 10 °C/min from 30 to 300 °C, under nitrogen (50 mL/min).

The TGA analysis of this crystalline form according to the invention shows no weight loss at temperatures lower than the melting point (see Figure 36).

## XRPD

XRPD analysis was performed using a Philips X'Pert diffractometer with Cu $K_\alpha$ radiation in Bragg-Brentano geometry. The system is equipped with a monodimensional, real time multiple strip detector. The diffractogram was recorded from 3° to 40° (2θ) at a scan rate of 17.6° per minute (see Figure 37).

List of selected peaks (only peaks with relative intensity greater than or equal to 1 % are indicated):

| 2θ (°) | d (Å) | I (%) | | 2θ (°) | d (Å) | I (%) |
|---|---|---|---|---|---|---|
| 7.66 | 11.55 | 100 | | 23.13 | 3.85 | 14 |
| 10.57 | 8.37 | 4 | | 23.45 | 3.79 | 2 |
| 11.25 | 7.87 | 9 | | 23.97 | 3.71 | 52 |
| 15.36 | 5.77 | 36 | | 24.44 | 3.64 | 2 |
| 15.57 | 5.69 | 3 | | 25.05 | 3.56 | 5 |
| 16.02 | 5.53 | 6 | | 25.86 | 3.45 | 9 |
| 16.79 | 5.28 | 13 | | 27.19 | 3.28 | 3 |
| 16.95 | 5.23 | 6 | | 30.17 | 2.96 | 2 |
| 17.67 | 5.02 | 15 | | 30.99 | 2.89 | 2 |
| 19.47 | 4.56 | 21 | | 31.52 | 2.84 | 4 |
| 20.69 | 4.29 | 5 | | 33.99 | 2.64 | 2 |
| 21.25 | 4.18 | 3 | | 36.17 | 2.48 | 3 |
| 22.62 | 3.93 | 8 | | 39.05 | 2.31 | 1 |

**Example 11: (*S*)-Pregabalin - lauric acid (1:1) (co-crystal)**

Wet grinding with IPA (solvent assisted grinding)

To a 2 mL eppendorf containing (*S*)-pregabalin (44 mg, 0.28 mmol), lauric acid (56 mg, 0.28 mmol, 1.0 eq) and three 4 mm stainless steel grinding balls, one drop of IPA was added. The reactor was stirred 90 minutes at a rate of 30 Hz (6 x 15 minutes). The product was dried under vacuum at room temperature overnight. Co-crystal of (*S*)-pregabalin - lauric acid (1:1) was obtained as a white powder in quantitative yield.

Crystallization in IPA/water (1.5 g scale)

To a round-bottom flask equipped with magnetic stirrer containing (*S*)-pregabalin (664 mg, 4.17 mmol) and lauric acid (835 mg, 4.17 mmol, 1.0 eq), was added a mixture of IPA/water (10/90 vol/vol) (12 mL). The resultant suspension was heated at reflux for 30 min ((*S*)-pregabalin is soluble at this temperature and lauric acid melts), cooled down to room temperature and stirred overnight. A white precipitate was formed. The solid was filtered with a sintered funnel (porosity 3) and dried under vacuum at room temperature to give co-crystal of (*S*)-pregabalin - lauric acid (1:1) as a white powder (659 mg, 44% yield).

**$^1$H NMR**

Proton nuclear magnetic resonance analysis was recorded in deuterated methanol (d4-MeOH) in a Varian Mercury 400 spectrometer, equipped with a broadband probe ATB 1H/19F/X of 5 mm. Spectrum was acquired dissolving 5-10 mg of sample in 0.6 mL of deuterated solvent.

$^1$H NMR spectrum in d4-MeOD at 400 MHz shows peaks at 2.96 (dd, *J* = 3.9 Hz, *J* = 12.9 Hz, 1 H); 2.84 (dd, *J* = 7.8 Hz, *J* = 12.9 Hz, 1 H); 2.44 (dd, *J* = 3.5 Hz, *J* = 15.6 Hz, 1 H); 2.27 (dd, *J* = 8.6 Hz, *J* = 16.0 Hz, 1 H); 2.25 (t, *J* = 7.4 Hz, 2H); 2.15-2.02 (m, 1 H); 1.77-1.63 (m, 1 H); 1.64-1.53 (m, 2H); 1.40-1.13 (m, 16+2H); 0.94 (d, *J* = 6.6 Hz, 3H); 0.92 (d, *J* = 6.6 Hz, 3H); 0.89 (t, *J* = 7.0 Hz, 2H) ppm.

**IR**

FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 $cm^{-1}$.

The sample (KBr pellets) shows a Fourier Transform Infra Red spectrum with absorption bands at 2851.7 (m), 2156.4 (m, br), 1731.4 (m), 1644.0 (m), 1530.7 (m, br), 1467.9 (m), 1432.8 (m), 1396.9 (m), 1339.2 (m), 1239.3 (m), 1207.7 (m), 1173.8 (s), 1097.5 (m), 1015.6 (m), 851.0 (s), 818.7 (s), 721.4 (m), 694.7 (s), 562.2 (m) $cm^{-1}$.

**DSC**

DSC analysis was recorded with a Mettler DSC822$^e$. A sample of 2.3100 mg was weighed into a 40 μL aluminium crucible with a pinhole lid and was heated, under nitrogen (50 mL/min), at 10 °C/min from 30 to 300 °C.

The novel type of crystal of the present invention shows multiple phase transitions before melting. The endothermic sharp peak corresponding to the melting point has an onset at 121.99 °C (fusion and decomposition enthalpy -204.37 J/g), measured by DSC analysis (10 °C/min) (see Figure 38).

**TGA**

Thermogravimetric analysis was recorded in a thermogravimetric analyzer Mettler TGA/SDTA851$^e$. A sample of 4.9829 mg was weighed into a 70 μL alumina crucible with a pinhole lid and was heated at 10 °C/min from 30 to 300 °C, under nitrogen (50 mL/min).

The TGA analysis of this crystalline form according to the invention shows no weight loss at temperatures lower than the melting point (see Figure 39).

**XRPD**

XRPD analysis was performed using a Philips X'Pert diffractometer with Cu $K_\alpha$ radiation in Bragg-Brentano geometry. The system is equipped with a monodimensional, real time multiple strip detector. The diffractogram was recorded from 3° to 40° (2θ) at a scan rate of 17.6° per minute (see Figure 40).

List of selected peaks (only peaks with relative intensity greater than or equal to 1 % are indicated):

| 2θ (°) | d (Å) | I (%) |
|---|---|---|
| 4.02 | 21.96 | 100 |
| 8.03 | 11.01 | 43 |
| 12.07 | 7.34 | 8 |
| 12.87 | 6.88 | 13 |
| 13.69 | 6.47 | 5 |
| 14.43 | 6.14 | 7 |
| 16.54 | 5.36 | 4 |
| 17.44 | 5.08 | 6 |
| 17.85 | 4.97 | 11 |
| 18.42 | 4.82 | 7 |
| 19.19 | 4.63 | 14 |
| 20.12 | 4.41 | 39 |
| 20.84 | 4.26 | 29 |
| 21.22 | 4.19 | 41 |
| 22.45 | 3.96 | 12 |
| 22.97 | 3.87 | 34 |
| 23.25 | 3.83 | 17 |
| 23.69 | 3.76 | 8 |
| 24.16 | 3.68 | 7 |
| 25.24 | 3.53 | 2 |
| 26.65 | 3.35 | 3 |
| 27.06 | 3.29 | 2 |
| 27.68 | 3.22 | 3 |
| 29.30 | 3.05 | 1 |
| 32.23 | 2.78 | 2 |

**Single crystal X-ray diffraction**

The crystal structure of co-crystal of (*S*)-pregabalin - lauric acid (1:1) has been determined from single crystal X-ray diffraction data. The colourless crystal used (0.46 × 0.15 × 0.06 mm) was obtained from the evaporation of a solution of (*S*)-pregabalin and lauric acid in DMSO, water and methanol.

Analysis was performed at room temperature using an Oxford Diffraction Gemini diffractometer with graphite monochromated Cu $K_\alpha$ radiation equipped with a CCD detector, under a $N_2$ atmosphere. Data were collected using phi and omega scans. No significant decay of standard intensities was observed. Data reduction (Lorentz and polarization corrections) and absorption correction were applied (software for data collection and processing: CrysAlis$^{Pro}$ 1.171.34.36).

The structure was solved with direct methods and least-squares refinement of $F_o^2$ against all measured intensities was carried out (programs: SIR2006 and SHELXL97). All non-hydrogen atoms were refined with anisotropic displacement parameters.

Relevant structural data:

| | |
|---|---|
| Crystal system | Orthorhombic |
| Space group | $P2_12_12_1$ |
| a (Å) | 6.7553(4) |
| b (Å) | 7.7591(3) |
| c (Å) | 43.796(2) |
| Volume (Å$^3$) | 2295.6(2) |
| Z | 4 |
| D calc. (Mg/m$^3$) | 1.040 |
| N. of refl. | 4108 |
| Refl. with I>2σ(I) | 3514 |
| R(I>2σ(I)) | 0.0523 |

The unit cell contents of this crystal structure of are depicted in Figure 41 (hydrogen atoms have been omitted for clarity; program used: *Mercury* 2.3, C.F. Macrae, I.J. Bruno, J.A. Chisholm, P.R. Edgington, P. McCabe, E. Pidcock, L. Rodriguez-Monge, R. Taylor, J. van de Streek and P.A. Wood, J. Appl. Cryst., 41, 2008, 466-470).

Positions of peaks in XRPD diffractogram simulated from single crystal data are almost identical to those in the experimental one presented above.

**Example 12: Determination of the hygroscopicity of the co-crystals and crystalline form (co-crystal or salt) according to the invention:**

The objective was to measure the hygroscopicity of some crystalline forms of the present invention. The percentage increase of mass was calculated in order to evaluate the adsorption of water in the samples.

Materials and Methods:

Dynamic Vapour Sorption (DVS) analysis is a technique specifically designed to provide information related to material response to changes in relative humidity and T°C. A Q5000 SA DVS equipment from TA Instruments was used for running the experiment.

Water adsorption was evaluated by increasing humidity stepwise over a broad range from 15 to 95% RH, at constant temperature. A previous program at 5% RH / 60°C was run until constant weight of the sample in order to begin the analysis with the dried sample.

- Three samples were analysed:

  Sample 1: 5 mg of (S)-pregabalin - L-proline - HCl (1:1:1) co-crystal of example 6,
  Sample 2: 8 mg of crystalline form (*S*)-pregabalin - maleic acid - $H_2O$ (1:1:0.5) (co-crystal or salt) of example 7.
  Sample 3: 11 mg of (*S*)-Pregabalin - lauric acid (1:1) co-crystal of example 11.

Results:

The variation of the weight is controlled and the following percentage is calculated:

$$\Delta \text{ \%weight} = (P_n - P_1)/P_1 \times 100$$

wherein: $P_1$ is the initial weight , $P_n$ is the weight measured for the sample.

The results are summarized in the following table 1:

Table 1: % of variation of the weight

| Sample | Δ %weight 24 h |
|---|---|
| Sample 1 | 95% |
| Sample 2 | 37,9% |
| Sample 3 | 0% |

In the case of sample 1, sufficient water was absorbed to form a liquid. Co-crystal of (*S*)-pregabalin - L-proline - HCl (1:1:1) is deliquescent, form of (*S*)-pregabalin - maleic - $H_2O$ (1:1:0.5) (co-crystal or salt) is very hygroscopic and co-crystal of (*S*)-Pregabalin - lauric acid (1:1) is not hygroscopic.

Finally, the results are collected in below table summarizing the respective results achieved with the crystalline forms of pregabalin and at least one co-crystal former.

***SUMMARY TABLE***

| Composition | Ratio | Crystal Form (salt or co-crystal) | Solubility | Hygroscopicity (85-95%) | Example |
|---|---|---|---|---|---|
| PGB·L-tartaric | 1:1 | | | | 1 |
| PGB·tartaric·$H_2O$ | 1:1:1 | Salt | | | 2 |
| PGB·L-malic | 1:1 | Salt | | | 3 |
| PGB·maleic | 1:1 | Salt | | | 4 |
| PGB·benzoic | 1:1 | co-crystal | pH 1 insoluble pH 5.7 soluble | | 5 |
| PGB·L-proline·HCl· | 1:1:1 | co-crystal | pH 1 soluble> 25 mg/ml pH 6.1 soluble> 25 mg/ml | 95% | 6 |
| PGB·maleic·$H_2O$ | 1:1:0,5 | | | 37.9% | 7 |
| PGB·caprylic | 1:1 | co-crystal | pH 1 partially soluble (oil) pH 5.7 soluble | | 8 |
| PGB·capric | 1:1 | co-crystal | pH 1 partially soluble pH 6.1 insoluble | | 9 |
| PGB·maleic anhydrous | 1:1 | | | | 10 |
| PGB·lauric | 1:1 | co-crystal | pH 1 insoluble | Non-hygroscopic | 11 |
| PGB free base | | reference | pH 1 soluble pH 6.6 soluble | Non-hygroscopic | - |

Solubility was tested dissolving 25 mg of the crystalline form in 1ml in two different pH (1 and 6.8) and evaluating if it was soluble, insoluble or partially soluble (solubility 25mg/ml)

## Claims

**1.** A crystalline form of pregabalin with at least one co-former with the provisos that

- the crystalline form of the salt pregabalin hydrochloride,

- the crystalline form of the salt pregabalin (S)-mandelate,
- the co-crystal of (S)-pregabalin and (S)-mandelic acid,
- the crystalline form of the acid addition salts of pregabalin being hydrochloride, hydrobromide, hydrosulfate salts,
- the crystalline form of sodium, potassium, and magnesium salt of pregabalin are excluded.

**2.** The crystalline form according to claim 1, wherein the crystalline form is the crystalline form of a salt or is a co-crystal.

**3.** The crystalline form according to any of claims 1 or 2, wherein the co-former is selected from the group consisting of tartaric acid, benzoic acid, capric acid, caprylic acid, lauric acid, maleic acid, L-proline, nicotinamide, malic acid, urea or their stereoisomers; more preferably wherein the co-former is selected from the group consisting of tartaric acid, malic acid, benzoic acid, capric acid, caprylic acid, lauric acid, maleic acid, L-proline.

**4.** The crystalline form according to any one of claims 1 to 3, wherein the crystalline form is the crystalline form of a salt of pregabalin and a co-former being a salt-co-former.

**5.** The crystalline form according to claim 4, wherein the pregabalin is (S)-pregabalin.

**6.** The crystalline form according to any one of claims 4 or 5, wherein the salt-co-former is selected from the group consisting of tartaric acid, benzoic acid, capric acid, caprylic acid, lauric acid, maleic acid, L-proline, nicotinamide, malic acid, urea or their stereoisomers; more preferably wherein the co-former is selected from the group consisting of tartaric acid, malic acid, maleic acid, or their stereoisomers; most preferably wherein the co-former is selected from the group consisting of L-(+)-tartaric acid, L-malic acid, or maleic acid.

**7.** The crystalline form according to any of claims 4 to 6, wherein the salt is selected from the group consisting of (*S*)-pregabalin - L-(+)-tartaric acid (1:1), (*S*)-pregabalin - L-(+)-tartaric acid - $H_2O$ (1:1:1), (*S*)-pregabalin - L-malic acid (1:1), (*S*)-pregabalin - maleic acid (1:1), (*S*)-pregabalin - maleic acid - $H_2O$ (1:1:0.5) or (*S*)-pregabalin - maleic acid anhydrous (1:1); preferably wherein the salt is selected from the group consisting of (*S*)-pregabalin - L-(+)-tartaric acid - $H_2O$ (1:1:1), (*S*)-pregabalin - L-malic acid (1:1), or (*S*)-pregabalin - maleic acid (1:1).

**8.** The crystalline form according to any one of claims 1 to 3, wherein the compound is a co-crystal comprising pregabalin as a free base or as its pharmaceutically acceptable salt and at least one co-former being a co-crystal former.

**9.** The crystalline form according to claim 8, wherein the pregabalin is (*S*)-pregabalin.

**10.** The crystalline form according to any one of claims 8 or 9, wherein the co-crystal former is selected from the group consisting of tartaric acid, benzoic acid, capric acid, caprylic acid, lauric acid, maleic acid, proline, or their stereoisomers;
more preferably wherein the co-former is selected from the group consisting of tartaric acid, benzoic acid, maleic acid, proline, caprylic acid, capric acid, lauric acid or their stereoisomers;
most preferably wherein the co-former is selected from the group consisting of benzoic acid, L-proline, caprylic acid, capric acid, or lauric acid.

**11.** The crystalline form according to any one of claims 8 to 10, wherein the co-crystal is selected from (*S*)-pregabalin - L-tartaric acid (1:1), (*S*)-pregabalin - benzoic acid (1:1), (*S*)-pregabalin - L-proline - HCl (1:1:1), (*S*)-pregabalin - maleic acid - $H_2O$ (1:1:0.5), (*S*)-pregabalin - caprylic acid (1:1), (*S*)-pregabalin - capric acid (1:1), (*S*)-pregabalin - maleic acid anhydrous (1:1), or (*S*)-pregabalin - lauric acid (1:1);
more preferably wherein the co-crystal is selected from (*S*)-pregabalin - benzoic acid (1:1), (*S*)-pregabalin - L-proline - HCl (1:1:1), (*S*)-pregabalin - caprylic acid (1:1), (*S*)-pregabalin - capric acid (1:1); or (*S*)-pregabalin - lauric acid (1:1).

**12.** The crystalline form according to claim 2, wherein the crystal form is selected from forms of (*S*)-pregabalin - L-(+)-tartaric acid (1:1), (*S*)-pregabalin - maleic acid - $H_2O$ (1:1:0.5) or (*S*)-pregabalin - maleic acid anhydrous (1:1).

**13.** The crystalline forms according to one of the preceding claims, wherein the ratio between pregabalin and co-former is chosen in such a way that if compared to either pregabalin alone and/or to a mixture of pregabalin and co-former

- the solubility of the co-crystal is increased; and/or
- the dose response of the co-crystal is increased; and/or

- the efficacy of the co-crystal is increased; and/or
- the dissolution of the co-crystal is increased; and/or
- the bioavailability of the co-crystal is increased; and/or
- the stability of the co-crystal is increased; and/or
- the hygroscopicity of the co-crystal is decreased; and/or
- the form diversity of the co-crystal is decreased; and/or
- the morphology of the co-crystal is modulated.

**14.** Process for the production of a crystalline form according to any of claims 1 to 13 comprising the steps of:

a) dissolving or suspending pregabalin and co-former in a solvent or mixture of solvents,
b) optionally mixing or stirring the solution or suspension,
c) optionally before, during or after step (b) cooling or keeping the mixed solution/suspension to or at ambient temperature or below;
d) optionally filtering-off and/or washing the resulting solid with a solvent, and
e) drying the solid optionally at ambient temperature and/or vacuum.

**15.** Medicament comprising at least one crystalline form either as a salt or as a co-crystal according to any of claims 1 to 13 and optionally one or more pharmaceutically acceptable excipients.

**16.** The crystalline form either as a salt or as a co-crystal according to any one of claims 1 to 13 for use in the treatment of pain, preferably neuropathic pain, fibromyalgia or spinal cord injury, or of generalized anxiety disorder.

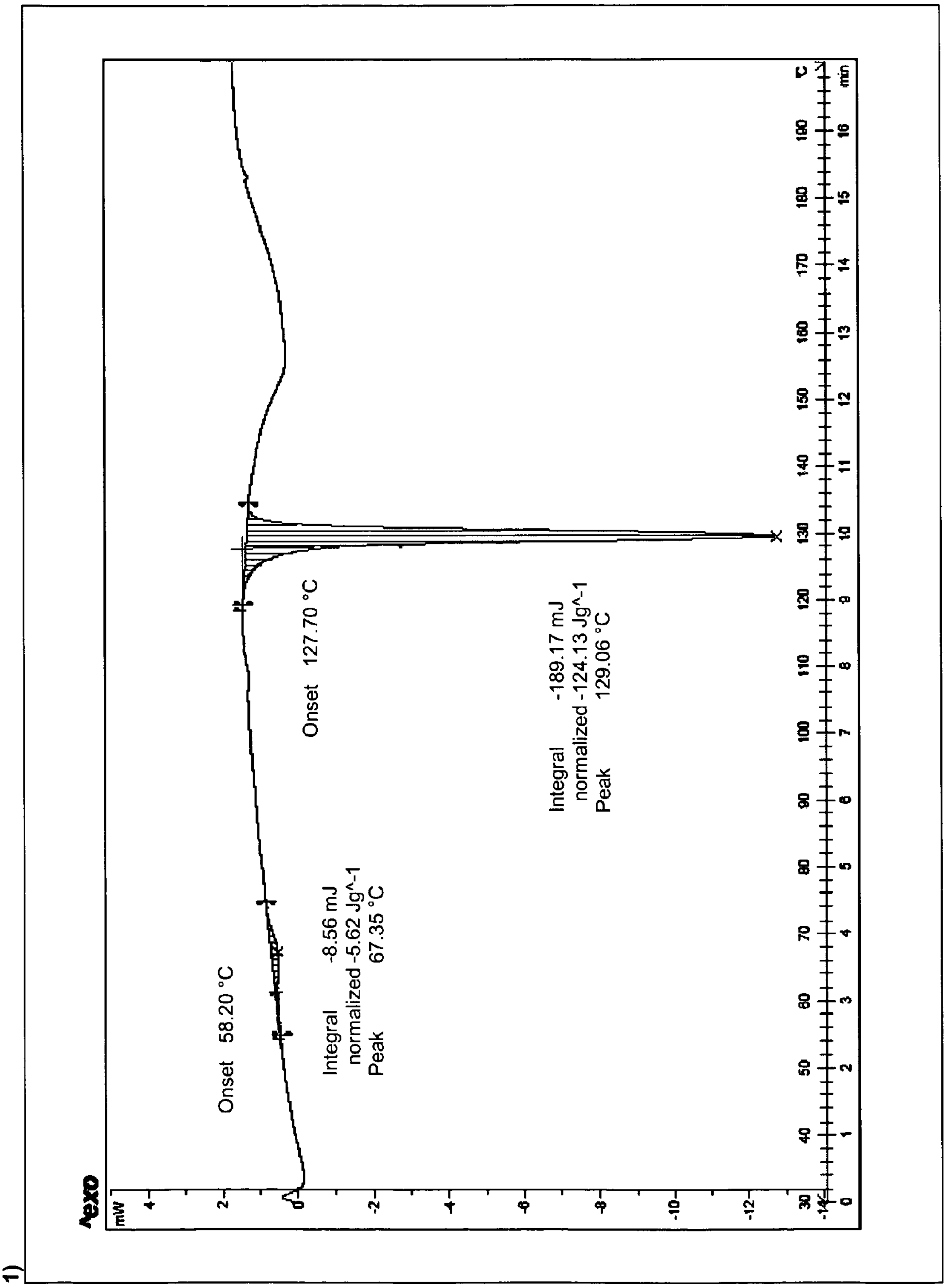
Onset 58.20 °C
Integral -8.56 mJ
normalized -5.62 Jg^-1
Peak 67.35 °C
Onset 127.70 °C
Integral -189.17 mJ
normalized -124.13 Jg^-1
Peak 129.06 °C
^exo
mW
°C
min

Figure 1)

Figure 2)

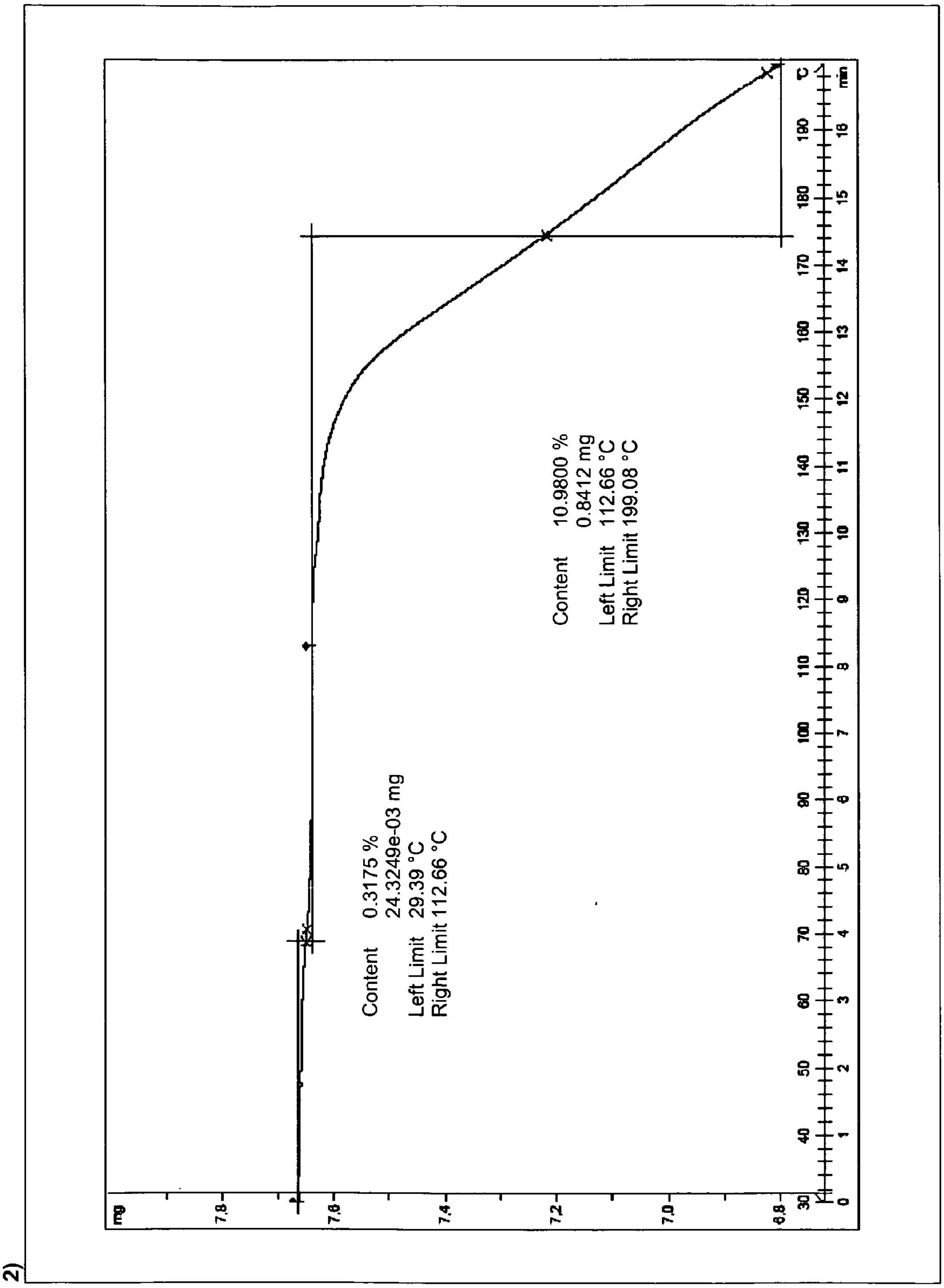
Content 0.3175 %
24.3249e-03 mg
Left Limit 29.39 °C
Right Limit 112.66 °C
Content 10.9800 %
0.8412 mg
Left Limit 112.66 °C
Right Limit 199.08 °C
mg
7,8
7,6
7,4
7,2
7,0
6,8
°C
min

Figure 3)

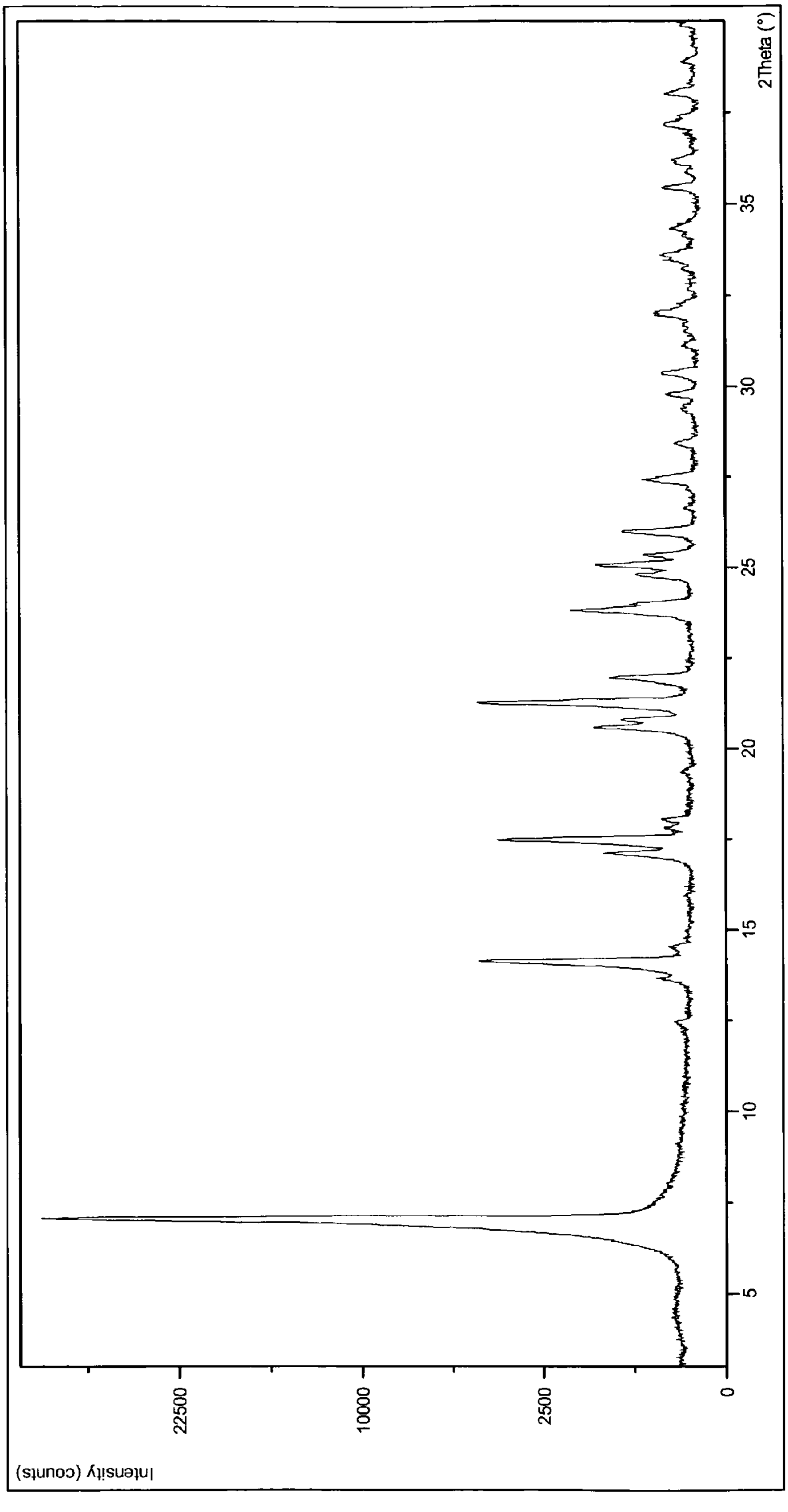

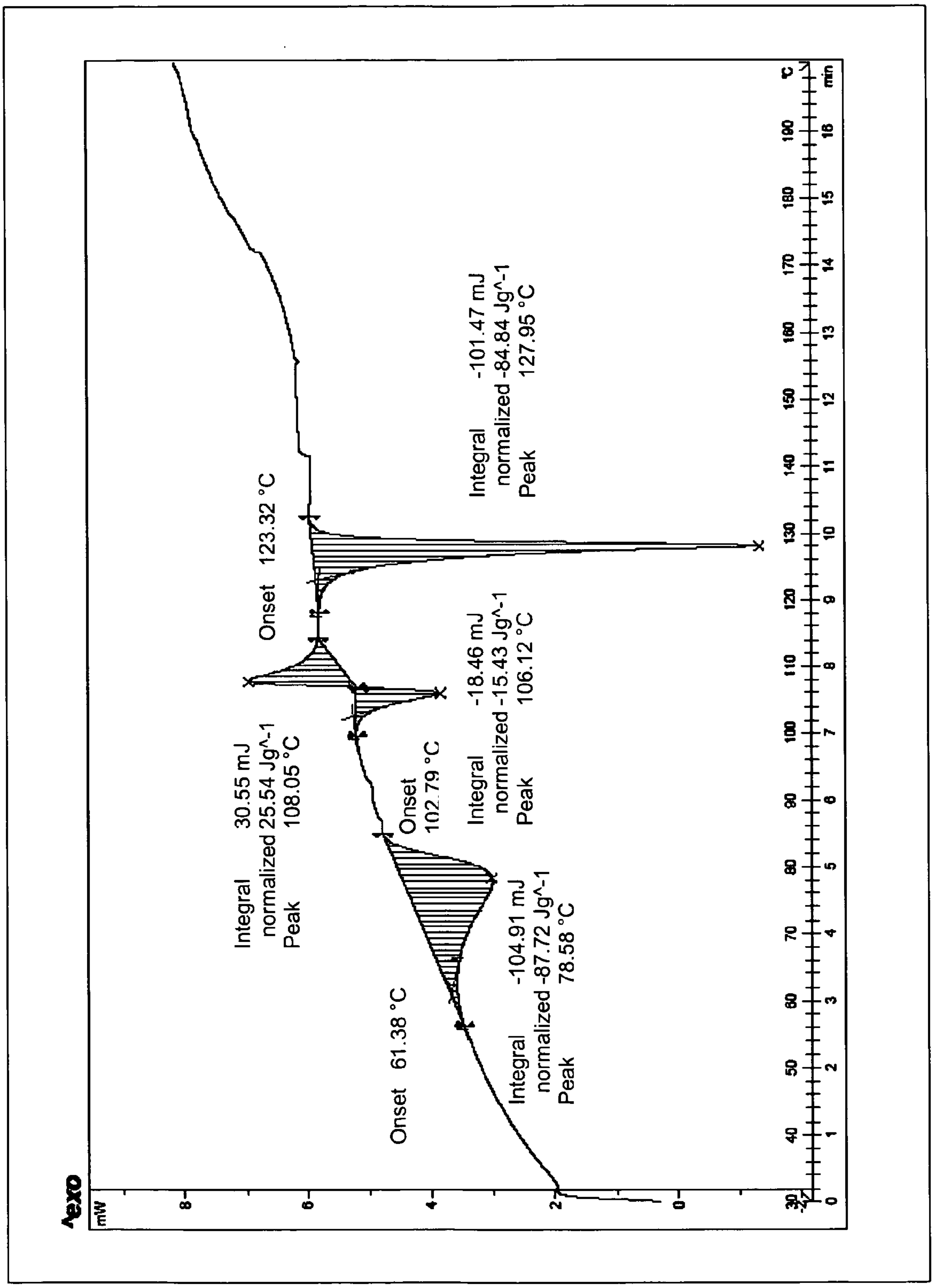
mW
^exo
Integral 30.55 mJ
normalized 25.54 Jg^-1
Peak 108.05 °C
Onset 123.32 °C
Integral -101.47 mJ
normalized -84.84 Jg^-1
Peak 127.95 °C
Onset 102.79 °C
Integral -18.46 mJ
normalized -15.43 Jg^-1
Peak 106.12 °C
Onset 61.38 °C
Integral -104.91 mJ
normalized -87.72 Jg^-1
Peak 78.58 °C
°C
min

Figure 4)

Figure 5)

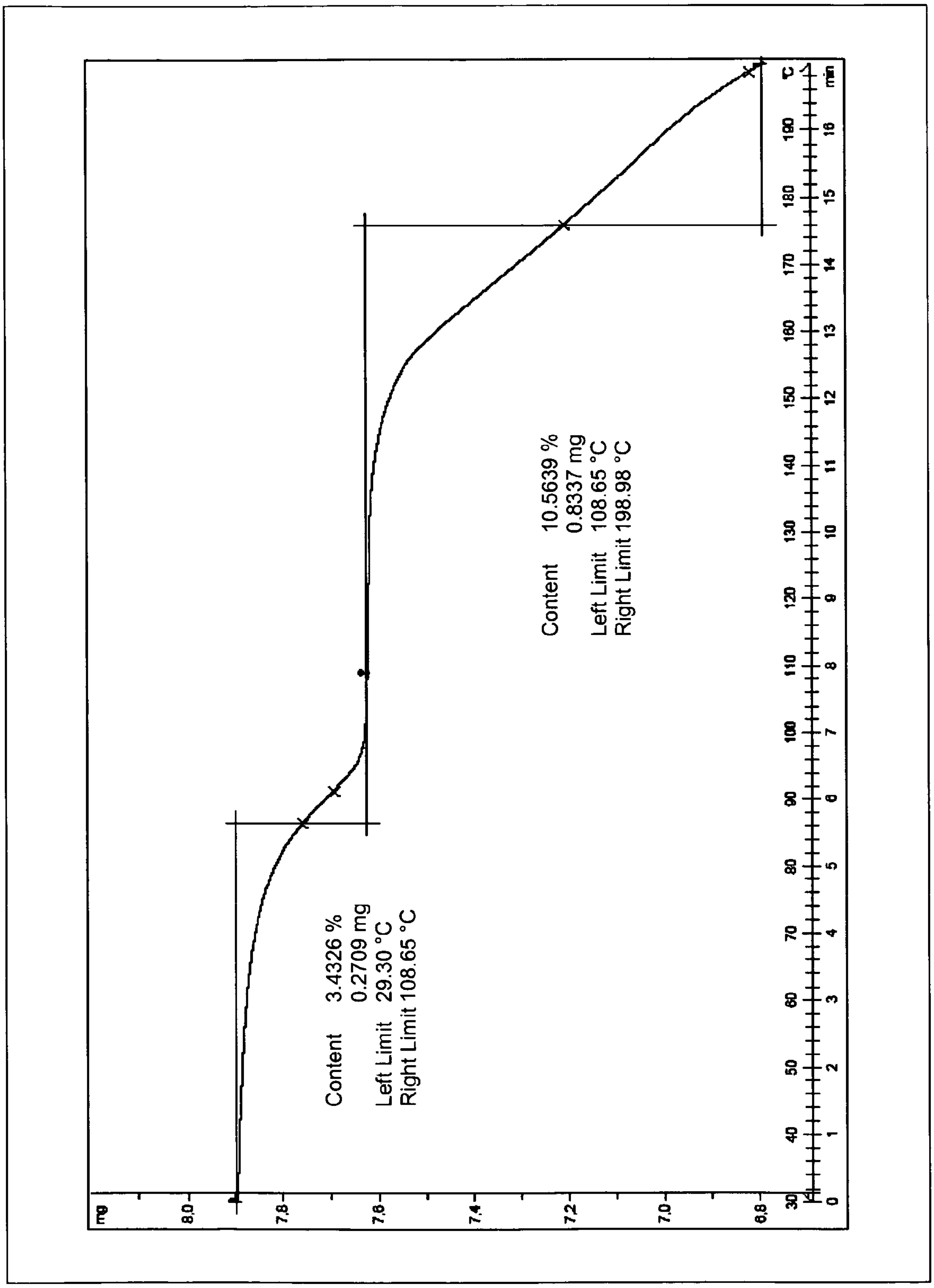

Content 3.4326 %
0.2709 mg
Left Limit 29.30 °C
Right Limit 108.65 °C
Content 10.5639 %
0.8337 mg
Left Limit 108.65 °C
Right Limit 198.98 °C
mg
8.0
7.8
7.6
7.4
7.2
7.0
6.8
°C
min

Figure 6)

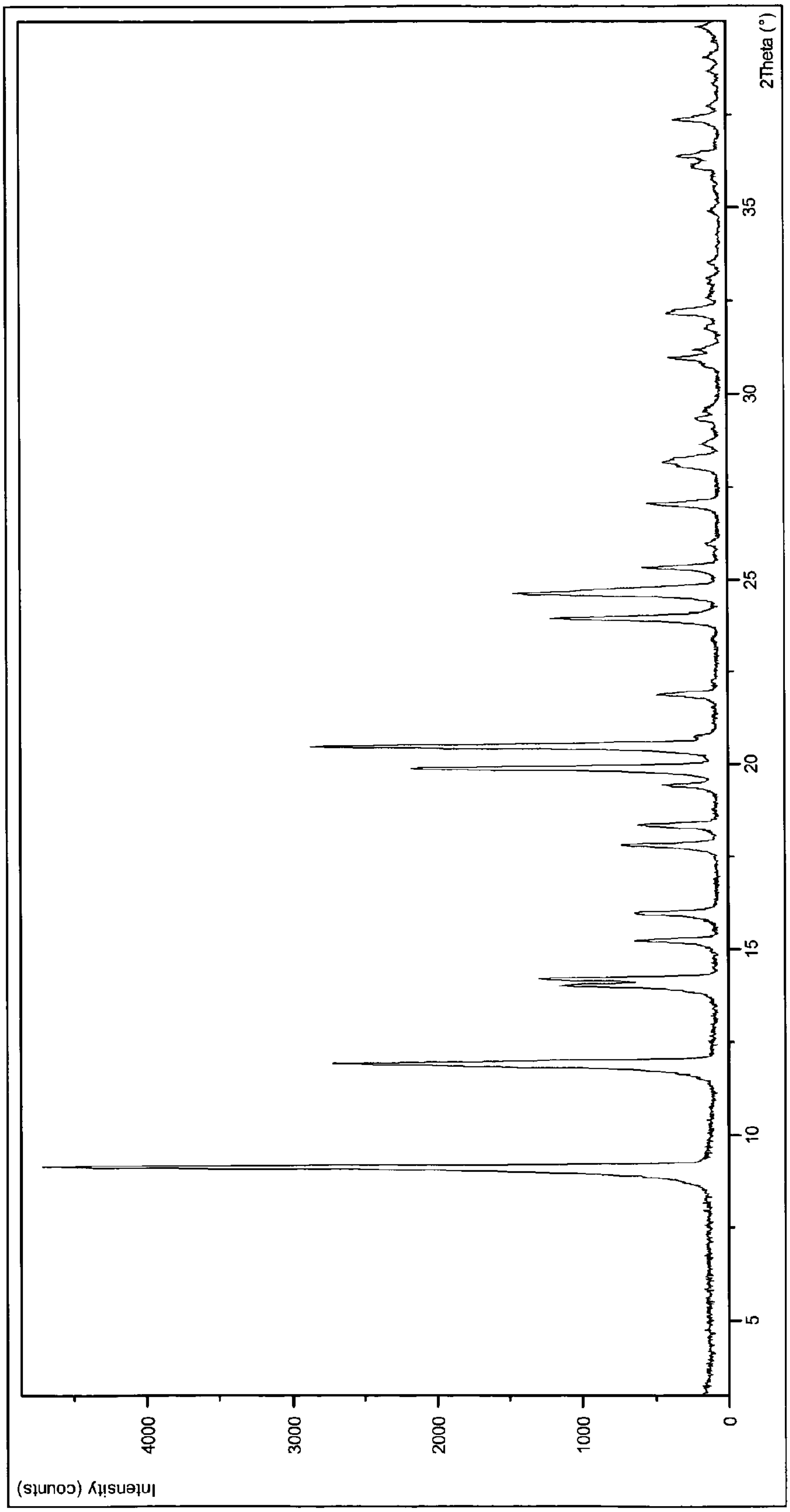
Intensity (counts)
4000
3000
2000
1000
0
5
10
15
20
25
30
35
2Theta (°)

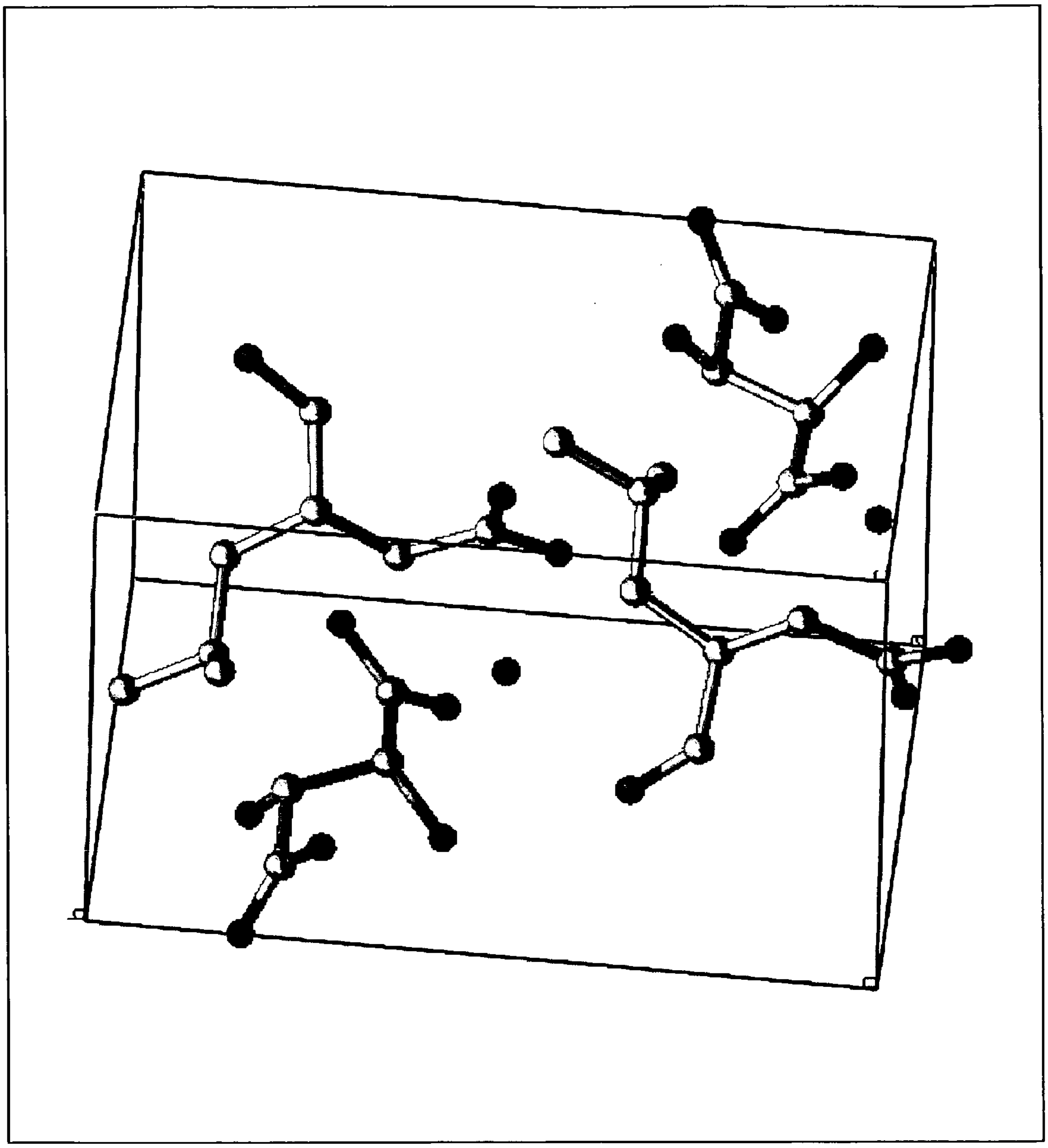

Figure 7)

Figure 8)

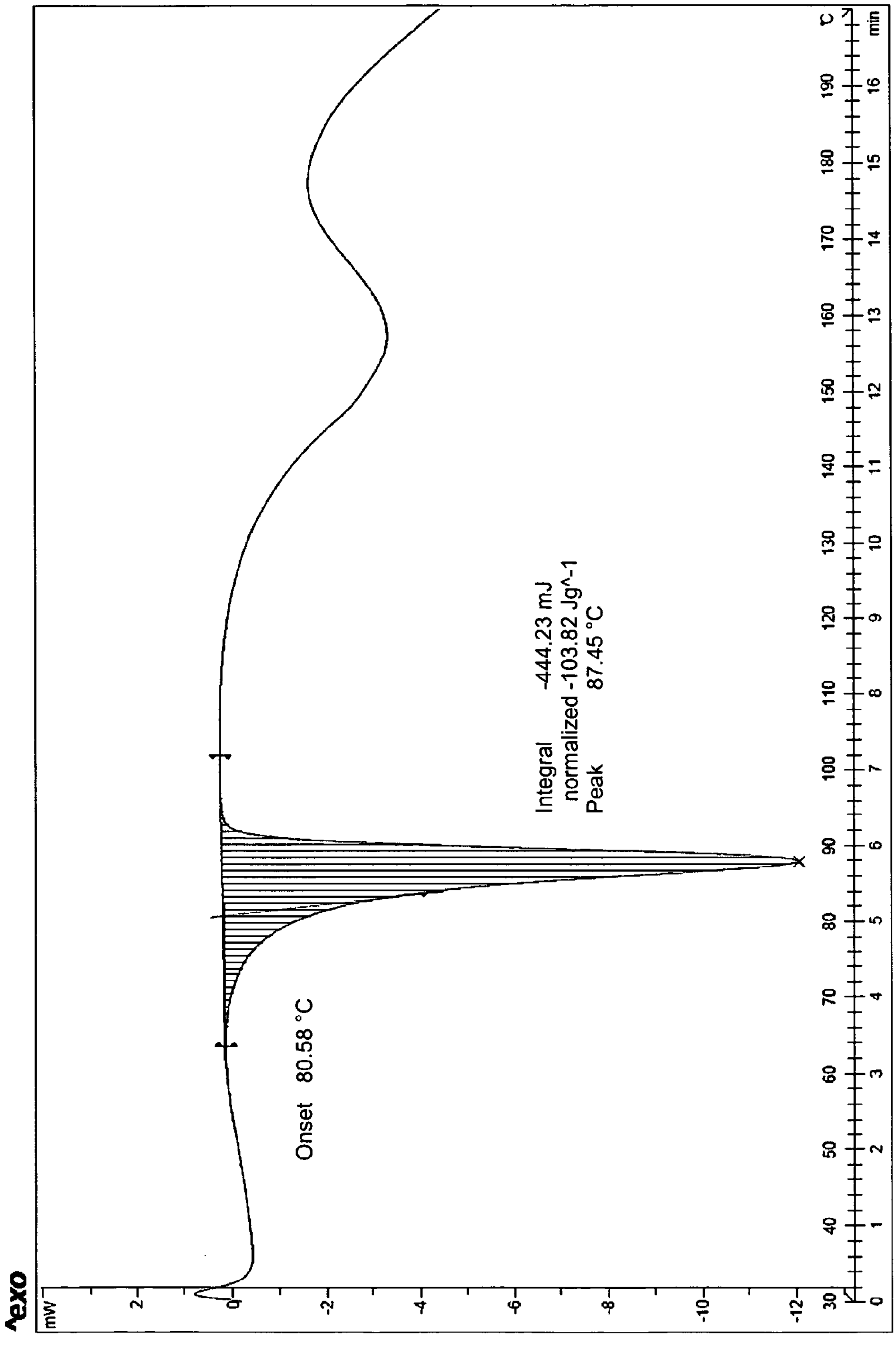
^exo
mW
2
0
-2
-4
-6
-8
-10
-12
Onset 80.58 °C
Integral -444.23 mJ
normalized -103.82 Jg^-1
Peak 87.45 °C
30 40 50 60 70 80 90 100 110 120 130 140 150 160 170 180 190 °C
0 1 2 3 4 5 6 7 8 9 10 11 12 13 14 15 16 min

Figure 9)

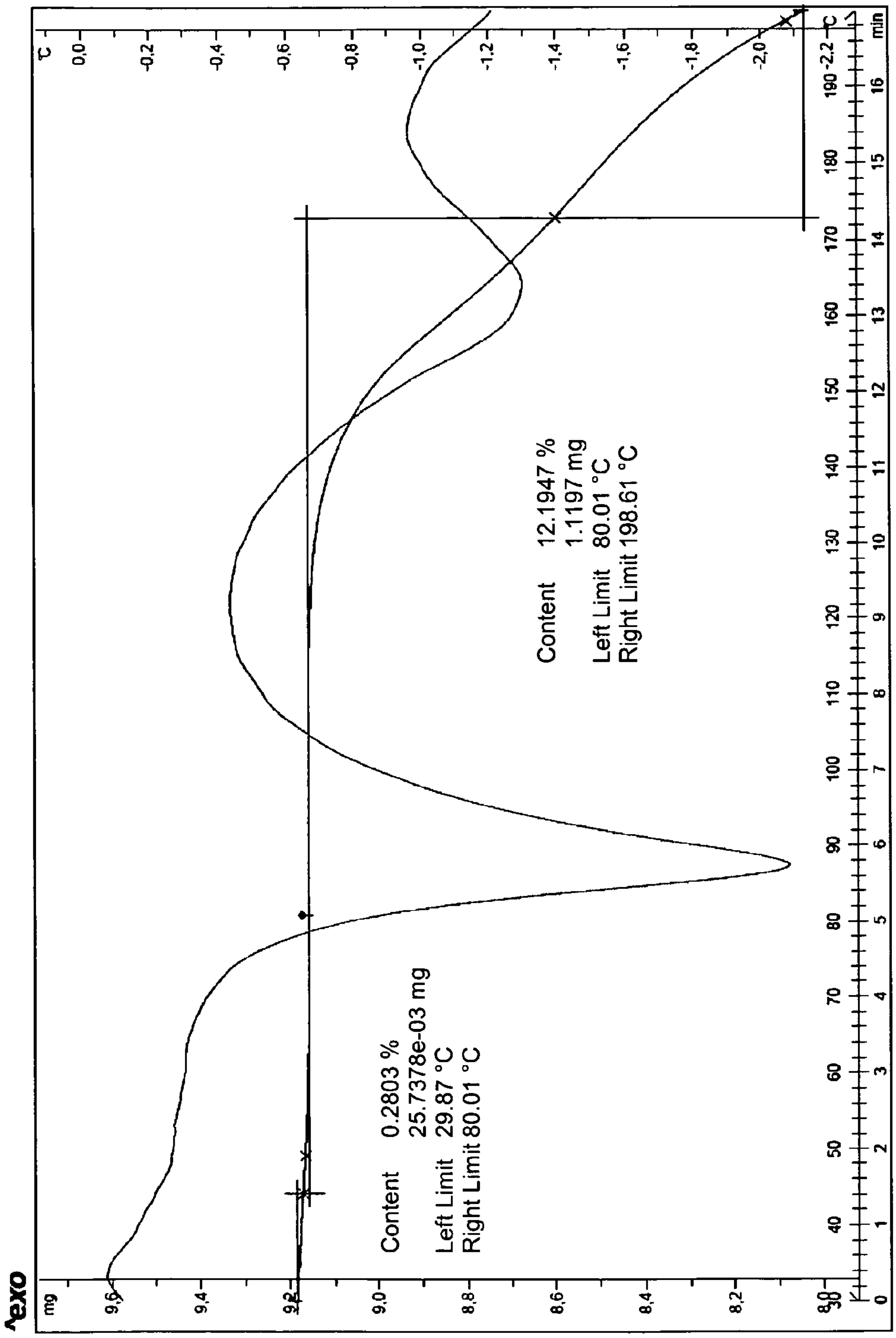
^exo
mg
9,6
9,4
9,2
9,0
8,8
8,6
8,4
8,2
8,0
°C
0,0
-0,2
-0,4
-0,6
-0,8
-1,0
-1,2
-1,4
-1,6
-1,8
-2,0
-2,2
Content 0.2803 %
25.7378e-03 mg
Left Limit 29.87 °C
Right Limit 80.01 °C
Content 12.1947 %
1.1197 mg
Left Limit 80.01 °C
Right Limit 198.61 °C
30
40
50
60
70
80
90
100
110
120
130
140
150
160
170
180
190
°C
0
1
2
3
4
5
6
7
8
9
10
11
12
13
14
15
16
min

Figure 10)

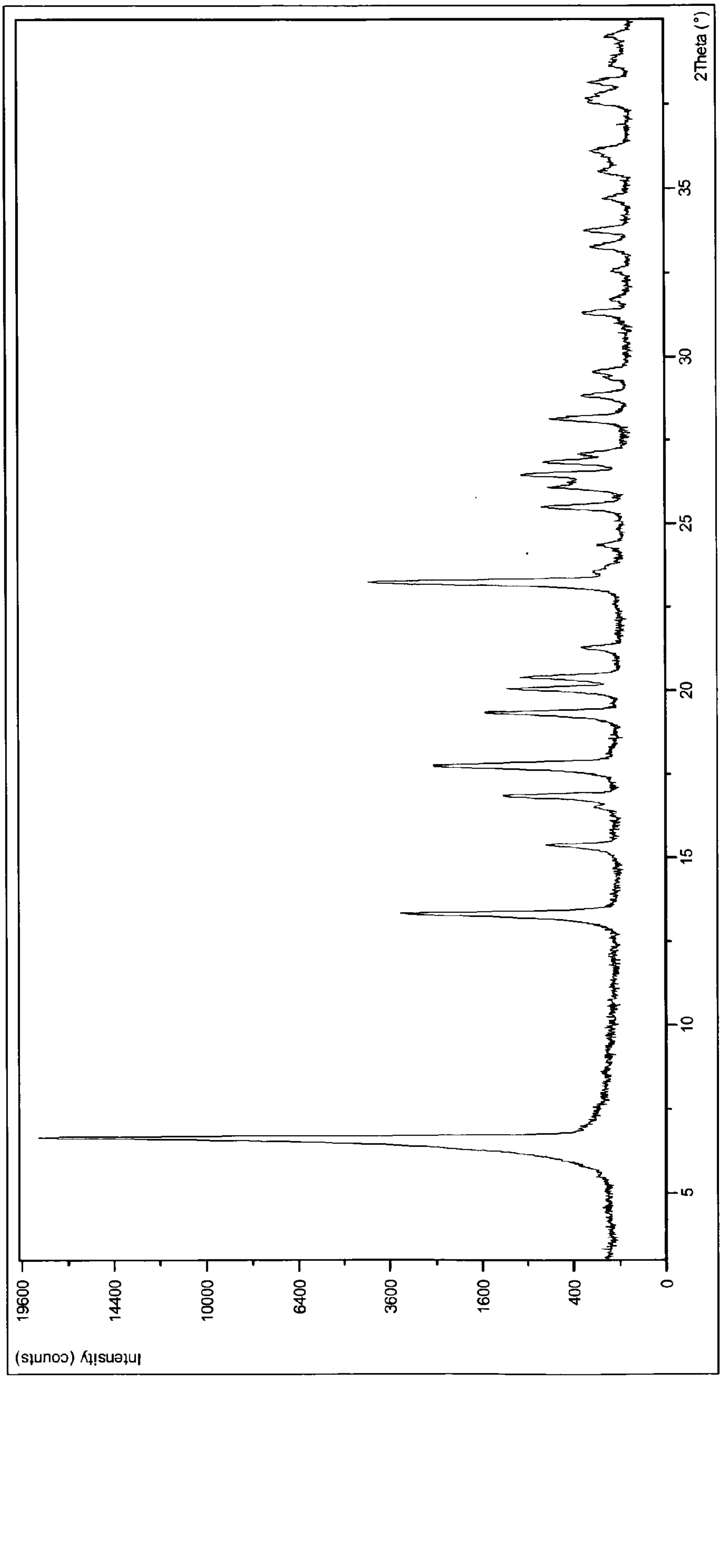
Intensity (counts)
19600
14400
10000
6400
3600
1600
400
0
5
10
15
20
25
30
35
2Theta (°)

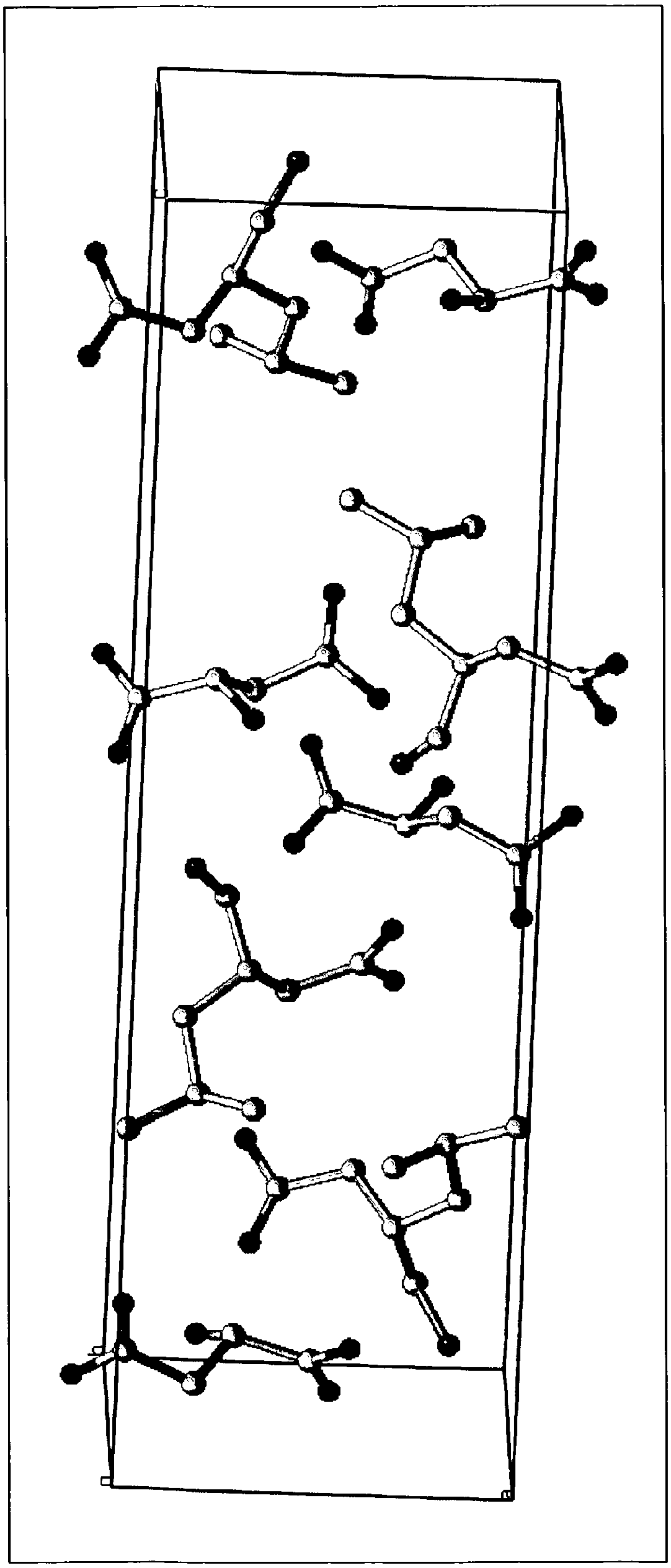

Figure 11)

Figure 12)

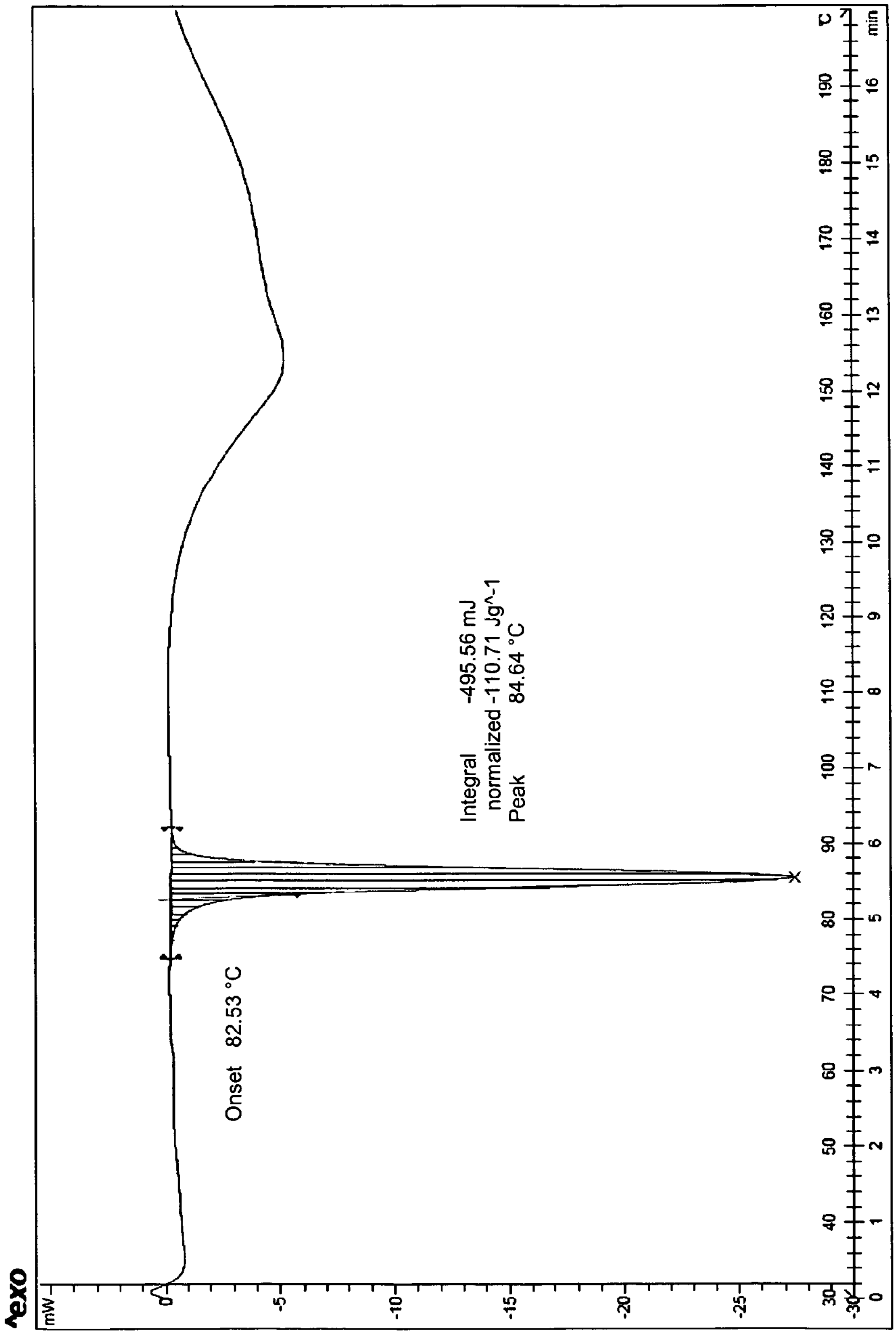
^exo
mW
0
-5
-10
-15
-20
-25
-30
Onset 82.53 °C
Integral -495.56 mJ
normalized -110.71 Jg^-1
Peak 84.64 °C
30 40 50 60 70 80 90 100 110 120 130 140 150 160 170 180 190 °C
0 1 2 3 4 5 6 7 8 9 10 11 12 13 14 15 16 min

Figure 13)

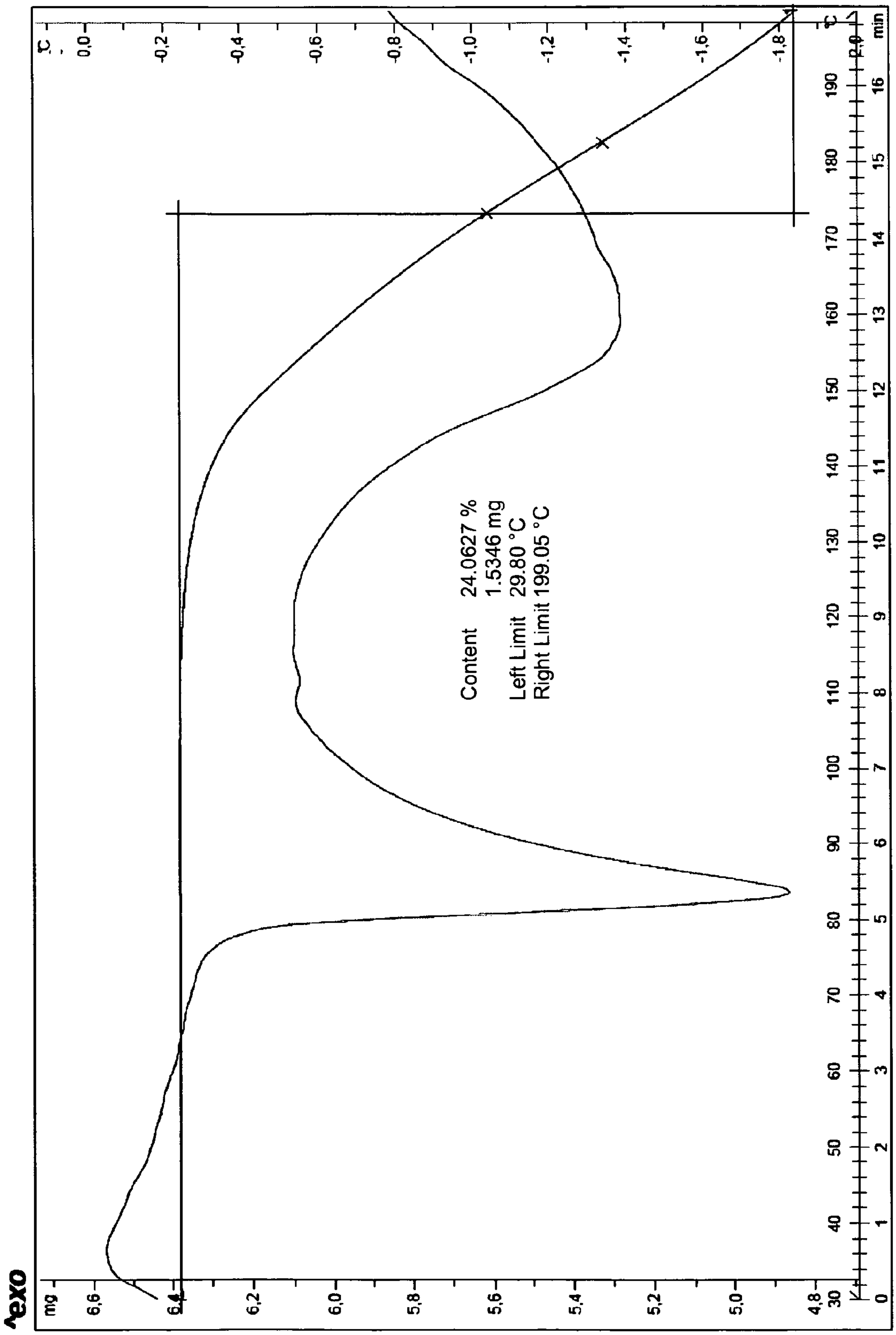
^exo
mg
°C
min
Content 24.0627 %
1.5346 mg
Left Limit 29.80 °C
Right Limit 199.05 °C

Figure 14)

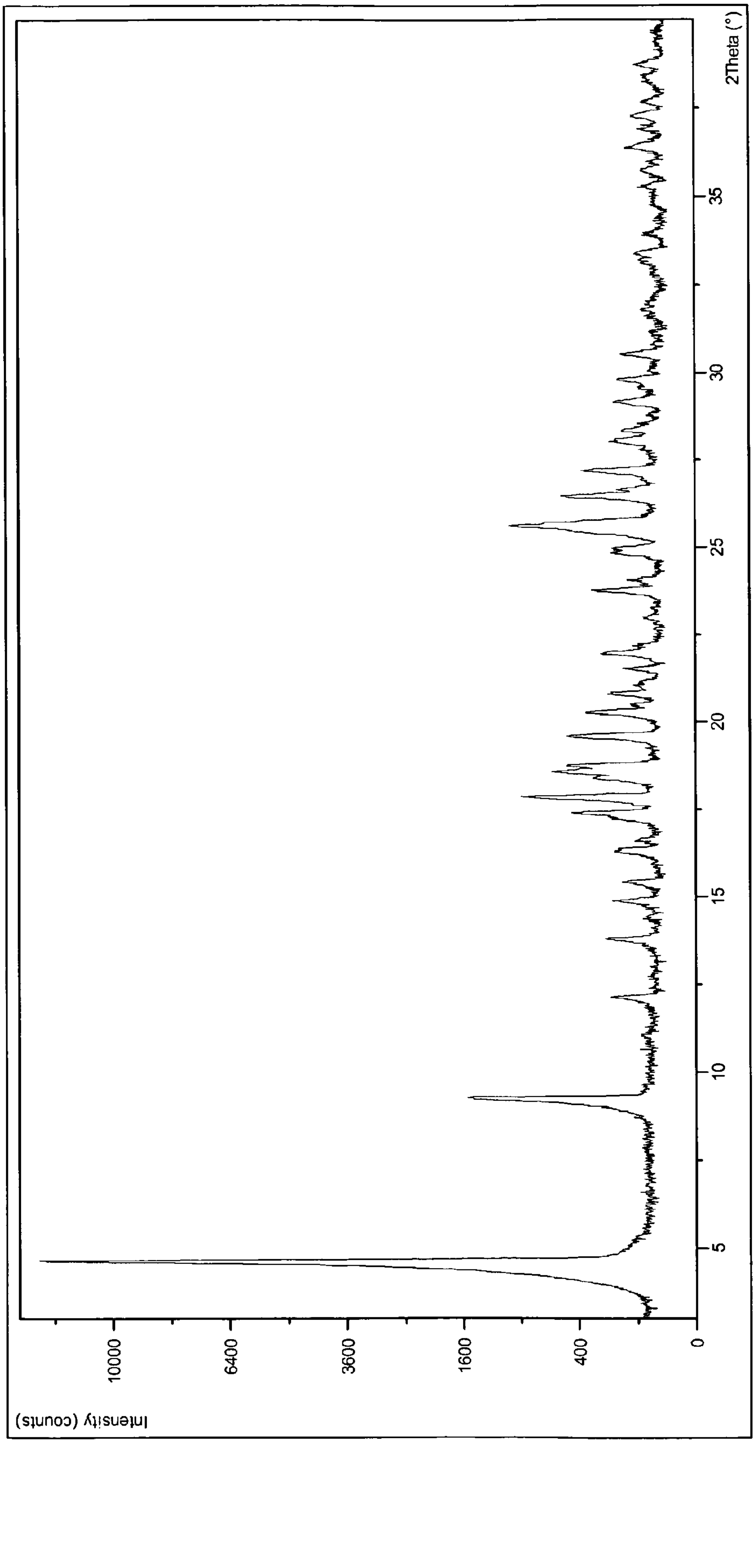
Intensity (counts)
10000
6400
3600
1600
400
0
5
10
15
20
25
30
35
2Theta (°)

Figure 15)

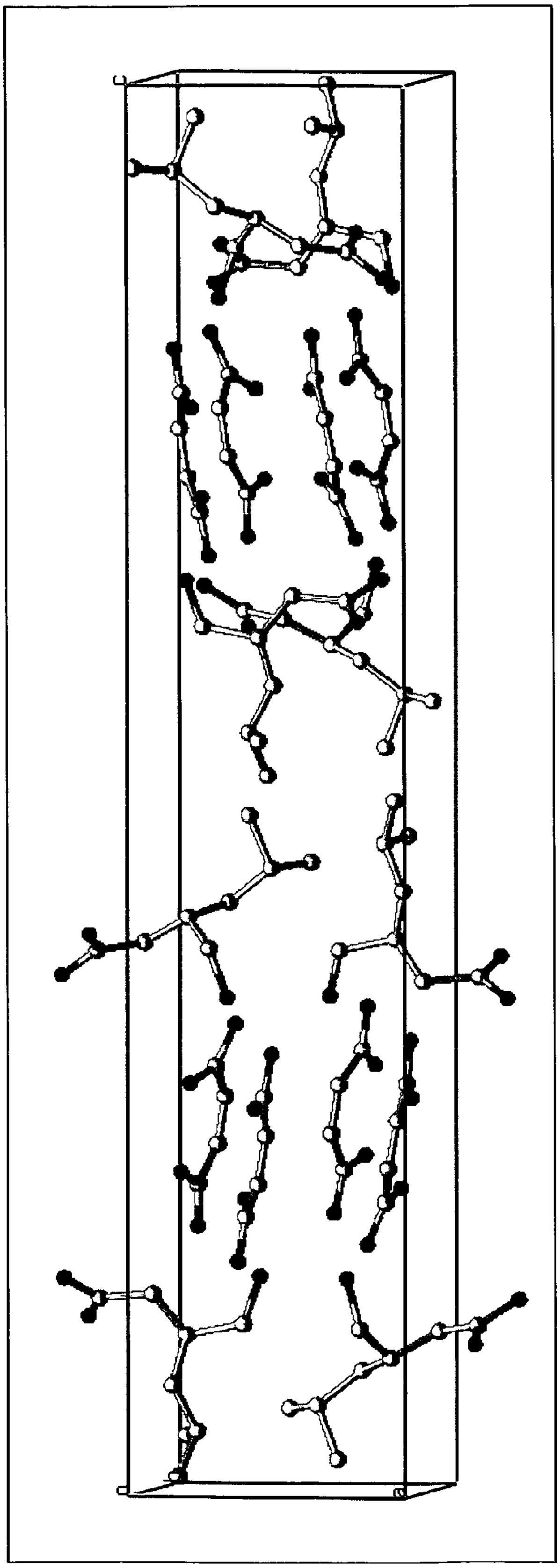

Figure 16)

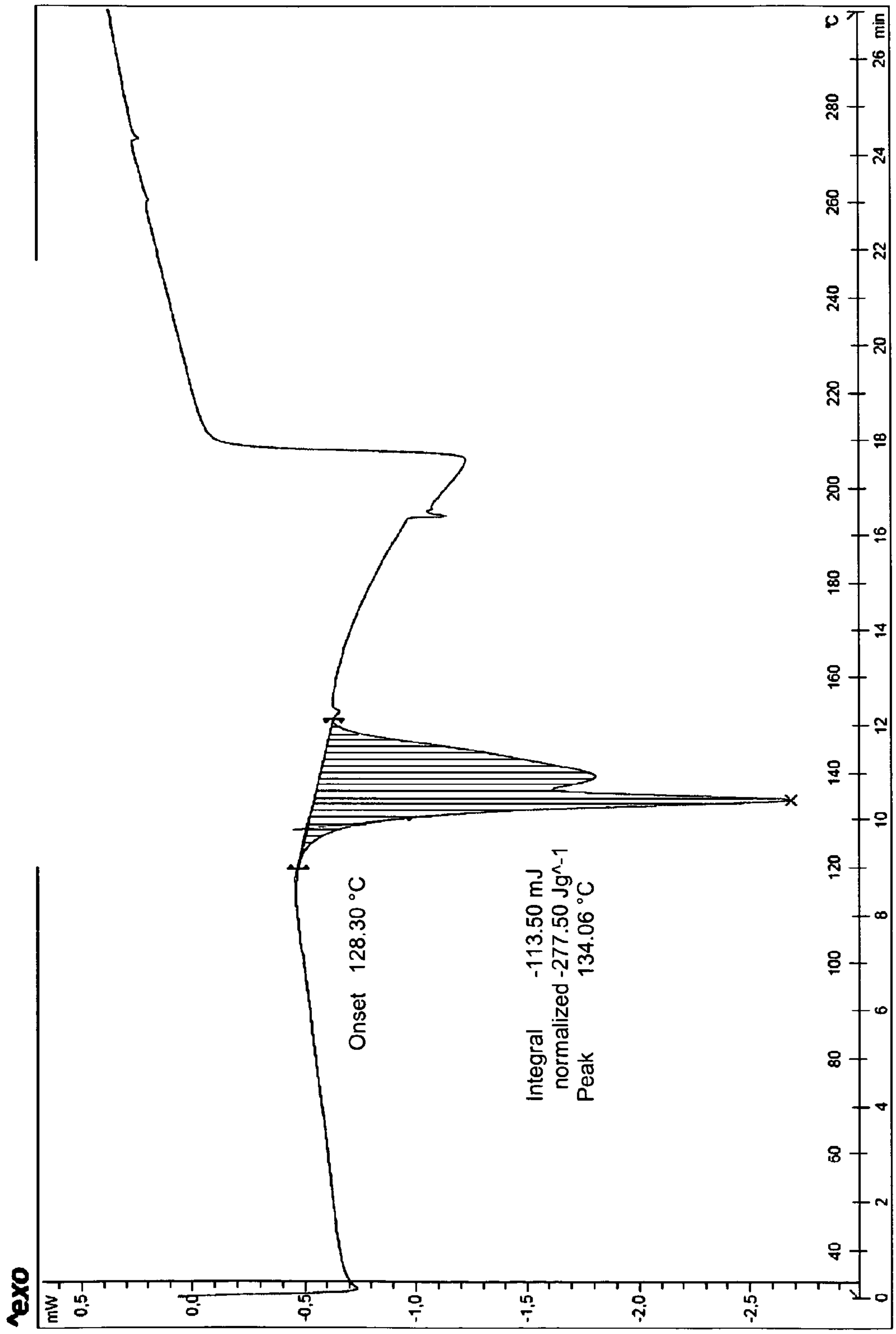
^exo
mW
Onset 128.30 °C
Integral -113.50 mJ
normalized -277.50 Jg^-1
Peak 134.06 °C
°C
min

Figure 17)

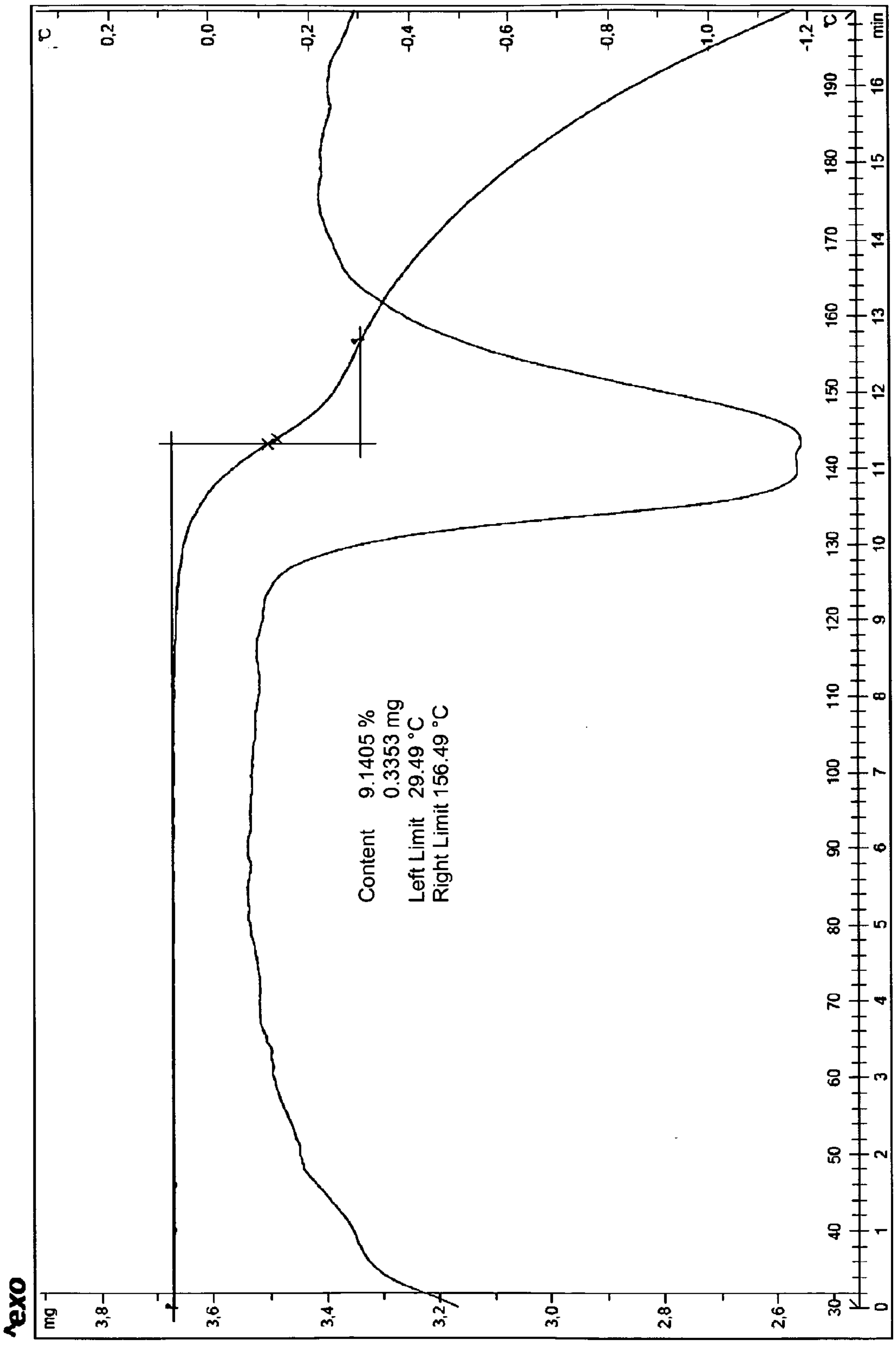
^exo
mg
3,8
3,6
3,4
3,2
3,0
2,8
2,6
°C
0,2
0,0
-0,2
-0,4
-0,6
-0,8
-1,0
-1,2
°C
30
40
50
60
70
80
90
100
110
120
130
140
150
160
170
180
190
°C
0
1
2
3
4
5
6
7
8
9
10
11
12
13
14
15
16
min
Content 9.1405 %
0.3353 mg
Left Limit 29.49 °C
Right Limit 156.49 °C

Figure 18)

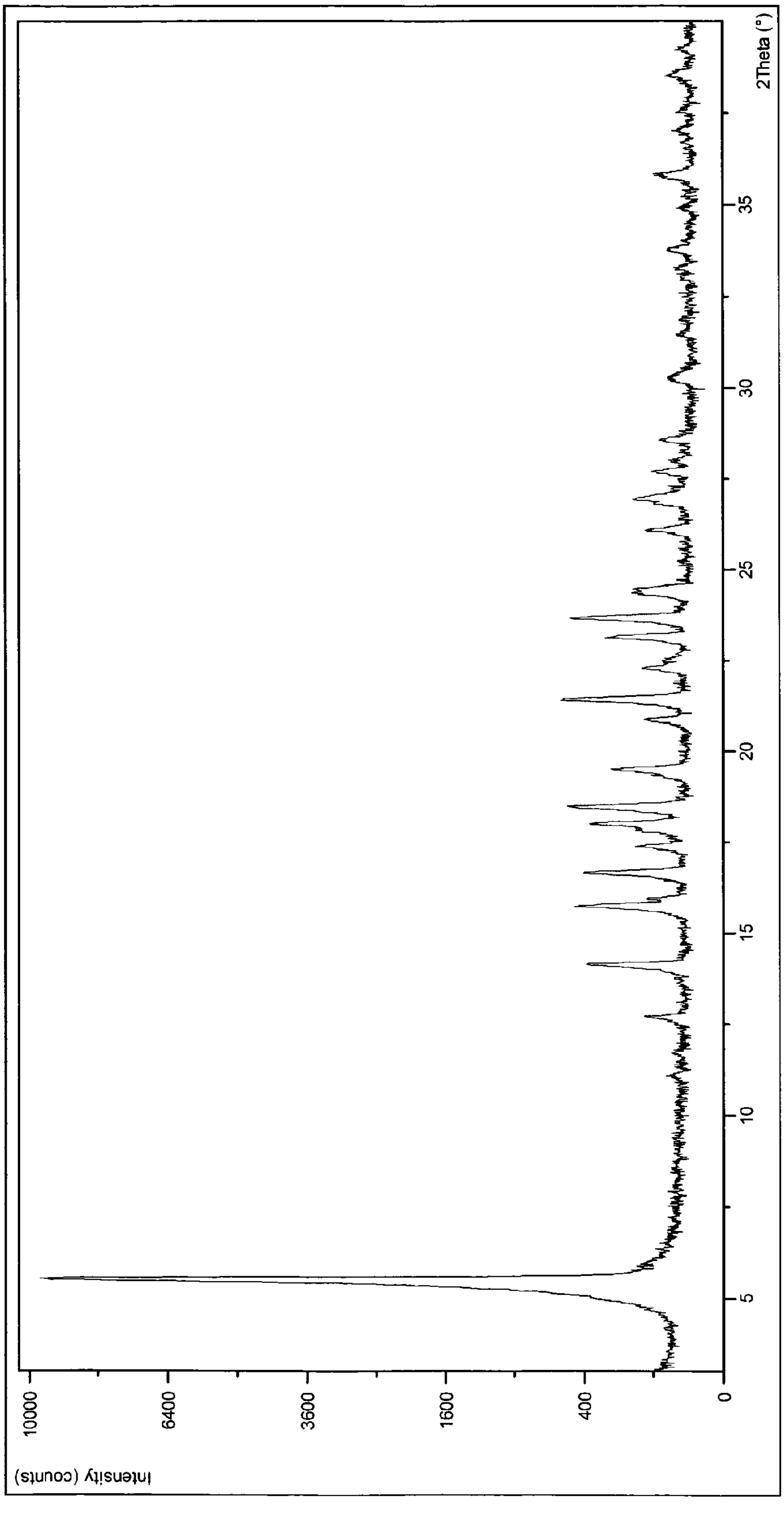
Intensity (counts)
10000
6400
3600
1600
400
0
5
10
15
20
25
30
35
2Theta (°)

Figure 19)

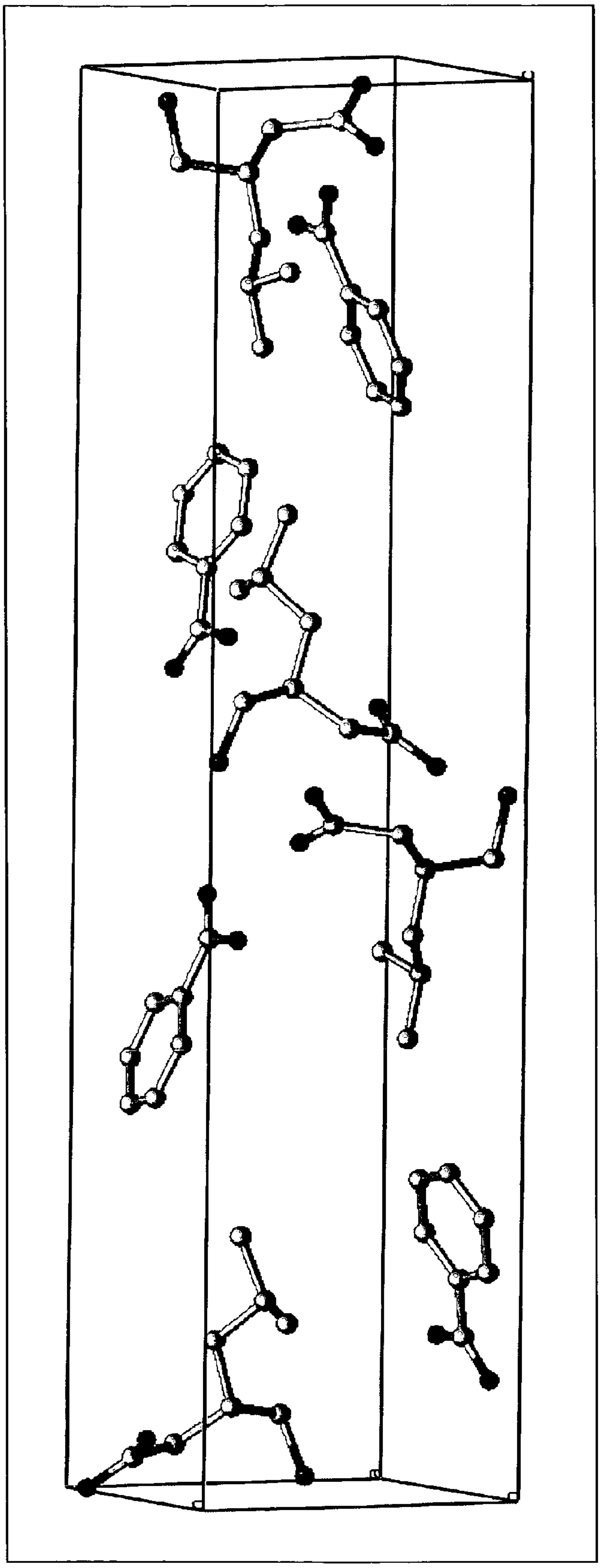

Figure 20)

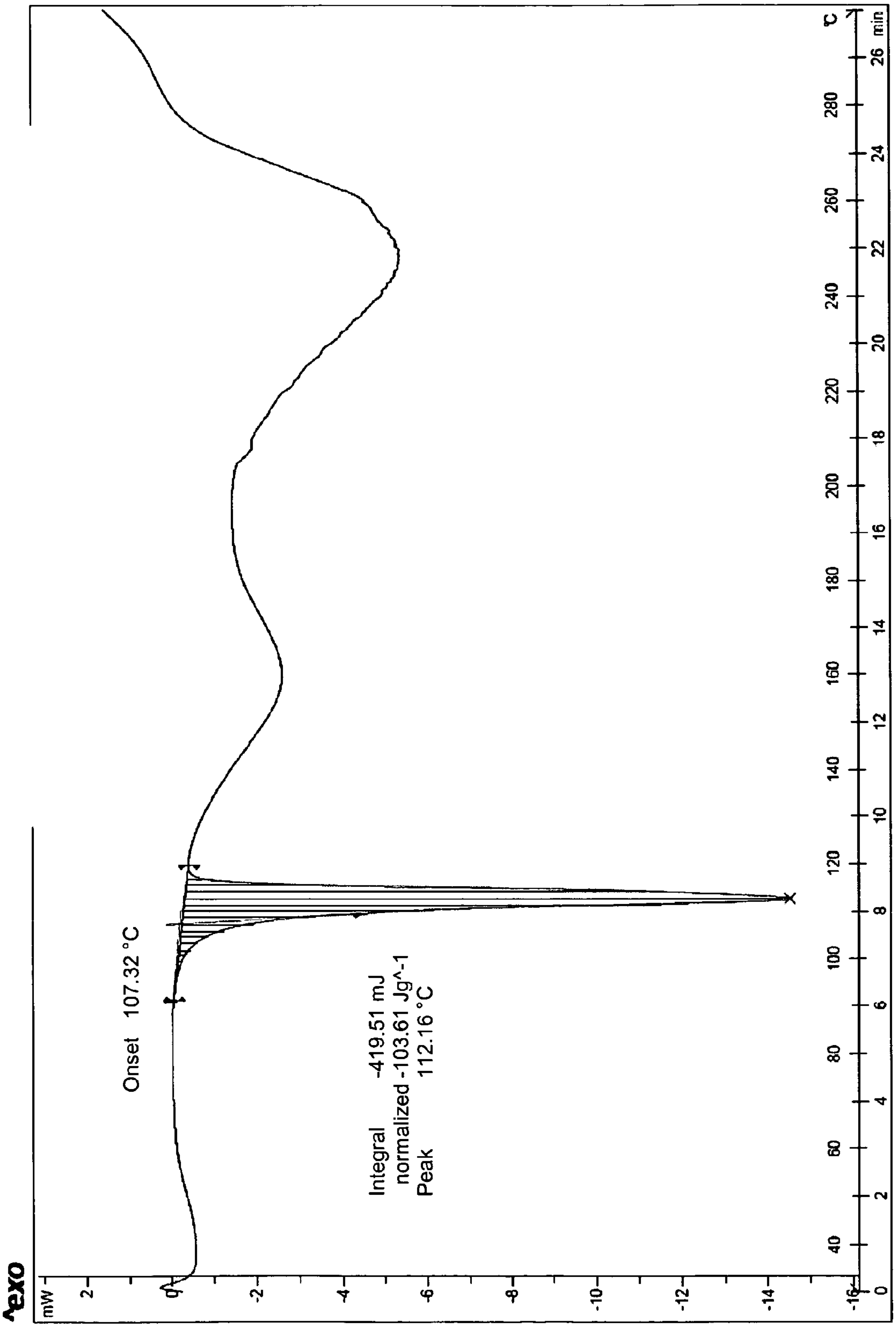
^exo
mW
Onset 107.32 °C
Integral -419.51 mJ
normalized -103.61 Jg^-1
Peak 112.16 °C
°C
min

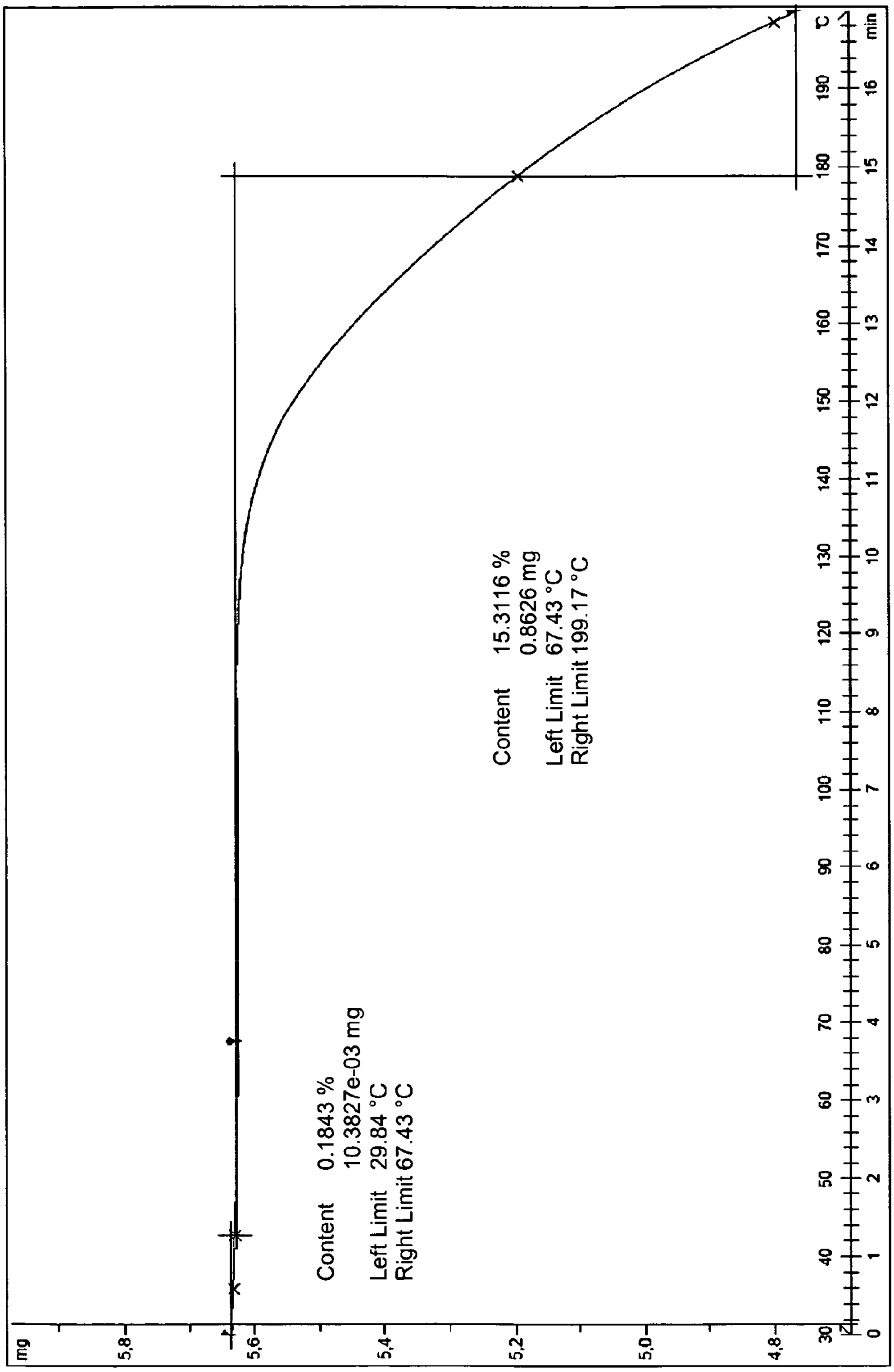
mg
5,8
5,6
5,4
5,2
5,0
4,8
Content 0.1843 %
10.3827e-03 mg
Left Limit 29.84 °C
Right Limit 67.43 °C
Content 15.3116 %
0.8626 mg
Left Limit 67.43 °C
Right Limit 199.17 °C
30 40 50 60 70 80 90 100 110 120 130 140 150 160 170 180 190 °C
0 1 2 3 4 5 6 7 8 9 10 11 12 13 14 15 16 min

Figure 21)

Figure 22)

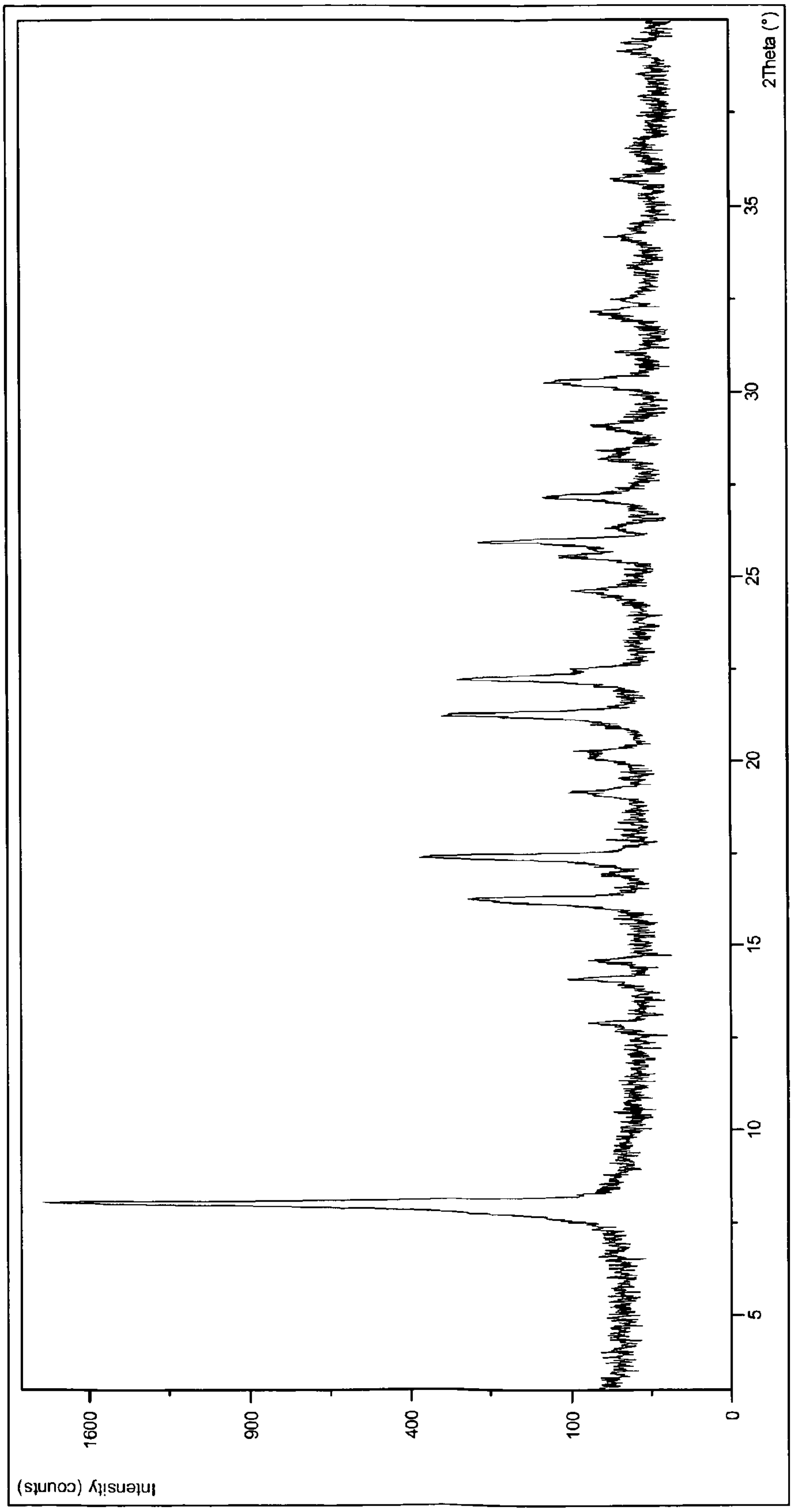

Figure 23)

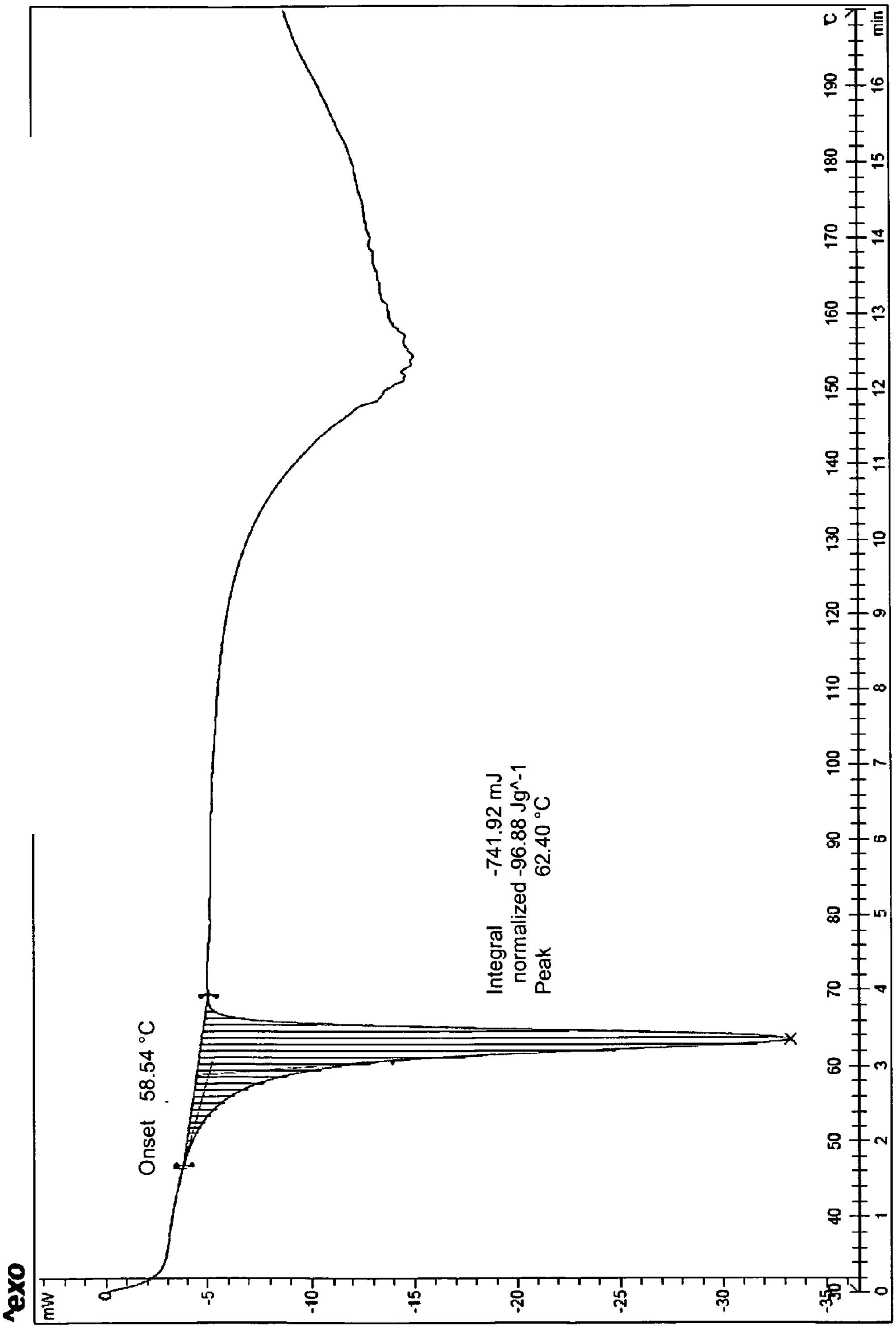
^exo
mW
Onset 58.54 °C
Integral -741.92 mJ
normalized -96.88 Jg^-1
Peak 62.40 °C
°C
min

Figure 24)

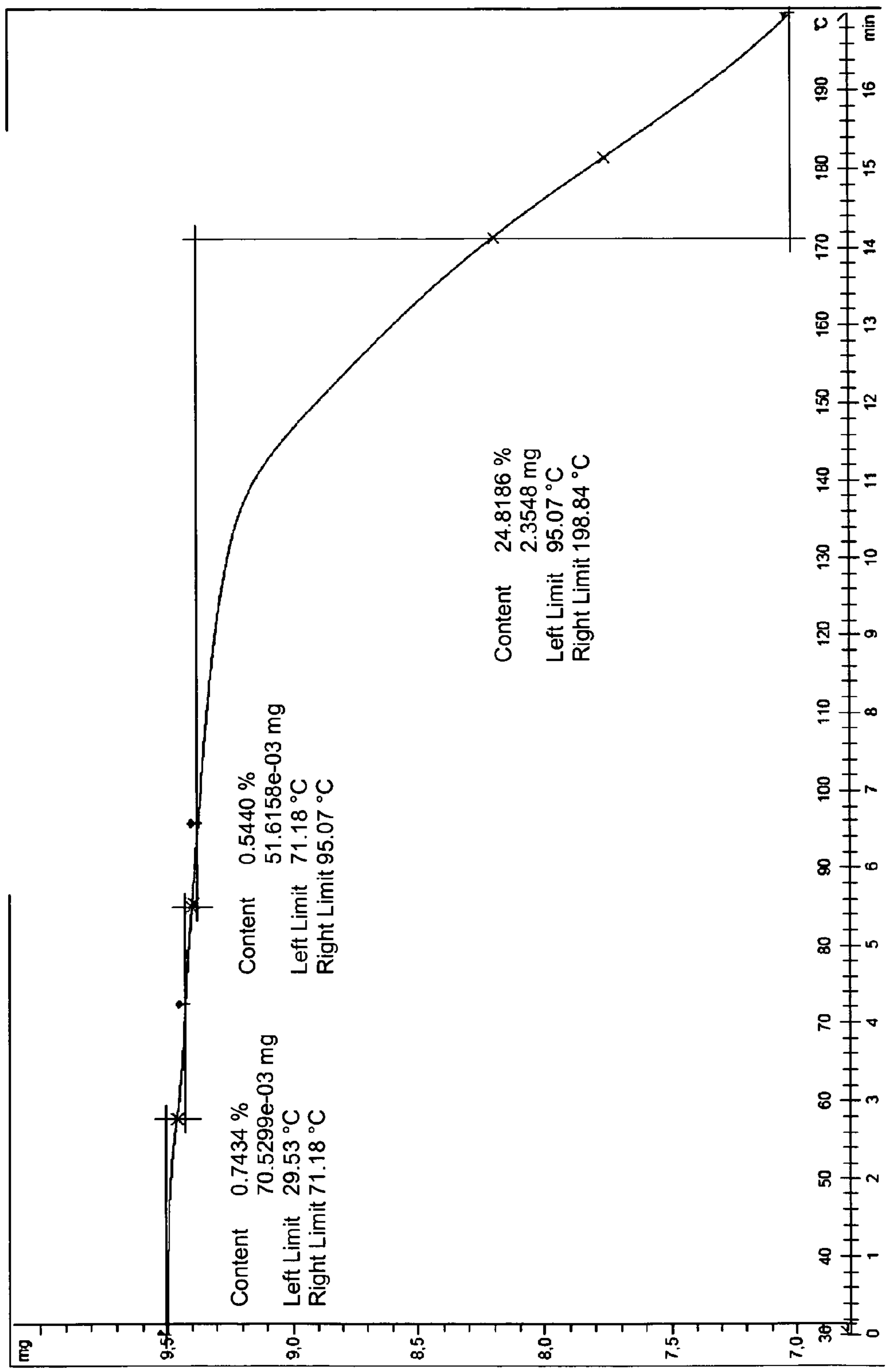
Content 0.7434 %
70.5299e-03 mg
Left Limit 29.53 °C
Right Limit 71.18 °C
Content 0.5440 %
51.6158e-03 mg
Left Limit 71.18 °C
Right Limit 95.07 °C
Content 24.8186 %
2.3548 mg
Left Limit 95.07 °C
Right Limit 198.84 °C
mg
9,5
9,0
8,5
8,0
7,5
7,0
°C
min

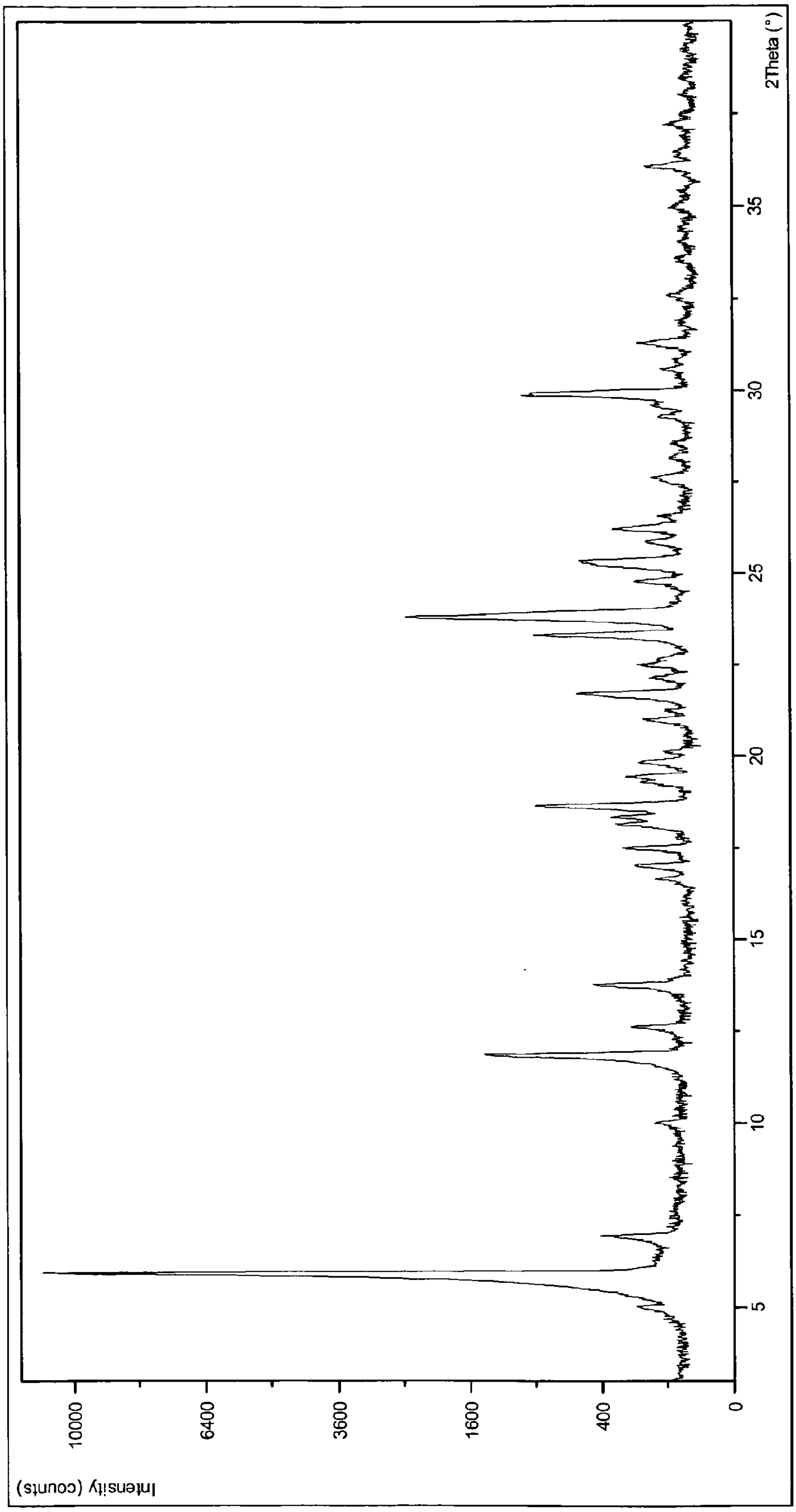
Intensity (counts)
10000
6400
3600
1600
400
0
5
10
15
20
25
30
35
2Theta (°)

Figure 25)

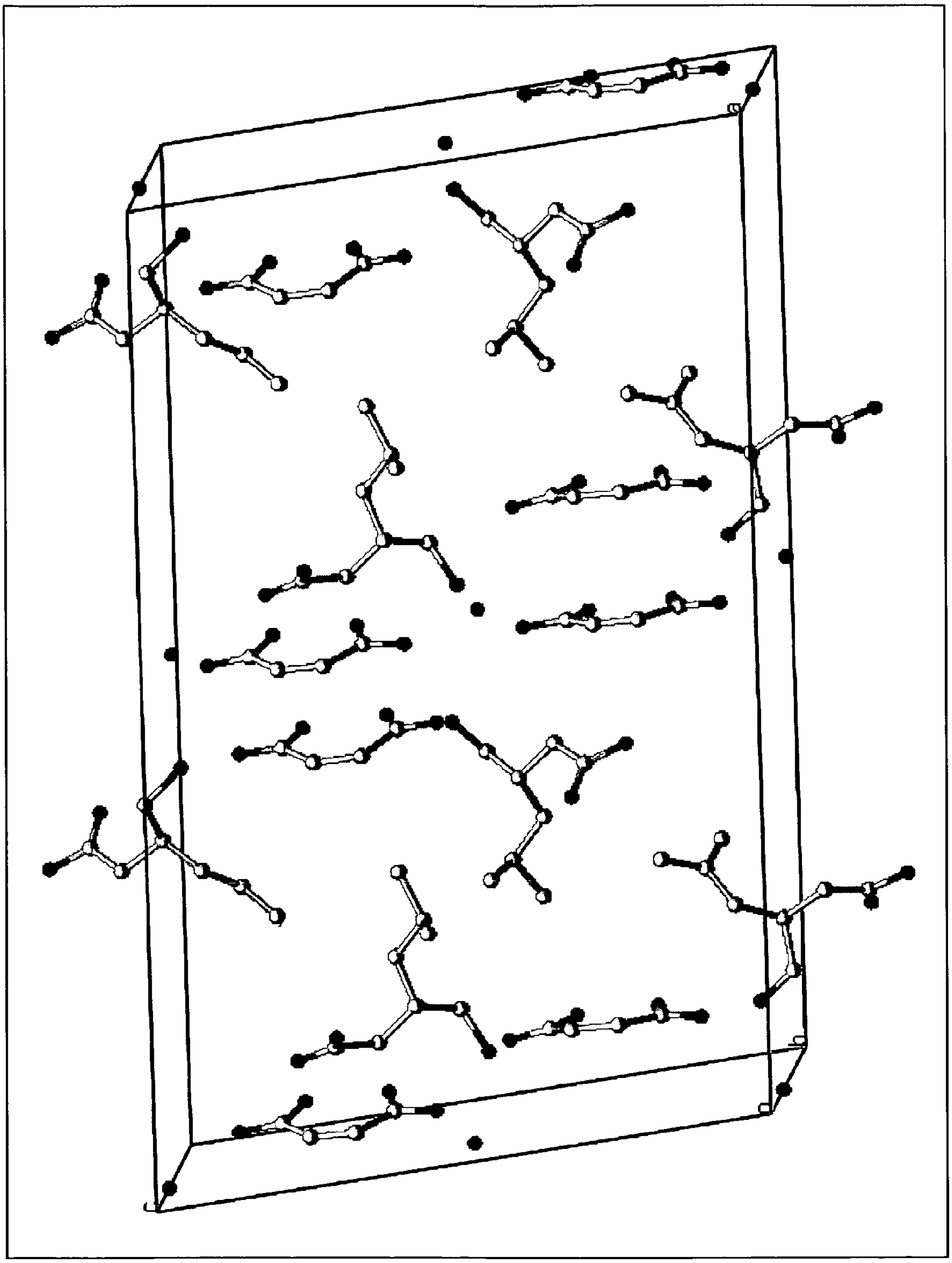

Figure 26)

Figure 27)

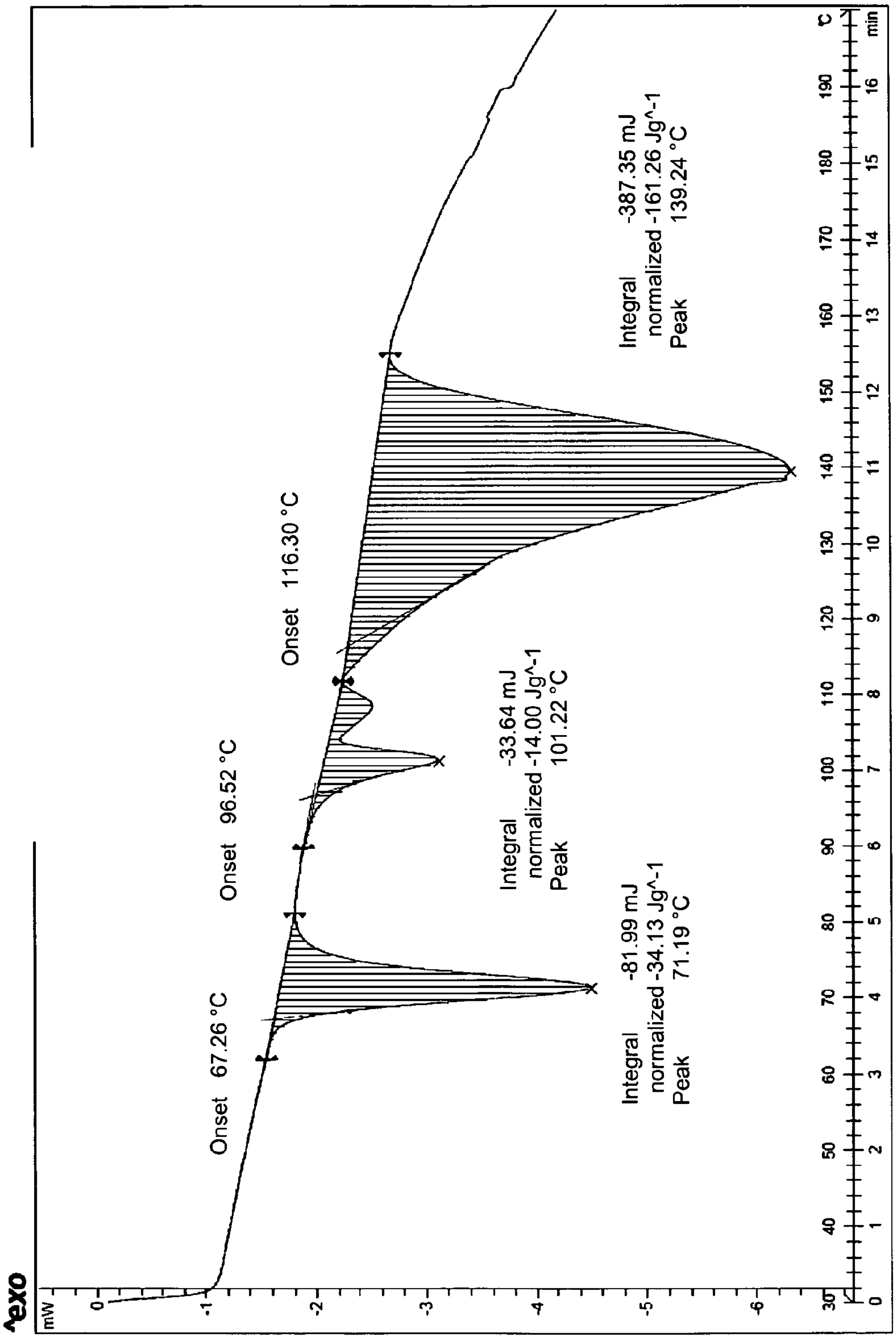
^exo
mW
Onset 67.26 °C
Onset 96.52 °C
Onset 116.30 °C
Integral -81.99 mJ
normalized -34.13 Jg^-1
Peak 71.19 °C
Integral -33.64 mJ
normalized -14.00 Jg^-1
Peak 101.22 °C
Integral -387.35 mJ
normalized -161.26 Jg^-1
Peak 139.24 °C
°C
min

Figure 28)

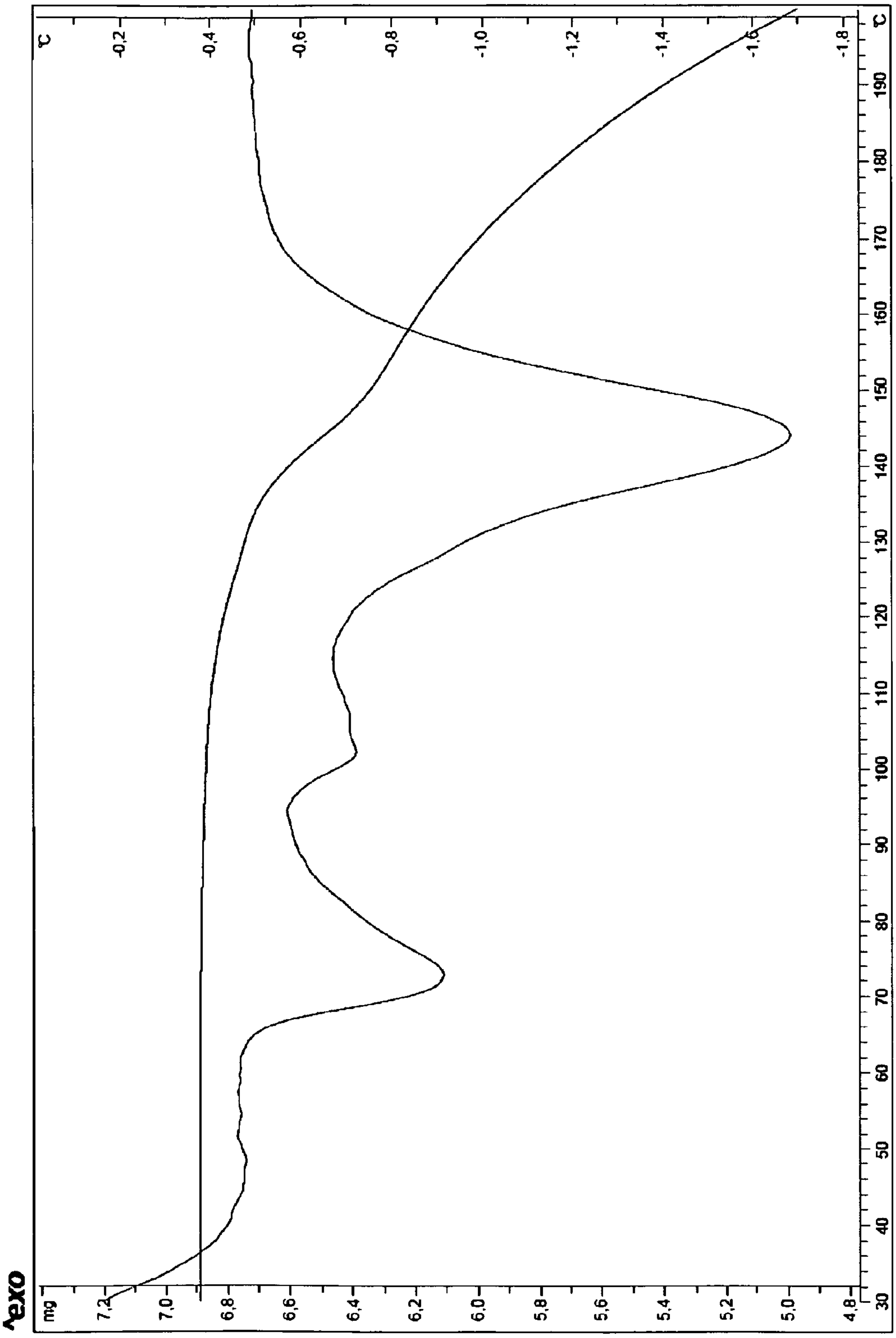
^exo
mg
7,2
7,0
6,8
6,6
6,4
6,2
6,0
5,8
5,6
5,4
5,2
5,0
4,8
°C
-0,2
-0,4
-0,6
-0,8
-1,0
-1,2
-1,4
-1,6
-1,8
°C
30
40
50
60
70
80
90
100
110
120
130
140
150
160
170
180
190

Figure 29)

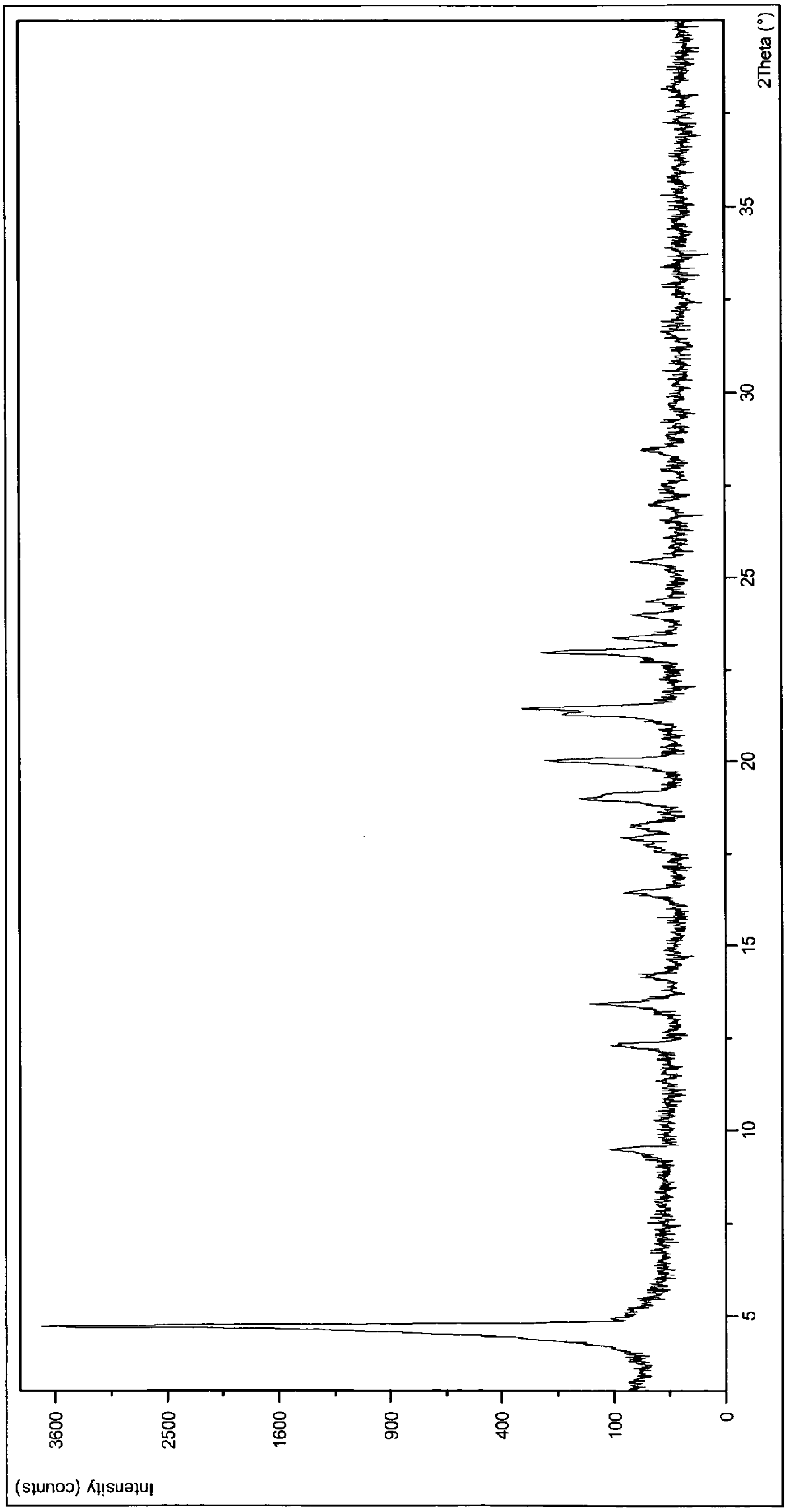
Intensity (counts)
3600
2500
1600
900
400
100
0
5
10
15
20
25
30
35
2Theta (°)

Figure 30)

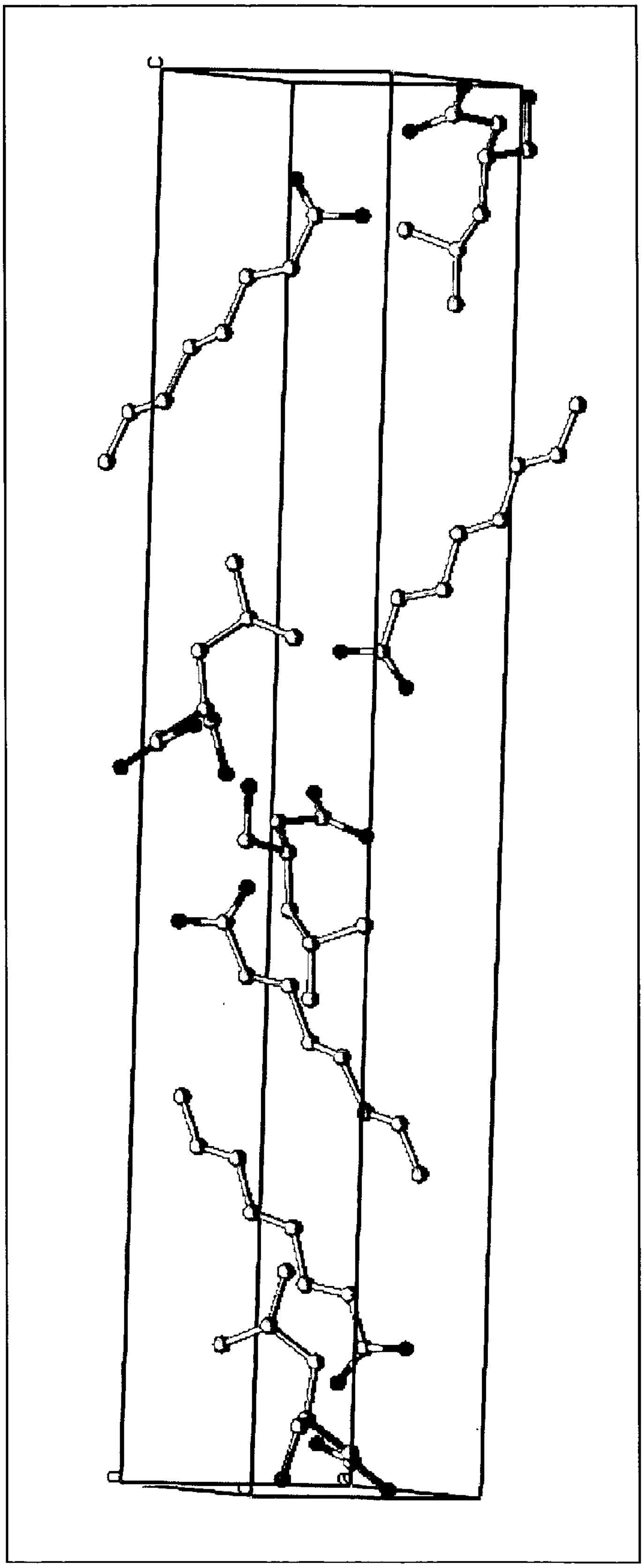

Figure 31)

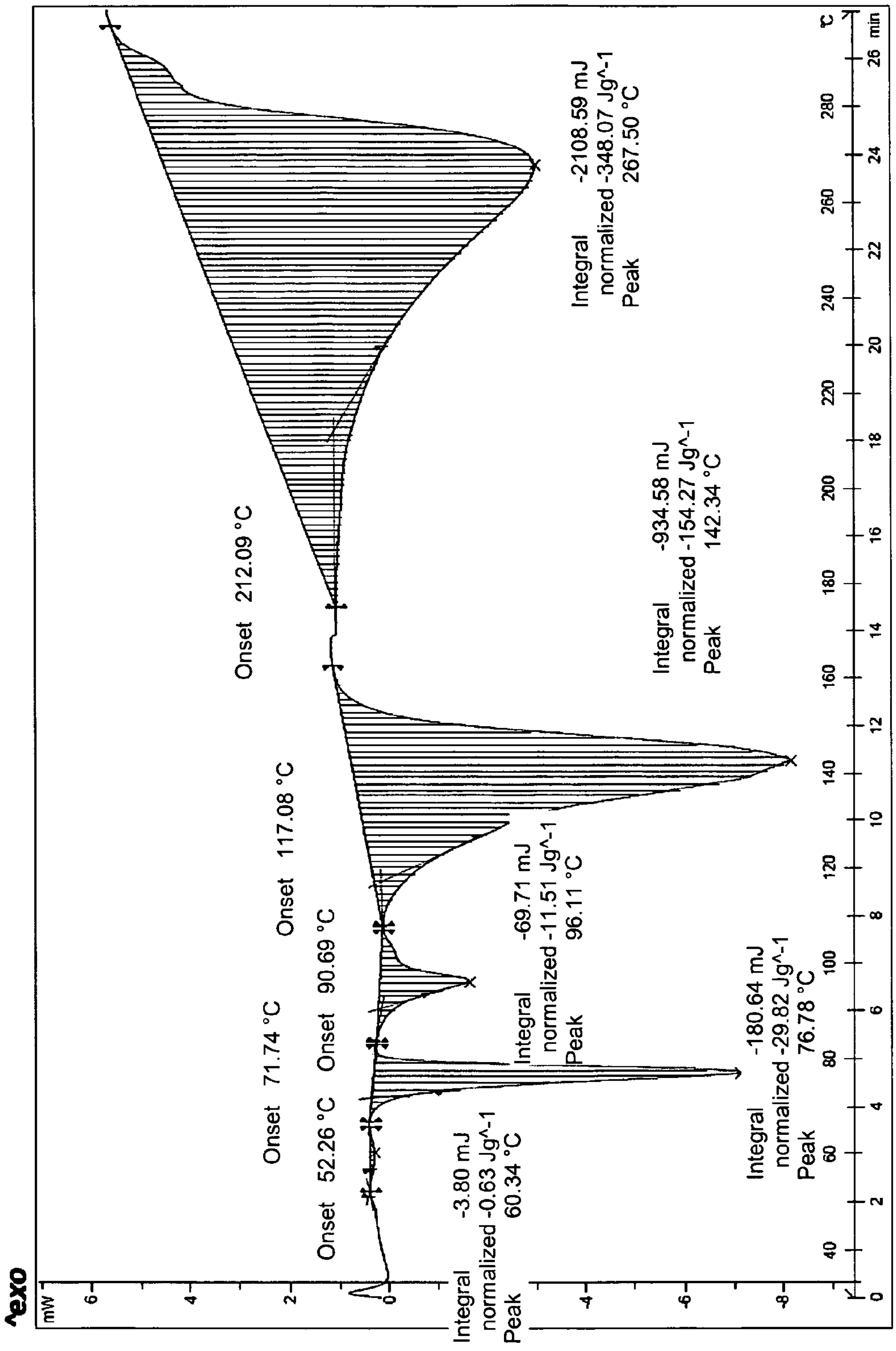

^exo
mW
Onset 52.26 °C
Onset 71.74 °C
Onset 90.69 °C
Onset 117.08 °C
Onset 212.09 °C
Integral -3.80 mJ
normalized -0.63 Jg^-1
Peak 60.34 °C
Integral -180.64 mJ
normalized -29.82 Jg^-1
Peak 76.78 °C
Integral -69.71 mJ
normalized -11.51 Jg^-1
Peak 96.11 °C
Integral -934.58 mJ
normalized -154.27 Jg^-1
Peak 142.34 °C
Integral -2108.59 mJ
normalized -348.07 Jg^-1
Peak 267.50 °C
°C
min

Figure 32)

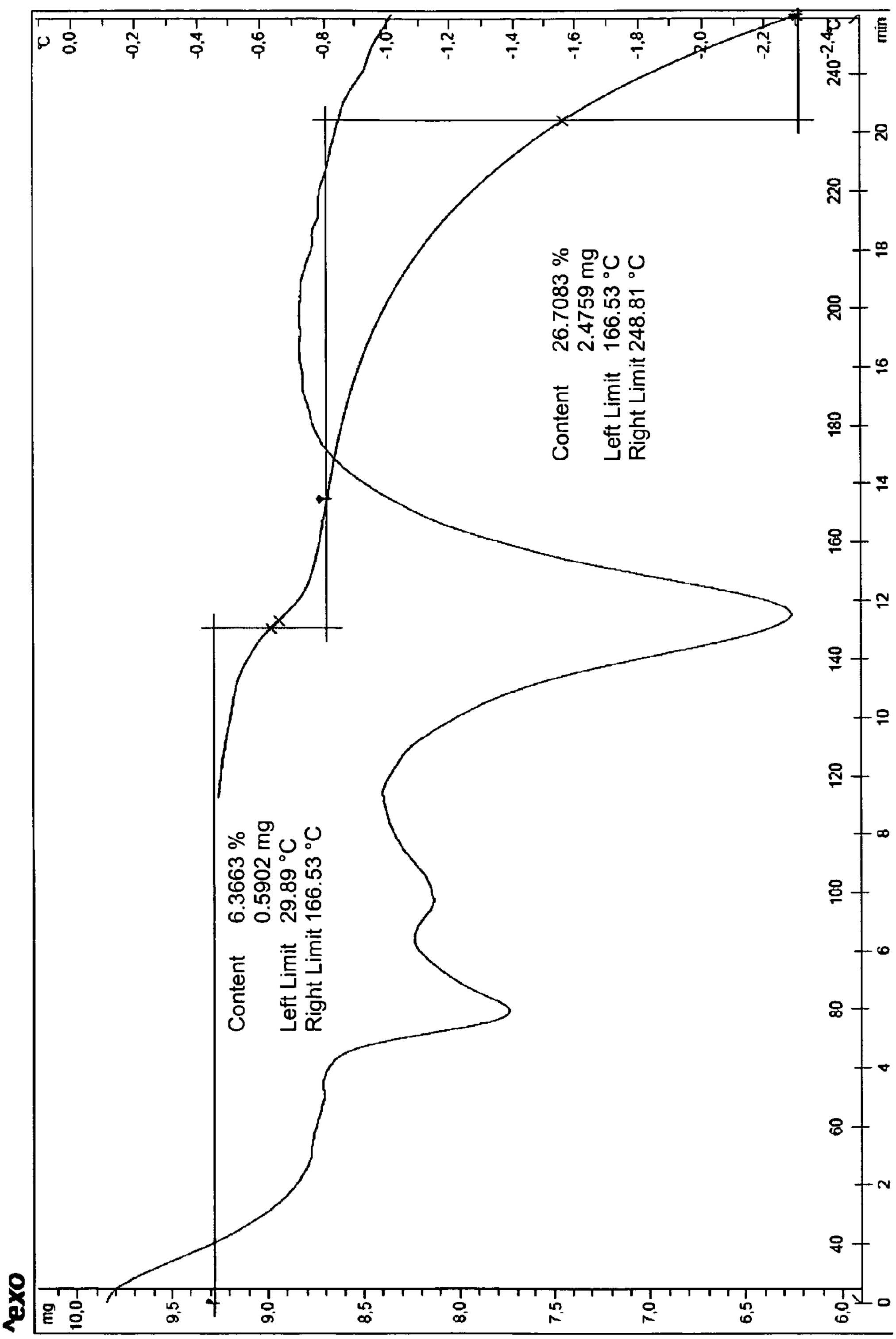
^exo
mg
°C
min
Content 6.3663 %
0.5902 mg
Left Limit 29.89 °C
Right Limit 166.53 °C
Content 26.7083 %
2.4759 mg
Left Limit 166.53 °C
Right Limit 248.81 °C

Figure 33)

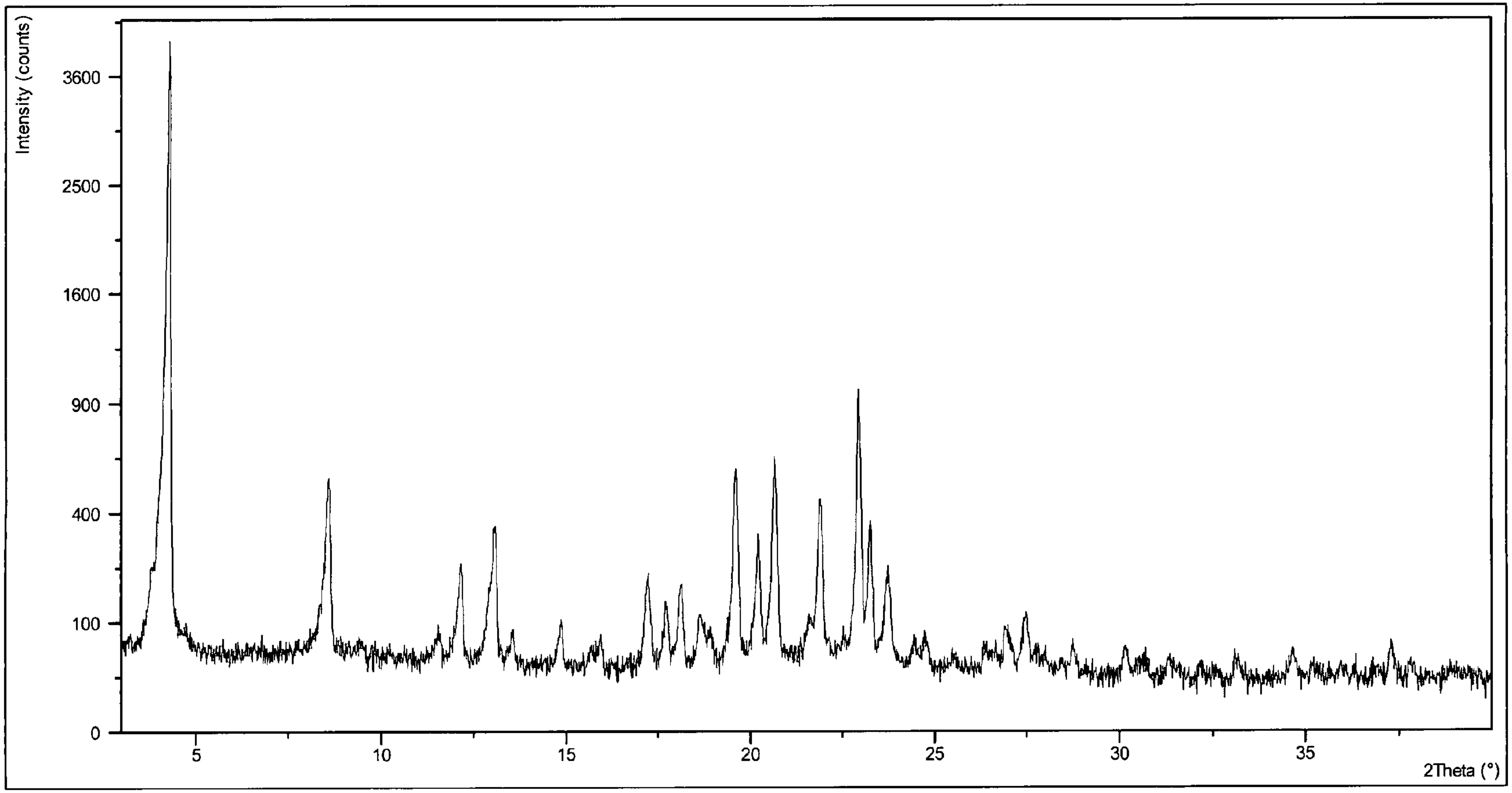
Intensity (counts)
3600
2500
1600
900
400
100
0
5
10
15
20
25
30
35
2Theta (°)

Figure 34)

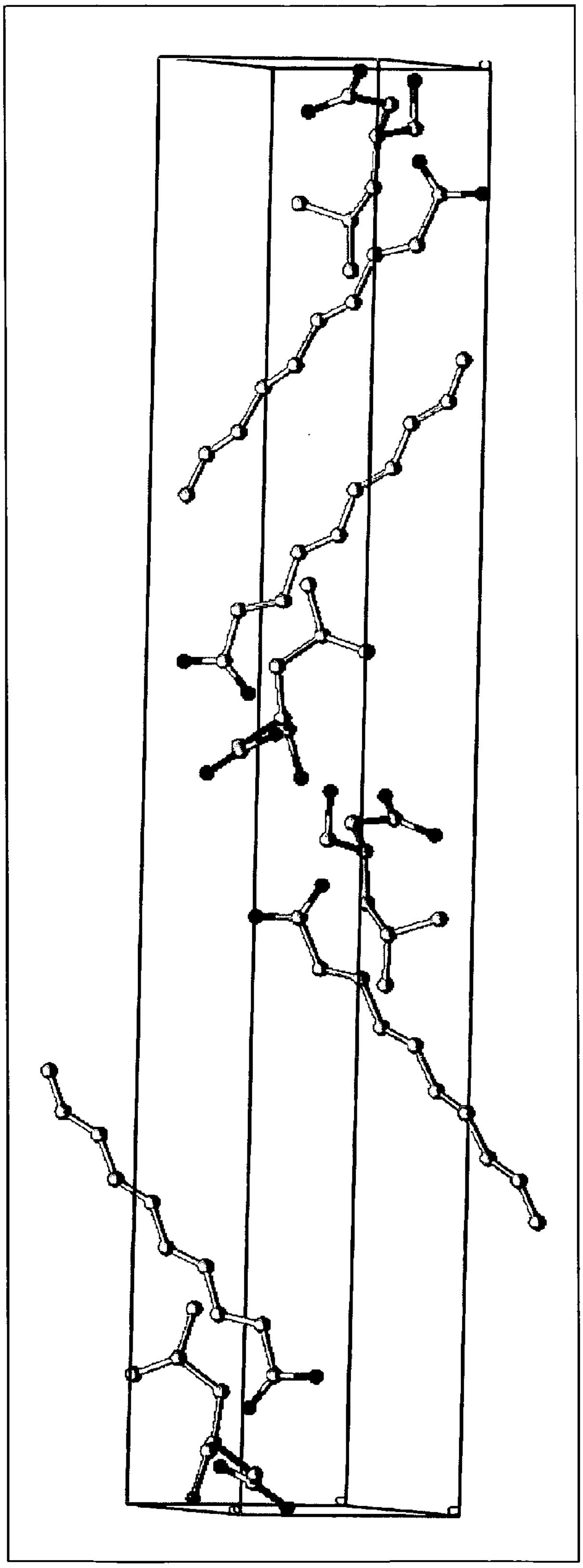

Figure 35)

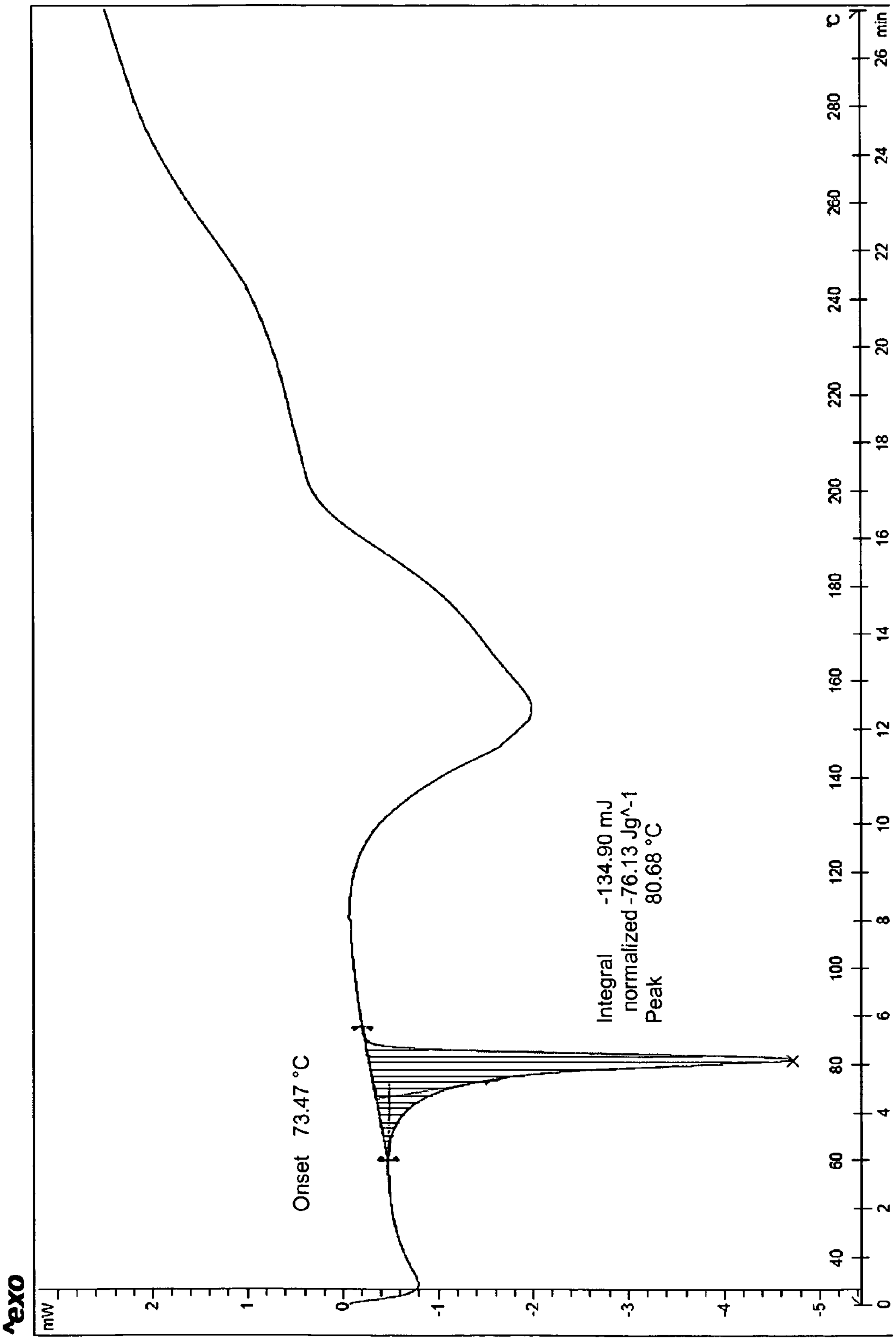
^exo
mW
Onset 73.47 °C
Integral -134.90 mJ
normalized -76.13 Jg^-1
Peak 80.68 °C
°C
min

Figure 36)

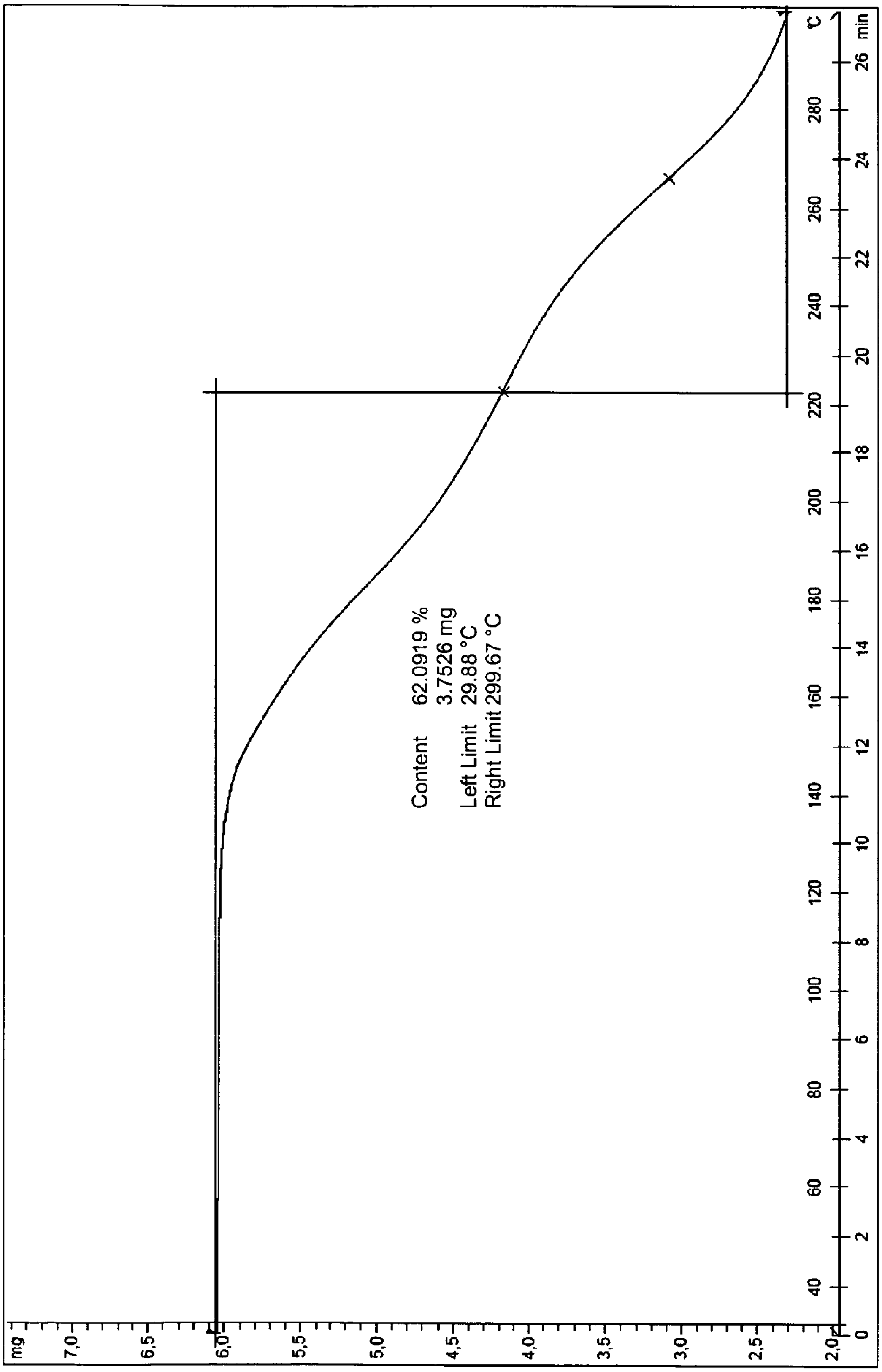
Content 62.0919 %
3.7526 mg
Left Limit 29.88 °C
Right Limit 299.67 °C
mg
7,0
6,5
6,0
5,5
5,0
4,5
4,0
3,5
3,0
2,5
2,0
40
60
80
100
120
140
160
180
200
220
240
260
280
°C
0
2
4
6
8
10
12
14
16
18
20
22
24
26
min

Figure 37)

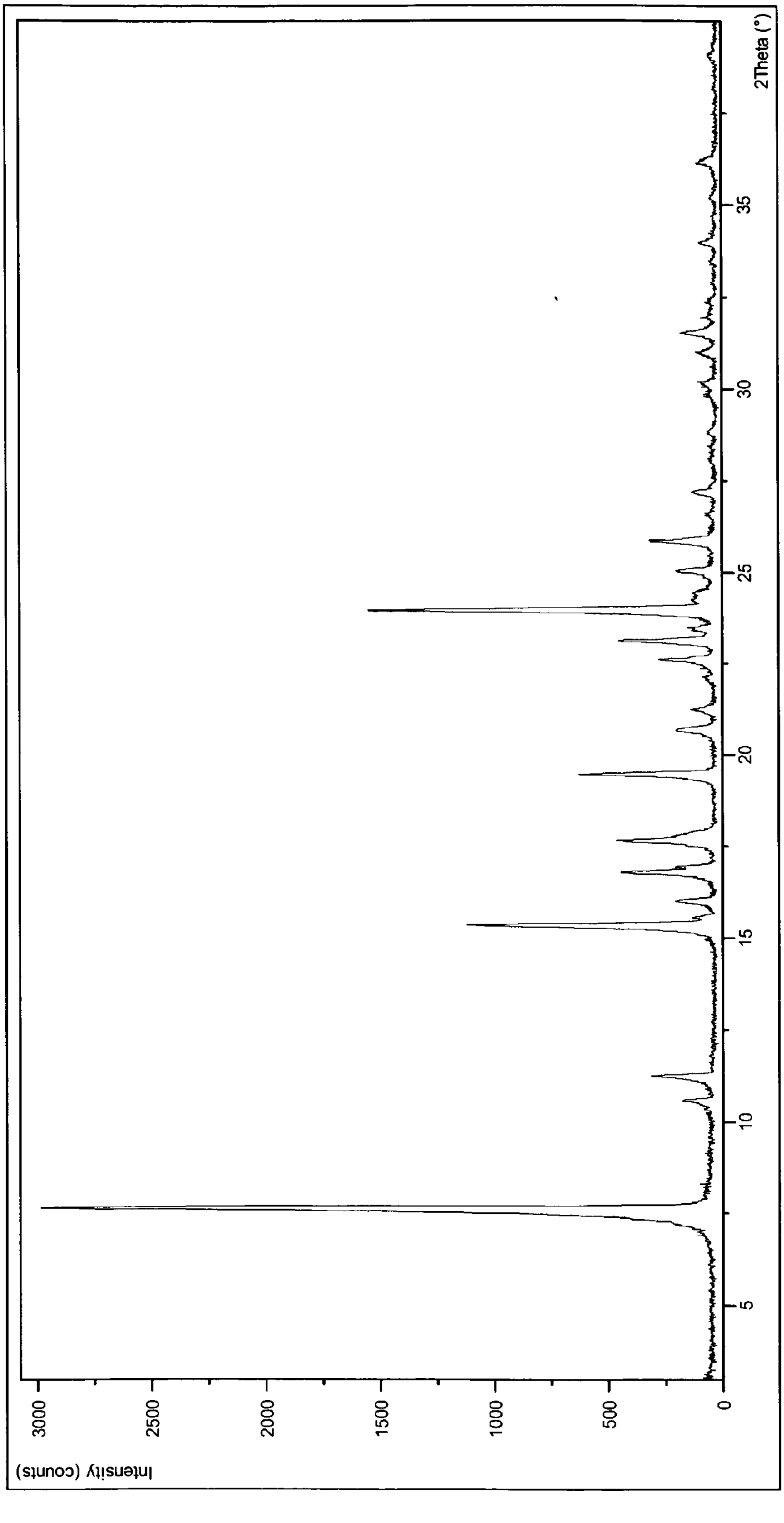
Intensity (counts)
2Theta (°)
0
500
1000
1500
2000
2500
3000
5
10
15
20
25
30
35

Figure 38)

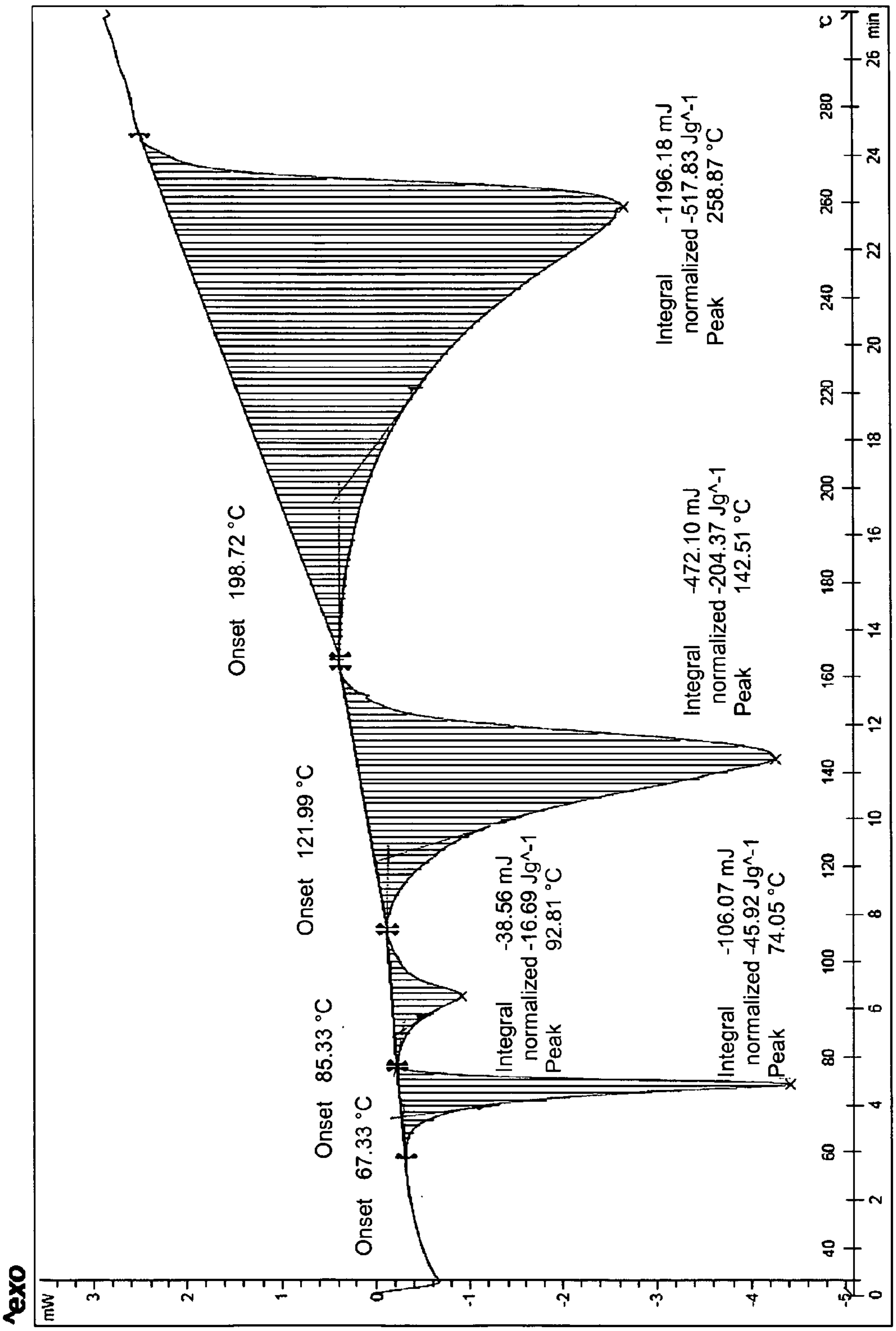
^exo
mW
Onset 67.33 °C
Onset 85.33 °C
Onset 121.99 °C
Onset 198.72 °C
Integral -38.56 mJ
normalized -16.69 Jg^-1
Peak 92.81 °C
Integral -106.07 mJ
normalized -45.92 Jg^-1
Peak 74.05 °C
Integral -472.10 mJ
normalized -204.37 Jg^-1
Peak 142.51 °C
Integral -1196.18 mJ
normalized -517.83 Jg^-1
Peak 258.87 °C
°C
min

Figure 39)

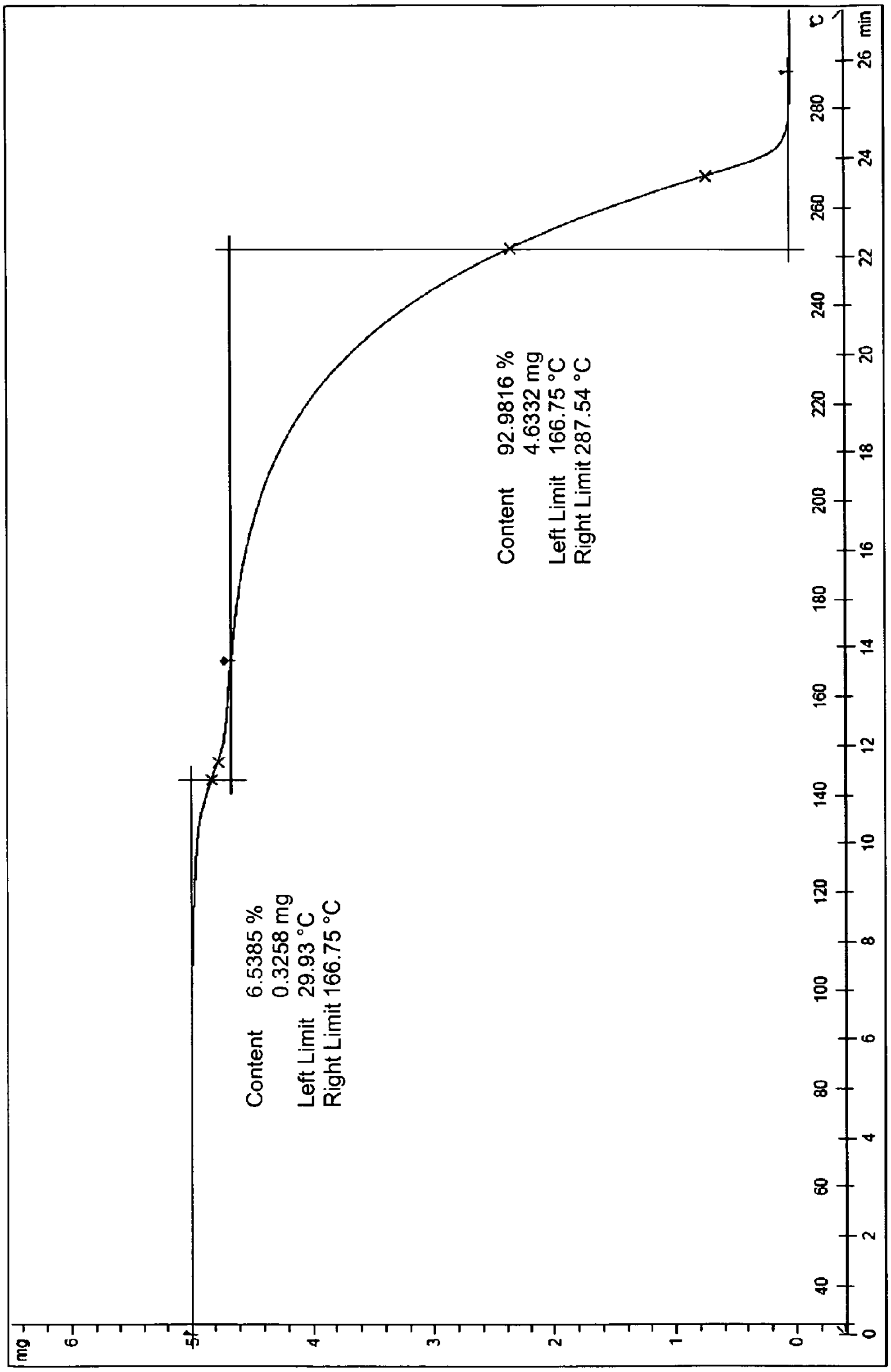

mg
°C
min
Content 6.5385 %
0.3258 mg
Left Limit 29.93 °C
Right Limit 166.75 °C
Content 92.9816 %
4.6332 mg
Left Limit 166.75 °C
Right Limit 287.54 °C

Figure 40)

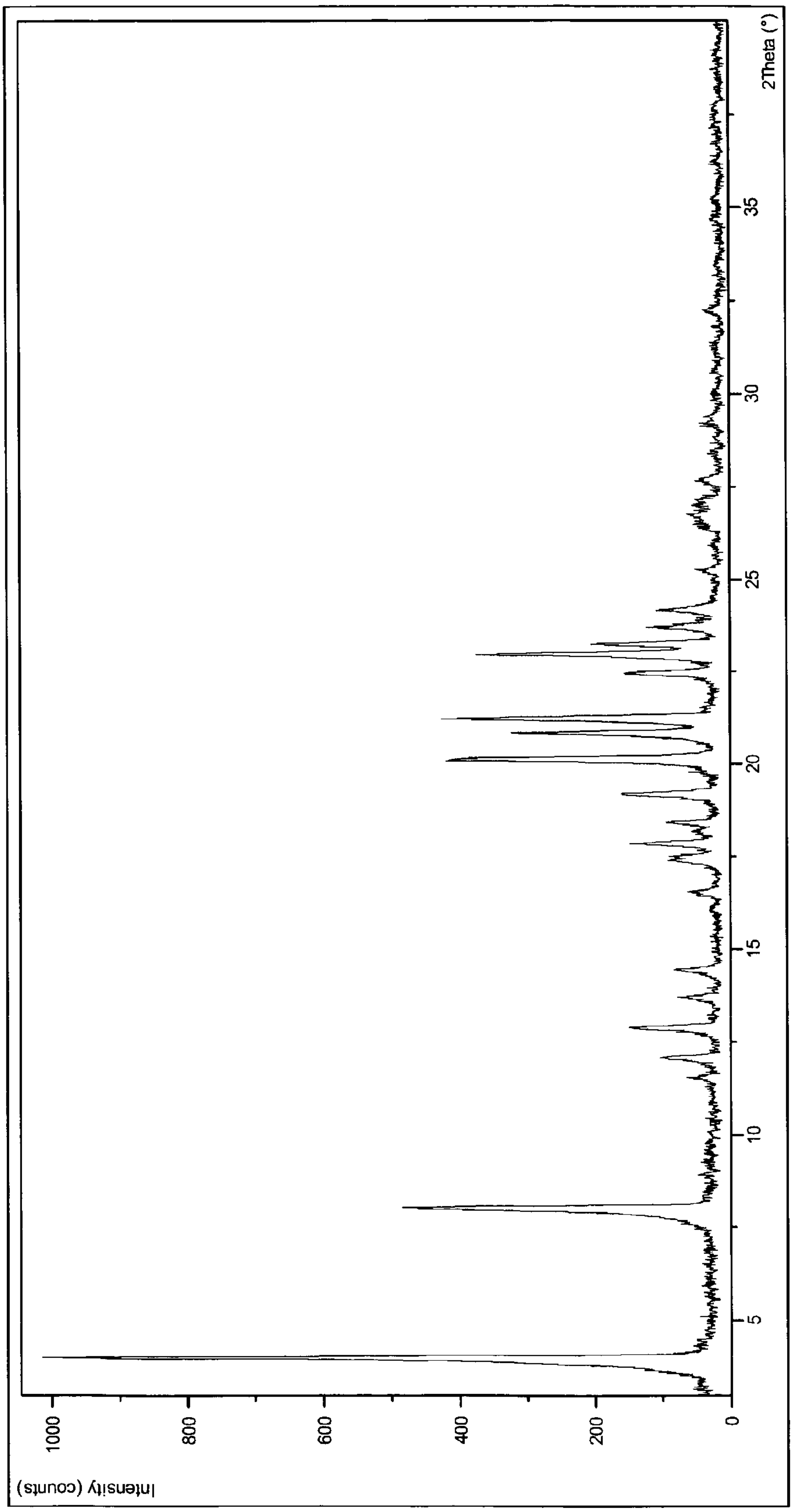
Intensity (counts)
1000
800
600
400
200
0
5
10
15
20
25
30
35
2Theta (°)

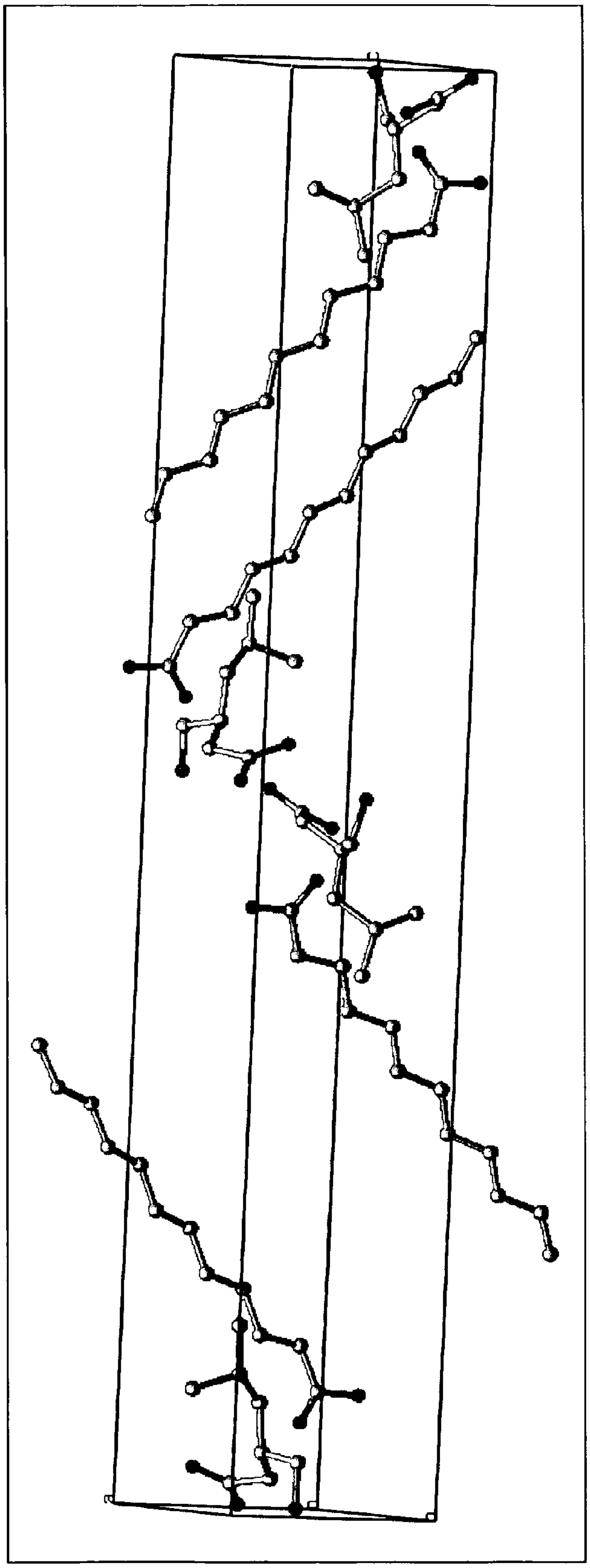

Figure 41)

Europäisches Patentamt
European Patent Office
Office européen des brevets

# EUROPEAN SEARCH REPORT

Application Number
EP 11 38 4001

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/170945 A1 (WESTHEIM RAYMOND J H [NL]) 2 July 2009 (2009-07-02)<br>* page 1, paragraphs [0008] and [0009]; page 2, paragraphs [0027] and [0030]; page 3, paragraphs [0040] and [0050]; claims *<br>----- | 1-16 | INV.<br>C07C229/08<br>C07C275/02<br>C07C53/126<br>C07C57/145<br>C07C59/245<br>C07C59/255<br>C07C63/06<br><br>ADD.<br>A61K31/197<br>A61P29/00 |
| X | WO 2009/122215 A1 (GENERICS UK LTD [GB]; MYLAN INDIA PRIVATE LTD [IN]; GORE VINAYAK [IN];) 8 October 2009 (2009-10-08)<br>* claims; examples *<br>----- | 1-14 | |
| X | WO 2009/044409 A2 (NATCO PHARMA LTD [IN]; KONAKANCHI DURGA PRASAD [IN]; PILLI RAMAKRISHNA) 9 April 2009 (2009-04-09)<br>* claims; examples *<br>----- | 1 | |
| X | WO 2008/138874 A1 (CHEMO IBERICA S A [ES]; TUFARO ROBERTO [IT]; MARRAS GIOVANNI [IT]) 20 November 2008 (2008-11-20)<br>* claims; examples *<br>----- | 1 | TECHNICAL FIELDS SEARCHED (IPC) |
| X | WO 2009/087674 A2 (WATSON PHARMA PRIVATE LTD [IN]; CHAVAN AMIT ANANT [IN]; PADWAL SHASHIK) 16 July 2009 (2009-07-16)<br>* claims 1-6; examples 6-9 *<br>----- | 1 | C07C |
| X | WO 2009/082861 A1 (ZHEJIANG JIUZHOU PHARMACEUTICA [CN]; XU JIANKANG [CN]; ZHANG DA [CN];) 9 July 2009 (2009-07-09)<br>* abstract *<br>-& US 2011/098502 A1 (XU JIANKANG [CN] ET AL) 28 April 2011 (2011-04-28)<br>* claims; examples *<br>-----<br>-/-- | 1 | |

The present search report has been drawn up for all claims

2

| Place of search | Date of completion of the search | Examiner |
|---|---|---|
| The Hague | 28 October 2011 | Zervas, Brigitte |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 11 38 4001

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SHAN N ET AL: "The role of cocrystals in pharmaceutical science", DRUG DISCOVERY TODAY, ELSEVIER, RAHWAY, NJ, US, vol. 13, no. 9-10, 1 May 2008 (2008-05-01), pages 440-446, XP022649919, ISSN: 1359-6446, DOI: 10.1016/J.DRUDIS.2008.03.004 [retrieved on 2008-04-22] * the whole document * ----- | 1-16 | |
| A,D | BRIAN SAMAS ET AL: "1:1 Cocrystal of ( S )-3-(ammoniomethyl)-5-methylhexanoate and ( S )-mandelic acid", ACTA CRYSTALLOGRAPHICA SECTION E STRUCTURE REPORTS ONLINE, vol. 63, no. 10, 15 October 2007 (2007-10-15), pages O3938-O3938, XP55010450, ISSN: 1600-5368, DOI: 10.1107/S1600536807041803 * the whole document * ----- | 1-16 | TECHNICAL FIELDS SEARCHED (IPC) |
| A | WO 2009/001372 A2 (SATYANARAYANA REDDY MANNE [IN]; THIRUMALAI RAJAN SRINIVASAN [IN]; ESWA) 31 December 2008 (2008-12-31) * claims 34-38; example 14 * ----- | 1-16 | |

The present search report has been drawn up for all claims

2

| Place of search | Date of completion of the search | Examiner |
|---|---|---|
| The Hague | 28 October 2011 | Zervas, Brigitte |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 11 38 4001

This annex lists the patent family members relating to the patent documents cited in the above-mentioned European search report.
The members are as contained in the European Patent Office EDP file on
The European Patent Office is in no way liable for these particulars which are merely given for the purpose of information.

28-10-2011

| Patent document cited in search report | Publication date | Patent family member(s) | Publication date |
|---|---|---|---|
| US 2009170945 A1 | 02-07-2009 | AR 071274 A1 | 09-06-2010 |
| | | EP 2242737 A1 | 27-10-2010 |
| | | WO 2009080365 A1 | 02-07-2009 |
| WO 2009122215 A1 | 08-10-2009 | AU 2009233536 A1 | 08-10-2009 |
| | | CA 2719892 A1 | 08-10-2009 |
| | | CN 102089273 A | 08-06-2011 |
| | | EP 2262761 A1 | 22-12-2010 |
| | | JP 2011516459 A | 26-05-2011 |
| | | US 2011124909 A1 | 26-05-2011 |
| WO 2009044409 A2 | 09-04-2009 | NONE | |
| WO 2008138874 A1 | 20-11-2008 | NONE | |
| WO 2009087674 A2 | 16-07-2009 | EP 2225199 A2 | 08-09-2010 |
| | | US 2010312010 A1 | 09-12-2010 |
| WO 2009082861 A1 | 09-07-2009 | CN 101910112 A | 08-12-2010 |
| | | DE 112008003594 T5 | 25-11-2010 |
| | | US 2011098502 A1 | 28-04-2011 |
| US 2011098502 A1 | 28-04-2011 | WO 2009082861 A1 | 09-07-2009 |
| | | CN 101910112 A | 08-12-2010 |
| | | DE 112008003594 T5 | 25-11-2010 |
| WO 2009001372 A2 | 31-12-2008 | EP 2170813 A2 | 07-04-2010 |
| | | US 2010179345 A1 | 15-07-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9323383 A
- EP 641330 A
- WO 2005041927 A
- WO 9640617 A

### Non-patent literature cited in the description


- *Acta Crystallographica, Section E: Structure Reports Online,* 2007, vol. E63 (10), o3938, So3938, 1-So393811
- **AULTON, M.E.** Pharmaceutics: The Science of Dosage Forms. 2002
- Encyclopedia of Pharmaceutical Technology. Marcel Dekker, Inc, 2002
- Modern Pharmaceutics. Marcel Dekker, Inc, 2002
- The Theory and Practice of Industrial Pharmacy. Lea & Febiger, 1986
- Modified-Release Drug Delivery Technology. Marcel Dekker, Inc, 2002
- Handbook of Pharmaceutical Controlled Release Technology. Marcel Dekker, Inc, 2000
- Controlled Drug Delivery. Basic Concepts. CRD Press Inc, 1983, vol. I
- Oral Drug Delivery. **TAKADA, K. ; YOSHIKAWA, H.** Encyclopedia of Controlled Drug Delivery. John Wiley & Sons, Inc, 1999, vol. 2, 728-742
- Oral drug delivery, small intestine and colon. **FIX, J.** Encyclopedia of Controlled Drug Delivery. John Wiley & Sons, Inc, 1999, vol. 2, 698-728
- **C. F. MACRAE ; I. J. BRUNO ; J. A. CHISHOLM ; P. R. EDGINGTON ; P. MCCABE ; E. PIDCOCK ; L. RODRIGUEZ-MONGE ; R. TAYLOR ; J. VAN DE STREEK ; P. A. WOOD.** *J. Appl. Cryst.,* 2008, vol. 41, 466-470
- **C.F. MACRAE ; I.J. BRUNO ; J.A. CHISHOLM ; P.R. EDGINGTON ; P. MCCABE ; E. PIDCOCK ; L. RODRIGUEZ-MONGE ; R. TAYLOR ; J. VAN DE STREEK ; P.A. WOOD.** *J. Appl. Cryst.,* 2008, vol. 41, 466-470